(19) Europäisches Patentamt
European Patent Office
Office européen des brevets

(11) EP 2 679 301 A1

(12) **EUROPÄISCHE PATENTANMELDUNG**

(43) Veröffentlichungstag:
**01.01.2014 Patentblatt 2014/01**

(21) Anmeldenummer: 13157246.3

(22) Anmeldetag: **28.02.2013**

(51) Int Cl.:
*B01J 20/26* (2006.01)          *A61M 1/36* (2006.01)
*B01J 20/28* (2006.01)          *B01J 20/32* (2006.01)
*A61K 31/745* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **28.06.2012 EP 12174028**

(71) Anmelder: **Zentrum für biomedizinische
Technologie der Donau-
Universität Krems
3500 Krems (AT)**

(72) Erfinder:
• **Falkenhagen, Dieter
3500 Krems (AT)**
• **Hartmann, Jens
3511 Furth (AT)**
• **Harm, Stephan
3601 Dürnstein (AT)**

(74) Vertreter: **Patentanwaltskanzlei
Matschnig & Forsthuber OG
Siebensterngasse 54
1071 Wien (AT)**

(54) **Extrakorporale Perfusionsvorrichtung**

(57) Die Erfindung betrifft eine extrakorporale Perfusionsvorrichtung (100, 200, 300, 400, 500, 600, 700) umfassend einen extrakorporalen Blutkreislauf (102, 202, 302, 402, 502, 602, 702) zum Führen von Blut, einen Filtratkreislauf (105, 205, 305, 405, 505, 605, 705) zum Führen von Blutplasma und eine Steuerung (110, 210, 310, 410, 510, 610, 710),
wobei der Filtratkreislauf (105, 205,...705) mit dem extrakorporalen Blutkreislauf (102, 202,...702) mittels eines Filters (104, 204, 304, 404, 504, 604, 704) in Verbindung steht, wobei der Filter (104, 204...704) einen Siebkoeffizienten von 5% für Substanzen mit einer molaren Masse von 340 000 g/mol (relative Molekülmasse von 340 kDa) aufweist, und
wobei im Filtratkreislauf (105, 205,...705) ein Abreicherungsmittel (107, 207, 307, 407, 507, 607, 707) umfassend einen ersten Träger (107a, 207a, 307a, 407a, 507a, 607a, 707a) mit einer neutralen, hydrophoben Oberfläche angeordnet ist, wobei die Perfusionsvorrichtung ein Abgabemittel (107, 207, 308, 408, 508, 607, 708) zum Zuführen eines endotoxinbindenden Lipopeptids in den extrakorporalen Blutkreislauf (102, 202,...702) umfasst, wobei das endotoxinbindende Lipopeptid aus der Gruppe bestehend aus Polymyxinen, Polymyxin-Derivaten, deren Prodrugs und einer Kombination davon ausgewählt ist.

Fig. 1

Printed by Jouve, 75001 PARIS (FR)

**(Forts. nächste Seite)**

EP 2 679 301 A1

Fig. 2

Fig. 3

Fig. 4

Fig. 5

Fig. 6

Fig. 7

**Beschreibung**

Die Erfindung betrifft eine extrakorporale Perfusionsvorrichtung umfassend einen extrakorporalen Blutkreislauf zum Führen von Blut, einen Filtratkreislauf zum Führen von Blutplasma und eine Steuerung, wobei der Filtratkreislauf mit dem extrakorporalen Blutkreislauf mittels eines Filters in Verbindung steht, wobei der Filter einen Siebkoeffizienten von 5% für Substanzen mit einer molaren Masse von 340 000 g/mol (relative Molekülmasse von 340 kDa) aufweist, und wobei im Filtratkreislauf ein Abreicherungsmittel umfassend einen ersten Träger mit einer neutralen, hydrophoben Oberfläche angeordnet ist.

Sepsis und damit verbundenen Komplikationen tragen in einem nicht unbeachtlichen Ausmaß zur Morbidität und Mortalität bei Menschen bei. In den meisten Fällen ist eine Sepsis auf eine Infektion mit gram-negativen Bakterien zurückzuführen, wenn hohe Endotoxinkonzentrationen in den Körper gelangen und systemische Wirkung zeigen.

Endotoxine sind Lipopolysaccharide (LPS) in der Zellwand gramnegativer Bakterien und werden durch Zelllyse und Zellteilung freigesetzt. In der Tat sind Lipopolysaccharide der häufigste Lipidbestandteil der äußeren Zellmembran gramnegativer Bakterien. Endotoxine sind pyrogene Substanzen und das betroffene Individuum reagiert mit einer starken Entzündungsreaktion und Fieber, wenn Endotoxine, beispielsweise im Zuge einer mikrobiellen Vergiftung, in den Körper gelangen und als Schlüsselmediatoren eine unkontrollierte Aktivierung des mononukleär-phagozytären Systems bewirken. Eine Akkumulation von Endotoxinen im Blutkreislauf infolge einer Endotoxinämie führt zu einer unkontrollierten Aktivierung der Immunzellen und einem Ungleichgewicht des Gerinnungssystems. Dies kann zu einer Sepsis führen, welche unter anderem durch hohes Fieber, niedrigen Blutdruck und, in schweren Fällen, durch Multiorganversagen gekennzeichnet ist. Eine Sepsis ist eine sehr ernstzunehmende Erkrankung; die Letalität von Individuen mit schwerer Sepsis oder septischem Schock ist je nach Schweregrad der Erkrankung ca. 30-60%. Eine Endotoxinämie infolge einer Infektion mit gram-negativen Bakterien gehört zur häufigsten Ursache für das Auftreten einer systemischen inflammatorischen Reaktion ("systemic inflammatory response syndrome", SIRS), einer Sepsis, einer schwerwiegenden Sepsis oder eines septischen Schocks und den daraus resultierenden schwerwiegenden Komplikationen. Auch Patienten mit beeinträchtigter Immunabwehr, wie z.B. Leberpatienten oder Patienten in Chemotherapie, neigen zu bakteriellen Infektionen und zeigen dadurch Symptome einer Endotoxinvergiftung. Bei akutem Leberversagen oder einer akuten Dekompensation bei chronischem Leberversagen kann es ebenfalls zu einer Endotoxinämie kommen, durch welche sich Zustände entwickeln, die - biochemisch gesehen - einer Sepsis sehr ähnlich sind. Beispielsweise kann es bei Patienten mit chronischem Leberversagen zu einer akuten Dekompensation kommen. In diesem Zustand können die aus der normalen Darmflora stammenden Endotoxine die Darmbarriere überwinden und im Körper die Freisetzung von Entzündungsmediatoren stimulieren und somit einen Sepsis-ähnlichen Zustand hervorrufen.

Ferner können septische Zustände auch durch gram-positive Bakterien, Viren und Pilze ausgelöst werden.

Es ist wie erwähnt allgemein bekannt, dass es bei einer Sepsis und bei anderen schwerwiegenden Erkrankungen zu einer unkontrollierten Aktivierung der Immunzellen und zu einem Ungleichgewicht des Gerinnungssystems kommen kann. Die unkontrollierte Aktivierung des mononukleär-phagozytären Systems stimuliert eine übermäßige Freisetzung von Entzündungsmediatoren, insbesondere von Zytokinen (auch als Zytokinsturm oder Hyperzytokinämie bezeichnet). Zytokine gelten als Schlüsselmediatoren bei Sepsis und septischem Schock. Als wichtigste pro-inflammatorische Vertreter sind Tumor-Nekrose-Faktor (TNF-$\alpha$, oft auch nur als TNF bezeichnet) und Interleukin-1$\beta$ (IL-1$\beta$) zu nennen. Weitere wichtige pro-inflammatorische Zytokine sind IL-6 und IL-8. Das initial freigesetzte Zytokin TNF-$\alpha$ löst über eine Mediatorkaskade eine biologischen Signalverstärkung aus, die zu physiologischen Veränderungen, einschließlich schwerer Störungen des biologischen Gleichgewichts, und in weiterer Folge zu Kreislaufkollaps und Multiorganversagen führt. Das klinische Bild einer Sepsis korreliert mit hohen Blut-Konzentrationen des Schlüsselmediators TNF-$\alpha$ aber auch anderer Zytokine wie IL1-ß, IL-6, IL-8 im Falle der proinflammatorischen Phase bzw. IL-10 bzw. IL-13 bei Auftreten einer anti-inflammatorischen Phase, in der die proinflammatorischen Mediatoren inklusive Zytokine sehr niedrige Konzentrationen aufweisen. Ferner stehen auch andere schwerwiegende Erkrankungen wie chronisch-entzündliche Darmerkrankungen, Psoriasis und Rheumatoide Arthritis mit einer übermäßigen TNF-$\alpha$-Freisetzung im Zusammenhang.

Zur Behandlung einer Sepsis werden neben der standardmäßig angewandten intensivmedizinischen Behandlung vor allem Antibiotika bzw. Cortikosteroide, Immunglobuline sowie Kreislauf-unterstützende Arzneimittel eingesetzt.

Ein Nachteil der Antibiotika-Therapie ist die zunehmende Verbreitung antibiotikaresistenter Bakterien. Ferner werden durch das Antibiotikum und der damit einhergehenden Zerstörung der Bakterienzellen Endotoxine vermehrt freigesetzt, was wiederum zu einer verstärkten Ausschüttung von Entzündungsmediatoren führt. Zudem ist eine Antibiotikagabe häufig mit Nebenwirkungen wie Veränderungen der Darmflora oder allergischen Reaktionen verbunden. Der Versuch, Antikörper gegen den Schlüsselfaktor TNF-alpha einzusetzen, scheiterte, da offenbar bei dieser Methode die Absenkung der TNF-Konzentration auf Null oder sehr geringe Werte eine anergische Situation auslöste, die von einer höheren Mortalität im Vergleich zur Kontrollgruppe begleitet war. Der therapeutische Einsatz spezifischer Antikörper gegen LPS und TNF-$\alpha$ ist technisch sehr aufwändig und daher mit hohen Kosten verbunden.

Mittels extrakorporaler Blut- bzw. Plasmareinigungssysteme (therapeutische Aphereseverfahren) wird, wie im Folgenden näher beschrieben, daher versucht, die genannten Zytokine insbesondere den Faktor TNF-$\alpha$ in einer die

Konzentrationen dieser Zytokine normalisierenden Weise zu entfernen, um somit die anergische (antiinflammatorische) Phase zu umgehen. Mittels sogenannter LPS-Adsorber (z.B. der Adsorber Toraymyxin®) können Endotoxine eliminiert werden, um somit eine Freisetzung der proinflammatorischen Zytokine zu vermeiden, die natürlich auch die antiinflammatorische Reaktion mindert.

Aphereseverfahren sind extrakorporal durchgeführte Verfahren, bei welchen pathophysiologisch relevante Blut- und Plasmakomponenten, beispielsweise Biomoleküle wie (Glyko)Proteine, Peptide, Lipide, Lipoproteine und Lipopolysaccharide, aber auch Blutzellen und Blutplasma entfernt werden. Aphereseverfahren können einerseits zu diagnostischen und therapeutischen Zwecken eingesetzt werden, andererseits stellen sie auch eine sehr effektive Möglichkeit dar, bestimmte Blutbestandteile von gesunden Individuen in ausreichender Menge und in ausreichend hoher Reinheit zu gewinnen. Große Bedeutung wird der therapeutischen Apherese zugemessen, da diese bei bestimmten Indikationen oft eine sehr wirkungsvolle und gleichzeitig nebenwirkungsarme Alternative zur medikamentösen Behandlung ist. So kann bei Plasmaphereseverfahren das Plasma entweder vollständig abgetrennt und durch eine Substitutionslösung ersetzt werden, oder es werden nur bestimmte Bestandteile wie Zytokine, LDL, Endotoxine oder Immunglobuline mittels eines Adsorbers daraus entfernt und das Plasma anschließend in den Spender/Patienten wieder zurückgeführt. Therapeutische Aphereseverfahren haben gegenüber den genannten medikamentösen Behandlungsstrategien auch den Vorteil, dass die Behandlung jederzeit durch Abschalten der Apheresevorrichtung mit sofortiger Wirkung angehalten wird.

Aphereseverfahren und Adsorbermaterialien zum Eliminieren toxischer und/oder schädlicher Blutbestandteile sind im Stand der Technik gut bekannt. Ebenso bekannt sind Adsorbermaterialien, welche spezifisch Zytokine, insbesondere TNF-$\alpha$, und/oder Endotoxine (LPS) adsorbieren und aus Körperflüssigkeiten wie Blut oder Blutplasma entfernen.

Das Dokument US 2001/0070424 A1 offenbart ein Adsorbermaterial basierend auf einem porösen Polymer, welches zumindest eine Transportpore mit einem Durchmesser von 25 bis 200 nm sowie effektive Poren mit einem Durchmesser von 10 bis 25 nm aufweist. Das Polymer kann unter anderem auch ein nicht-ionisches Harz (Neutralharz) sein. Der Adsorber wird zum Entfernen von Proteinmolekülen, insbesondere Zytokinen und $\beta$2-Mikroglobulin, verwendet.

Das Dokument WO 2011/123767 A1 offenbart ein Verfahren zum Behandeln von Entzündungen, wobei einem Patienten eine therapeutisch wirksame Dosis an porösen Adsorberpartikeln zum Adsorbieren von Entzündungsmediatoren verabreicht wird, wobei das Gesamtporenvolumen bei einer Porengröße von 5 bis 300 nm größer als 0,5 cc/g bis 3,0 cc/g ist.

In der WO 2003/090924 ist eine poröse Abtrennmatrix zum Abtrennen von Blutbestandteilen im Zusammenhang mit Entzündungsprozessen beschrieben. Die Abtrennmatrix weist eine Porengröße von 5 $\mu$m bis 500 $\mu$m sowie wenigstens eine an der Matrix angeordnete funktionelle Gruppe auf.

Die DE 19515554 A1 offenbart Verfahren und Vorrichtungen zur simultanen extrakorporalen Elimination von TNF-$\alpha$ und Lipopolysacchariden aus Vollblut und/oder Blutplasma. Dabei wird das Blut oder Blutplasma in einem extrakorporalen Perfusionssystem über ein poröses Kationenaustauscher- und ein Anionenaustauschermaterial geleitet. Die darin beschriebenen porösen Trägermaterialien weisen einen mittleren Porendurchmesser von < 30 nm und/oder eine molekulare Ausschlussgröße für globuläre Proteine von < $10^6$ Dalton und insbesondere < $2 \times 10^4$ Dalton auf.

Neutralharze zum Entfernen toxischer Bestandteile, einschließlich Zytokine, aus einer Körperflüssigkeit sind weiters in der WO 2005/082504 A2 offenbart. Die WO 2005/082504 A2 beschreibt eine Entgiftungsvorrichtung, welche Aktivkohle und zumindest ein nicht-ionisches Harz mit einer mittleren Porengröße von 30 nm und einem mittleren Partikeldurchmesser von 35-120 $\mu$m (Amberchrom CG300C) bzw. mit einer mittleren Porengröße von 45 nm und einem mittleren Partikeldurchmesser von 560 $\mu$m (Harz auf Basis aliphatischer Ester - Amberlite XAD-7HP) aufweist.

EP 0787500 B1 und EP 0958839 B1 offenbaren ein hydrophobes Trägermaterial mit einer Porengröße von 10 bis 30 nm und Partikelgrößen von 20 bis 350 $\mu$m, vorzugsweise 10 bis 100 $\mu$m oder 250 bis 350 $\mu$m zum Entfernen von toxischen Bestandteilen, insbesondere Zytokinen, aus einer Körperflüssigkeit.

Die EP 1 944 046 B1 offenbart ein Trägermaterial auf Basis eines Polystyrol-Divinylbenzol-Copolymers mit einer Porengröße von 30 nm und einer Partikelgröße von 75 bis 120 $\mu$m.

Tetta *et al.* (Tetta et al. 1998. Nephrol Dial Transplant 13:1458-1464) beschreiben einen Adsorber des Amberchrom-Typs CG 300md mit einer Porengröße von 30 nm zum Entfernen von Zytokinen aus einer Körperflüssigkeit.

Die Veröffentlichung von Cantaluppi *et al.* (Cantaluppi et al. 2010. Critical Care 14:R4) beschreibt einen Adsorber des Typs Amberchrom CG161m zur Zytokinadsorption.

Es hat sich ferner gezeigt, dass Anionenaustauscherharze (z.B. DEAE oder PEI-Gruppen an Cellulose gebunden) sehr gut für die Endotoxin-Bindung geeignet sind. Nachteilig ist jedoch die unerwünschte Bindung wichtiger Faktoren des intrakorporalen Gerinnungssystems wie Protein C und Protein S und die damit verbundene Gerinnungsproblematik. Diese Gerinnungsproblematik kann durch die Verwendung eines spezifischen Adsorbers, der immobilisierte Antikörper gegen Endotoxine aufweist, umgangen werden. Diese Möglichkeit ist jedoch aus ökonomischen Gründen nur limitiert anwendbar.

In der DE 199 13 707 A1 wird ein Immunadsorber zum Einsatz in der Sepsistherapie für die Plasmapherese beschrieben, bestehend aus einem Trägermaterial aus organischen oder synthetischen Polymeren und darauf gebundenen poly- oder monoklonalen Antikörpern, die gegen Komplementfaktoren, Lipopolysaccharide sowie gegen weitere Sepsis-Mediatoren wie z.B. TNF-$\alpha$ und Interleukine gerichtet sind.

Die DE 10 2004 029 573 A1 offenbart ein Apheresematerial bzw. Adsorbens sowie ein Verfahren zur Entfernung, Abreicherung oder Inaktivierung des Zytokins MIF (Makrophagenmigrationsinhibierender Faktor) aus Blut, Blutplasma oder anderen Körperflüssigkeiten. Das Adsorbens umfasst ein festes Trägermaterial auf dessen Oberfläche MIF-bindende Moleküle oder funktionale Gruppen immobilisiert sind.

In der DE 10 2005 046 258 A1 ist ein Immunadsorber zur Behandlung von Insulinresistenz und/oder des metabolischen Syndroms geoffenbart, wobei der Immunadsorber Trägermaterialen mit gebundenen Liganden, die für IL-6, IL-4 und C5a spezifisch sind, umfasst.

Eine bereits seit langem in der klinischen Anwendung eingesetzte Therapieform stellt die parenterale Verabreichung von Polymyxinen dar. Polymyxine sind antibiotische Substanzen, welche ursprünglich aus dem Bakterium *Bacillus polymyxa* stammen und die bereits seit Jahrzehnten zur Behandlung von Infektionen mit gram-negativen Bakterien bei Menschen und Tieren eingesetzt werden. Polymyxine greifen in die Zellwandstruktur ein, indem sie die Permeabilität der Zellmembran erhöhen, aufgrund dessen es zur Zelllyse kommt. Polymyxine binden nicht nur Phospholipide, sondern auch Endotoxine (LPS) unter Bildung eines Polymyxin-Endotoxin(LPS)-Komplexes mit hoher Affinität. Der antibakterielle Mechanismus der Polymyxine ist beispielsweise in einer Publikation von Tony Velkov *et al.* eingehend beschrieben (Tony Velkov et al. 2010. Journal of Medicinal Chemistry: 53(5):1898-1916).

Aufgrund der neurotoxischen und nephrotoxischen Wirkung der Polymyxine haben nur Polymyxin B und Polymyxin E (Colistin) gewisse therapeutische Bedeutung als Antibiotikum erlangt. Bis zum heutigen Zeitpunkt sind diese beiden Polymyxine die einzigen therapeutisch zugelassenen Vertreter ihrer Substanzklasse. Polymyxin B und Colistin sind in den USA von der FDA zur parenteralen Infusion zugelassen. Polymyxin B bzw. Colistin werden seit Jahrzehnten für orale oder topische Therapieformen eingesetzt. Für eine parenterale, systemische Behandlung bei Erkrankungen und Zuständen infolge einer Infektion mit gramnegativen Bakterien kommen sie aufgrund ihrer neuro- und nephrotoxischen Nebenwirkungen allerdings nur als letzte Auswegsmöglichkeit als Antibiotikum zur therapeutischen Anwendung. Colistin scheint weniger nephrotoxisch zu sein als Polymyxin B, durch die erforderliche höhere Dosierung wird dies jedoch zumindest teilweise ausgeglichen, so dass im klinischen Alltag etwa in gleichem Ausmaß mit nephrotoxischen Reaktionen gerechnet werden kann. Ausreichende Daten zur Nephrotoxizität der beiden Antibiotika liegen aus heutiger Sicht allerdings nicht vor. Infektiologen aus New York (USA) beschreiben Nierenversagen bei 14 % von 60 Patienten, die mit Polymyxin B behandelt wurden. Ärzte in Griechenland beschreiben eine deutliche Nephrotoxizität bei der Mehrheit der Patienten, bei welchen eine renale Insuffizienz bereits bei Therapiebeginn vorlag. Bei Patienten mit normaler Funktion der Nieren wurden dagegen keine wesentlichen Veränderungen festgestellt. Eine detaillierte Übersicht über die Toxizität von Polymyxinen findet sich in einer Publikation von Falagas und Kasiakou (Falagas und Kasiakou. 2006. Critical Care 10:R27). Die Dosierung von Polymyxinen spielt folglich eine zentrale Rolle bei der Vermeidung bzw. der Minimierung toxischer Nebenwirkungen, insbesondere nephrotoxischer Nebenwirkungen.

Aufgrund des in den letzten Jahren beobachteten gehäuften Auftretens schwerwiegender Krankheitsverläufe infolge von Infektionen mit multiresistenten pathogenen Stämmen, beispielsweise bei akuten Infektionen mit Stämmen des Bakteriums *Pseudomonas aeruginosa,* werden Polymyxine trotz ihrer Toxizität notgedrungen wieder vermehrt parenteral als Antibiotikum verabreicht. Eine Bezugsquelle für Polymyxin B in Form des Sulfatsalzes von Polymxin B1 und B2 zur parenteralen Verabreichung wird derzeit von Bedford Laboratories angeboten ("Polymyxin B for Injection 500 000 Units", Hersteller: Bedford Laboratories). Gemäß Herstellerinformation erfolgt die parenterale Verabreichung intravenös, intramuskulär oder im Fall einer Meningitis intrathekal, wobei die angegebene maximale Tagesdosis in der Regel 2,5 mg/kg Körpergewicht und Tag, aufgeteilt auf zwei bis drei Infusionen beträgt. Typischerweise liegt die Serumkonzentration von Polymyxin nach Verabreichung in einem Bereich von 1 bis 6 $\mu$g/ml. In schwerwiegenden Fällen kann diese auch höher in einem Bereich von 6 bis 50 $\mu$l/ml liegen. Colistin wird vorwiegend in Form von Colistin-Methansulfonat verabreicht, wobei die Serumkonzentration in einem Bereich von ca. 1 bis 3 $\mu$g/ml liegt. Colistin (Polymyxin E) wird in gleicher Weise wie Polymyxin B eingesetzt, zumeist in höherer Dosierung.

Eine Resistenz gegenüber Polymyxin B ist eher ungewöhnlich, kann sich aber entwickeln, wenn das Antibiotikum aufgrund von Veränderungen in der äußeren Membran die Zytoplasmamembran nicht erreicht. Polymyxine sind wirksam gegen viele gramnegative Erreger, wie *E. coli, Enterobacter, Klebsiella spp.* und auch gegen *P. aeruginosa. Proteus-Arten* und *S. marcescens,* die normalerweise resistent sind; die Empfindlichkeit von *B. fragilis* ist variabel. Die minimalen Hemmkonzentrationen für *E. coli* liegen im Bereich von 0,04 - 3,7 mg/l und für *P. aeruginosa* zwischen 1,2 und 33,3 mg/l (Garidel und Brandenburg. 2009. Anti-Infective Agents in Medicinal Chemistry, 8:367-385).

Da die bisher in der klinischen Anwendung eingesetzten Dosierungen für Polymyxin B und Colistin bei parenteraler Verabreichung nephro- und neurotoxische Nebenwirkungen induzieren, wurden in der Vergangenheit neue Behandlungsstrategien und Therapieansätze im Zusammenhang mit der Anwendung endotoxinbindender Lipopeptide wie Polymyxin entwickelt.

Als häufig angewandte Alternative zur Verabreichung von Polymyxinen in Form eines Medikaments haben sich die bereits zuvor genannten extrakorporalen Blut- und/oder Blutplasmareinigungsverfahren unter Verwendung geeigneter Adsorbermaterialien etabliert.

Bekannte Adsorbermaterialien umfassen poröse oder faserartige Trägermaterialien, auf deren Oberflächen Polymyxin B immobilisiert ist. Bislang wurde im Zusammenhang mit derartigen Adsorbermaterialien, die in hohem Maße in der Behandlung von septischen Zuständen zum Einsatz kommen, über keine neuro- und nephrotoxischen Nebenwirkungen berichtet.

In der EP 0110 409 A sind Polymyxin B-immobilisierte Träger aus porösem Glas (FPG 2000) sowie Polymyxin B-immobilisierte Polysaccharid-Träger auf Zellulose-Basis (Cellulofine A-3) offenbart. Ebenso bekannt sind Mikropartikel aus Zellulose oder derivatisierter Zellulose, an welche Polymyxin B kovalent gebunden ist (Weber V., Loth F., Linsberger I., Falkenhagen D.: Int. J. Artif. Organs 25(7), 679). Die EP 0 129 786 A2 beschreibt ein Endotoxin-Entgiftungsmaterial mit einem faserartigen Träger, an welchem Polymyxin kovalent immobilisiert ist. Der faserartige Träger ist mit funktionellen Gruppen ausgestattet ist, um Polymyxin kovalent an die Oberfläche des Trägers zu binden. Nachteilig an den genannten Endotoxin-Adsorbern ist die geringe Endotoxinbindungskapazität- und geschwindigkeit. Die Effektivität und die Qualität der Behandlung in Bezug auf faserartige Träger mit kovalent gebundendem Polymyxin B, wurden als suboptimal beschrieben (Cruz DN et al. 2007. Effectiveness of polymyxin B-immobilized fiber column in sepsis: a systematic review. Crit. Care 11(3):137).

In der WO 2010/083545 und der WO 2011/160149 wurden Adsorbermaterialien beschrieben, bei welchen Polymyxin über nicht-kovalente Wechselwirkungen (Adsorption) an hydrophoben Trägeroberflächen immobilisiert ist. Die WO 2007/142611 A1 und die US 5510242 beschreiben hydrophobe Trägeroberflächen mit adsorptiv gebundenen Polymyxinen. Die Verwendung von Polymyxin-beschichteten Polyester-Geweben zum Binden von LPS-Antigenen aus *Salmonella typhimurium* wurde von Blais und Yamazaki beschrieben (Blais und Yamazaki. 1990. Use of polymyxin-coated polyester cloth in the enzyme immunoassay of Salmonella lipopolysaccharide antigens. International journal of Food Microbiology 11:195-204).

Die WO 2011/133287 A1 offenbart eine Blutfiltrationsvorrichtung, die eine mikrofluidische Trennvorrichtung aufweist und mit welcher unerwünschte Substanzen wie Toxine, Arzneimittel, Pathogene und dergleichen aus dem Blut entfernt werden können. Die Vorrichtung kann Sensoren aufweisen, welche das Blut hinsichtlich der Gegenwart oder Konzentration der unerwünschten Substanzen überwacht. Die Überwachung kann auch die Infusion therapeutischer Wirkstoffe, wie beispielsweise eines Antibiotikums, in das Blut des Patienten einschließen.

Eine extrakorporale Perfusionsvorrichtung der eingangs genannten Art wurde beispielsweise von Falkenhagen *et al.* (Falkenhagen et al. 2006. Fluidized Bed Adsorbent System for Extracorporeal Liver Support. Therapeutic Apheresis and Dialysis 10(2):154-159) beschrieben. Der darin beschriebene Filter ist unter dem Handelsnamen "Albuflow®" (Fresenius Medical Care, Deutschland) erhältlich.

Obwohl die Letalität von Patienten mit Endotoxinämie-induzierten Erkrankungen, insbesondere Sepsis, durch die klinische Anwendung der oben genannten Polymyxin-basierten Adsorbermaterialien gesenkt werden konnte, ist die Letalität von Patienten mit schwerer Sepsis und septischen Schock trotz maximaler Therapie immer noch sehr hoch. Aus diesem Grund sowie aufgrund des immer größer werdenden Problems der Multiresistenz von Bakterien gegen Antibiotika und der damit verbundenen ansteigenden Inzidenz schwerwiegender Krankheitsverläufe besteht weiterhin ein hoher Bedarf an verbesserten Therapieformen und effizienteren extrakorporalen Perfusionsvorrichtungen, die zudem in der klinischen Anwendung unbedenklich sind.

Es ist daher eine Aufgabe der Erfindung, eine extrakorporale Perfusionsvorrichtung der eingangs genannten Art zur Verfügung zu stellen, mit der eine verbesserte Behandlung einer Sepsis und Sepsis-ähnlichen Zuständen möglich ist.

Die Aufgabe wird durch eine extrakorporale Perfusionsvorrichtung der eingangs genannten Art gelöst, die erfindungsgemäß dadurch gekennzeichnet ist, dass die Perfusionsvorrichtung ein Abgabemittel zum Zuführen eines endotoxinbindenden Lipopeptids in den extrakorporalen Blutkreislauf umfasst, wobei das endotoxinbindende Lipopeptid aus der Gruppe bestehend aus Polymyxinen, Polymyxin-Derivaten, deren Prodrugs und einer Kombination davon ausgewählt ist.

Dank der Erfindung ist eine verbesserte Behandlung von Sepsis und Sepsis-ähnlichen Zuständen im Vergleich zu den bisher bekannten Therapieansätzen möglich.

Ein erster großer Vorteil der erfindungsgemäßen Perfusionsvorrichtung liegt darin, dass der Filter nicht nur eine Barriere für Endotoxine und andere hochmolekulare Plasmabestandteile, sondern auch eine für die gebildeten Endotoxin-Lipopeptid-Komplexe darstellt, so dass im Blut des Patienten vorliegende Endotoxin-Lipopeptid-Komplexe, die vor deren Abbau in der Leber im extrakorporalen Blutkreislauf zirkulieren, nicht in den Filtratkreislauf und zum Träger gelangen können. Ein Kontakt mit dem Träger würde aufgrund von kompetitiven Wechselwirkungsvorgängen zwischen Komplex und erster Trägeroberfläche zu einem Auflösen des Endotoxin-Lipopeptid-Komplexes führen, wodurch sich eine Verschlechterung des Zustandes eines Patienten mit einer Sepsis ergeben kann. Die erneute Zufuhr von Endotoxinen bedingt durch die Dissoziation der Lipopeptid-Endotoxin-Komplexe ruft eine erneute Verstärkung der durch Endotoxine hervorgerufenen Aktivierungs-Prozesse des Komplement- bzw. Gerinnungssystem sowie zellulärer Systeme (Monozyten) hervor, die mit den entsprechenden klinischen Folgen wie der Initiierung bzw. Verstärkung eines Multiorganversagens bzw. der Initiierung des anergischen d.h. abwehrgeschächten Stadiums der Sepsis, verbunden sind. Die Konsequenz hieraus bedeutet, dass die Freisetzung von Endotoxinen in jedem Fall verhindert werden sollte.

Dank der Erfindung können durch das Abgabemittel endotoxinbindende Lipopeptide dem Blut zugeführt und Endotoxine durch Komplex-Bildung eliminiert werden, und gleichzeitig kann eine Abreicherung unerwünschter Blutbestandteile, insbesondere Zytokine, durch das Abreicherungsmittel erfolgen, wodurch eine maximale Therapie ohne zusätzliches Sicherheitsrisiko für den Patienten möglich ist.

Ein weiterer wichtiger Vorteil der erfindungsgemäßen Perfusionsvorrichtung liegt ferner darin, dass durch das im Filtratkreislauf angeordnete Abreicherungsmittel unerwünschte Blutbestandteile, insbesondere Zytokine, durch Adsorption an die Oberfläche des ersten Trägers aus dem Blutplasma entfernt werden. Die Erfinder konnten den überraschenden Sachverhalt feststellen, dass die Adsorptionsleistung für Zytokine, allen voran TNF-$\alpha$, bei Verwendung des erfindungsgemäß eingesetzten Filters signifikant besser ist im Vergleich zu einem Plasmafilter, der nur zelluläre Blutbestandteile zurückhält, obwohl weniger Zytokine aus dem extrakorporalen Blutkreislauf durch den Filter in das im Filtratkreislauf geführte fraktionierte Plasma gelangen. Der erfindungsgemäß eingesetzte Filter verhindert die Permeation von Proteinen bzw Lipo- und Glykoproteinen mit einer relativen Molmasse über 300.000 nahezu vollständig. Es wurde festgestellt, dass dieser Vorteil eine wesentlich bessere Reproduzierbarkeit der Zytokin-Eliminierung im Vergleich zur Nutzung eines Plasmafilters, der nur zelluläre Bestandteile zurückhält, garantiert.

Der erfindungsgemäß eingesetzte Filter lässt fraktioniertes Blutplasma durch, so dass hochmolekulare Blutbestandteile, Endotoxine sowie Endotoxin-Lipopeptid-Komplexe zurückgehalten werden, während kleinere Blutbestandteile die Filtermembran passieren können. Ein geeigneter Filter ist unter dem Handelsnamen "Albuflow®" erhältlich (Hersteller: Fresenius Medical Care; Material: Polysulfon-Hohlfasern; Siebkoeffizient für Albumin von $\geq$ 0,6 und für Fibrinogen $\leq$ 0,1).

Der hierin verwendete Begriff "Blutplasma", sofern es im Filtratkreislauf der erfindungsgemäßen Vorrichtung geführt ist, bezieht sich daher auf fraktioniertes Blutplasma.

Der Begriff "Träger mit einer neutralen, hydrophoben Oberfläche" bezieht sich im Rahmen dieser Offenbarung auf einen wasserunlöslichen Feststoff, der eine neutrale und hydrophobe Oberfläche aufweist. Unter dem Begriff "neutral" ist nicht-ionisch zu verstehen. Der Träger kann faserförmig oder partikelförmig sein. Der Träger kann ferner porös sein und äußere und innere Oberflächen aufweisen. Die äußeren und inneren Oberflächen sind neutral und hydrophob. Unter dem Begriff "innere Oberfläche" des Trägers wird die Gesamtheit der Oberflächen der Poren bezeichnet. Der Begriff "äußere Oberfläche" bezieht sich hingegen auf die Gesamtheit der Oberflächen des Trägers, die von außerhalb direkt zugänglich sind.

Die Begriffe "Polymyxin' bzw. "Polymyxine" wie hierin verwendet beziehen sich auf bekannte, natürlich vorkommende chemische Verbindungen, welche ursprünglich aus dem Bakterium *Bacillus polymyxa* (Polymyxin B) sowie *Bacillus colistinus* (Polymyxin E) stammen. Die Polymyxine können entweder aus Bakterien isoliert oder auf synthetischem Weg hergestellt sein. Das aus dem Bakterium stammende Polymyxin B setzt sich aus 6 Derivaten zusammen, die mit Polymyxin B1, Polymyxin B2, Polymyxin B3, Polymyxin B4, Polymyxin B5 und Polymyxin B6 bezeichnet werden. Im Vergleich dazu setzt sich das von der FDA zur parenteralen Infusion zugelassene Polymyxin nur aus Polymyxin B1 bis B4 zusammen. Klinisch relevant sind wie erwähnt bislang nur Polmyxin B und Polymyxin E (Colistin).

Der Begriff "Prodrug" wie hierin verwendet, bezieht sich auf Vorläuferverbindungen von Polymyxinen wie oben definiert, wobei die Vorläuferverbindungen *in vivo* in das aktive Polymyxin umgewandelt werden. Als repräsentative Beispiele sind die Prodrugs Colistin-Methansulfonat und Polymyxin B-Methansulfonat-Natrium zu nennen.

Der Begriff "Polymyxin-Derivat" bezieht sich auf eine von Polymyxin abgeleitete Verbindung, die durch Modifikation natürlich vorkommender Polymyxine erhältlich ist, beispielsweise durch chemische Modifikation der Dab-Seitenketten, des zyklischen Peptidrings oder der Fettsäurekette der Polymyxin-Molekülstruktur. Eine detaillierte Übersicht über Polymyxin-basierte Antibiotika, Analoga und Derivate ist in der Publikation von Velkov *et al.* beschrieben (Velkov et al. 2010. Journal of Medicinal Chemistry, 53(5):1898-1916). Die Eignung eines Polymyxin-Derivats zur Anwendung in der vorliegenden Erfindung kann von einem Fachmann auf dem Gebiet anhand einfacher Routineversuche ausgetestet werden. Der Begriff "Endotoxinämie" wird hierin für sämtliche Krankheitsbilder verwendet, bei welchen sich klinisch relevante Mengen an Endotoxinen im Blut des Patienten befinden, welche in weiterer Folge zu Krankheitsbildern wie Sepsis und SIRS führen.

Der Begriff "Abreicherungsmittel" bezieht sich auf ein Mittel, mit welchem unerwünschte Bestandteile aus dem im Filtratkreislauf geführten Blutplasma entfernt werden können. Abreicherungsmittel, die einen Träger mit einer neutralen, hydrophoben Oberfläche aufweisen, haben sich in der Vergangenheit als besonders günstig für die Eliminierung von Entzündungsmediatoren wie Zytokine durch Adsorption an deren Trägeroberfläche herausgestellt. Dabei handelt es sich mit Vorteil um potentiell schädliche proinflammatorische Zytokine. Repräsentative Beispiele für proinflammatorische Zytokine sind TNF-$\alpha$, IL-1$\beta$, IL-6 und IL-8, wobei TNF-$\alpha$ als initialem proinflammatorischen Entzündungsmediator besondere Bedeutung zukommt. Das erfindungsgemäß eingesetzte Abreicherungsmittel ist daher besonders günstig zum Behandeln von Erkrankungen und Zuständen, die auf die toxischen Wirkungen von TNF-$\alpha$ zurückzuführen sind. Beispielsweise liegen bei der Sepsis die Werte für das TNF-$\alpha$ in der proinflammatorischen Phase zumindest über 100-200 ng/ml. Die unten angeführten Beispiele liefern den Beweis, dass TNF-$\alpha$, IL-1$\beta$, IL-6, IL-8 sowie das antiinflammatorische IL-10 höchst effizient eliminiert werden und die erfindungsgemäße Vorrichtung zur Behandlung von Sepsis, septischem Schock und sepsis-ähnlichen Zuständen außerordentlich gut geeignet ist.

Der Begriff "Abgabemittel zum Zuführen eines endotoxinbindenen Lipopeptids in den extrakorporalen Blutkreislauf" bezieht sich einerseits auf Abgabemittel zum Zuführen des Lipopeptids direkt in den extrakorporalen Blutkreislauf. Andererseits bezieht sich dieser Begriff auch auf Abgabemittel zum indirekten Zuführen des Lipopeptids in den extrakorporalen Blutkreislauf, indem das Lipopeptid durch das Abgabemittel in den Filtratkreislauf abgegeben wird und das Lipopeptid dann von dort weiter in den extrakorporalen Blutkreislauf gelangt.

Da natürlich vorkommende Polymyxine, welche ursprünglich aus den Bakterien *Bacillus polymyxa* sowie *Bacillus colistinus* stammen, zu den am besten untersuchten Peptidantibiotika gehören und bereits seit Jahrzehnten in der Behandlung von Erkrankungen und Zuständen infolge einer Endotoxinämie zur Anwendung kommen, ist es bevorzugt, wenn das endotoxinbindende Lipopeptid ein Polymyxin ist. Besonders bevorzugt ist das Lipopeptid ausgewählt aus der Gruppe bestehend aus den bislang einzigen für die klinische Anwendung zugelassenen Polymyxinen Polymyxin B und Colistin (Polymyxin E) sowie deren Prodrugs. Als repräsentative Beispiele sind die Prodrugs Colistin-Methansulfonat und Polymyxin B-Methansulfonat-Natrium zu nennen. Am meisten bevorzugt wird jedoch Polymyxin B und dessen Prodrugs, da es sich für die Anwendung im humanmedizinischen Bereich am besten bewährt hat.

Eine erste vorteilhafte Ausführungsform sieht vor, dass das Abreicherungsmittel das Abgabemittel zum Zuführen des endotoxinbindenden Lipopeptids umfasst, wobei die Oberfläche des ersten Trägers des Abreicherungsmittels eine adsorptive Beschichtung aus dem endotoxinbindenden Lipopeptid aufweist. Bei dieser Ausführungsform fungiert das Abreicherungsmittel also auch als Abgabemittel für das endotoxinbindende Lipopeptid, indem der erste Träger adsorptiv mit dem Lipopeptid beschichtet ist. Das Lipopeptid wird fortlaufend durch Desorption in geringer Menge in den Filtratkreislauf freigesetzt und wird von dort weiter dem extrakorporalen Blutkreislauf zugeführt. Die Zufuhr des Lipopeptids in den extrakorporalen Blutkreislauf erfolgt also durch Abgabe des Lipopeptids (Desorption vom ersten Träger) in den Filtratkreislauf, von dem es weiter in den Blutkreislauf gelangt. Bei einer Untervariante kann der Filtratkreislauf als offener Filtratkreislauf realisiert sein, der stromab des Filters in den extrakorporalen Blutkreislauf mündet. Bei einer anderen Untervariante kann der Filtratkreislauf als ein im Filtratbereich geschlossener Kreislauf realisiert sein, der in den Filter mündet. In Laborversuchen hat sich herausgestellt, dass die adsorptive Beschichtung der Trägeroberfläche mit dem Lipopeptid keinerlei nachteilige Auswirkungen auf die Adsorption der Zytokine hat (siehe Beispiel 11 unten).

Unter dem in dieser Offenbarung verwendeten Begriff "adsorptive Beschichtung" ist zu verstehen, dass die endotoxinbindenden Lipopeptide über nichtkovalente, adsorptive Vorgänge und Wechselwirkungen an der neutralen, hydrophoben Trägeroberfläche binden. Es ist anzunehmen, dass insbesondere die hydrophobe Interaktion, auch hydrophobe Wechselwirkung genannt, eine wichtige Rolle spielt. Die hydrophobe Interaktion besitzt große biochemische Bedeutung und beruht auf dem Phänomen, dass hydrophobe Moleküle in einer polaren Umgebung zur Assoziation neigen. Die hydrophobe Interaktion ist daher keine Kraft *per se,* sondern wird durch eine polare Umgebung erzwungen. Auch andere nicht-kovalente Interaktionen, einschließlich, ohne Beschränkung, ionischer Bindungen, Wasserstoffbrückenbindungen und van der Waals Wechselwirkungen können bei der Adsorption endotoxinbindener Lipopeptide wie Polymyxin eine Rolle spielen. Die Bindung von endotoxinbindenden Lipopeptiden wie Polymyxin über nicht-kovalente Wechselwirkungen an hydrophobe Trägeroberflächen verschiedener Poren- und Partikelgrößen wurde bereits in der WO 2010/083545, der WO 2011/160149, der WO 2007/142611 A1 und der US 5510242 beschrieben. Das auf dem Träger adsorptiv gebundene endotoxinbindende Lipopeptid ist erfindungsgemäß aus der Gruppe bestehend aus Polymyxinen, deren Prodrugs und einer Kombination davon ausgewählt.

Alternativ zur ersten Ausführungsform ist bei einer zweiten vorteilhaften Ausführungsform das Abgabemittel zum Zuführen des endotoxinbindenden Lipopeptids im Filtratkreislauf stromab des Abreicherungsmittels angeordnet, wobei das Abgabemittel einen zweiten Träger mit einer neutralen, hydrophoben Oberfläche umfasst, wobei die Oberfläche des zweiten Trägers eine adsorptive Beschichtung aus dem endotoxinbindenden Lipopeptid aufweist. Das Lipopeptid wird fortlaufend durch Desorption in geringer Menge in den Filtratkreislauf freigesetzt und wird von dort weiter dem extrakorporalen Blutkreislauf zugeführt. Die Zufuhr des Lipopeptids in den extrakorporalen Blutkreislauf erfolgt also durch Abgabe des Lipopeptids (Desorption vom zweiten Träger) in den Filtratkreislauf, von dem es weiter in den Blutkreislauf gelangt. Bei einer Untervariante der zweiten Ausführungsform kann der Filtratkreislauf als offener Filtratkreislauf realisiert sein, der stromab des Filters in den extrakorporalen Blutkreislauf mündet. Bei einer anderen Untervariante der zweiten Ausführungsform kann der Filtratkreislauf als ein im Filtratbereich geschlossener Kreislauf realisiert sein, der in den Filter mündet. Aufgrund des geringeren konstruktiven/apparativen Aufwands wird die erste Ausführungsform jedoch gegenüber der zweiten Ausführungsform bevorzugt.

Die Entwicklung der oben genannten beiden Ausführungsformen (erste und zweite Ausführungsform) der erfindungsgemäßen Perfusionsvorrichtung basiert auf dem überraschenden Sachverhalt, dass bei Trägern mit einer neutralen, hydrophoben Oberfläche, die eine adsorptive Beschichtung aus Polymyxin aufweisen, die Endotoxineliminierung nicht wie bisher angenommen durch Adsorption der Endotoxine an die am Träger immobilisierten Polymyxin-Moleküle erfolgt, sondern über eine sehr geringe Menge an desorbierten und in das Blut bzw. Blutplasma übergegangenen Polymyxin-Molekülen. Diese überraschende und unvorhersehbare Erkenntnis basiert auf der Tatsache, dass nach gezieltem Waschen eines mit Polymyxin adsorptiv beschichteten Trägers keine Endotoxinadsorption durch die noch an der Trägeroberfläche immobilisierten Polymyxin-Moleküle festgestellt werden konnte. Die Erfinder konnten daher feststellen, dass die hervorragende Endotoxineliminierungseffizienz von neutralen, hydrophoben Trägerpolymeren, auf deren Oberflächen Polymyxin adsorptiv gebunden ist, auf eine sehr geringe, vom Trägermaterial desorbierte und in die Körperflüssigkeit, d.h. Blut bzw. Blutplasma, freigesetzte Menge an freien Polymyxin-Molekülen zurückzuführen ist. Ebenso überraschend war die Erkenntnis, dass die geringen Mengen an freigesetztem Polymyxin, die in Abhängigkeit der am Träger adsorbierten Polymyxin-Menge eine Polymyxin-Serumkonzentration von ungefähr 0,01 μg/ml bis ungefähr 0,8 μg/ml ergeben, bereits ausreichend sind, um Endotoxine in ihrer Aktivität zu hemmen, wobei neuro- und nephrotoxische Nebenwirkungen auszuschließen sind.

Nur aufgrund dieser überraschenden Erkenntnis war es den Erfindern möglich, die ersten und zweiten vorteilhaften Ausführungsformen der erfindungsgemäßen Perfusionsvorrichtung zu entwickeln. Davor war man immer davon ausgegangen, dass die Endotoxineliminierung durch Bindung der Endotoxine an die am Träger adsorbierten Polymyxin-Moleküle erfolgt. In Anbetracht der Tatsache, dass ein Filter mit einem Siebkoeffizienten von 5% für Substanzen mit einer molaren Masse von 340 000 g/mol (relative Molekülmasse von 340 kDa) eine Barriere für Endotoxine (LPS) darstellt und die Endotoxine somit nicht zu einem im Filtratkreislauf angeordneten Adsorbermaterial für Endotoxine gelangen können, hätte man erst in Kenntnis dieses neuen überraschenden Sachverhalts einen Anreiz darin gesehen, einen Träger, der eine adsorptive Beschichtung mit einem endotoxinbindenen Lipopeptid aufweist und der eine vorgebbare Menge an Lipopeptid in das Blutplasma abgibt, mit einem solchen Filter zu kombinieren. Durch das im Filtratkreislauf angeordnete Abgabemittel wird daher eine anhaltende Freisetzung sehr geringer, und vor allem gleichmäßiger Mengen endotoxinbindender Lipopeptide in das im Filtratkreislauf geführte Blutplasma über den gesamten Behandlungszeitraum, vorzugsweise von 4 bis 10 Stunden täglich über einen Zeitraum von 2 bis 8 Tagen, erreicht. Von dort gelangen die Lipopeptide in den extrakorporalen Blutkreislauf, wo sie mit den im Blut befindlichen Endotoxinen (LPS) einen Komplex bilden und diese daher unschädlich machen. Dank des Filters können diese Komplexe wie oben bereits beschrieben nicht mehr in den Filtratkreislauf gelangen und wieder aufgelöst werden, wodurch die Patientensicherheit hoch gehalten wird. Die Endotoxin-Lipopeptid-Komplexe werden anschließend vorwiegend in der Leber des Patienten abgebaut.

Mit Vorteil liegt das an der Oberfläche des ersten bzw. zweiten Trägers adsorbierte endotoxinbindende Lipopeptid in einer Menge vor, die bei Abgabe des Lipopeptids eine Lipopeptid-Serumkonzentration von 0,01 μg/ml bis 0,8 μg/ml ergibt. Es wurde wie bereits erwähnt der überraschende Sachverhalt festgestellt, dass die sehr geringe Desorption des Lipopeptids vom ersten bzw. zweiten Träger ausreichend ist, um Lipopeptid-Serumkonzentrationen von 0,01μg/ml bis 0,8 μg/ml zu erhalten. Es wurde festgestellt, dass bei diesen Serumkonzentrationen Endotoxine in ihrer Aktivität gehemmt werden, wobei neuro- und nephrotoxische Wirkungen auszuschließen sind. Vorzugsweise liegt die Lipopeptid-Serumkonzentration in einem Bereich von 0,1 μg/ml bis 0,6 μg/ml, vorzugsweise 0,1 μg/ml bis 0,4 μg/ml, am meisten bevorzugt zwischen 0,1 μg/ml bis 0,25 μg/ml, da bei diesen Serumkonzentrationen auch bei schwerwiegenden Krankheitsverläufen wie einer Sepsis, einer schwerer Sepsis oder einem septischen Schock eine effiziente Therapie ohne neuro- und nephrotoxische Nebenwirkungen durchführbar ist.

Vorzugsweise weist der erste bzw. zweite Träger eine Gesamtoberfläche von 100 bis 1500 m$^2$/g auf, wobei 50 bis 2000 mg an endotoxinbindenden Lipopeptid in Bezug auf die Trägergesamtoberfläche adsorptiv an der Oberfläche des ersten bzw. zweiten Trägers gebunden sind. Auf diese Weise kann durch Desorption des endotoxinbindenden Lipopeptids von der Trägeroberfläche eine Lipopeptid-Serumkonzentration von ca. 0,01 μg/ml bis ca. 0,8 μg/ml erhalten werden. Für einen Fachmann auf dem Gebiet lässt sich die zu erwartende Lipopeptid-Serumkonzentration in Bezug auf den verwendeten Träger unter Berücksichtigung der mittleren Porengröße und/oder mittleren Partikelgröße anhand von einfachen Routineversuchen und Berechnungen ermitteln; Berechnungsbeispiele sind weiter unten in den Beispielen angeführt.

Alternativ zu den oben genannten Ausführungsformen, die auf der Desorption sehr geringer Mengen des Lipopeptids von einer Trägeroberfläche basieren, sieht eine dritte vorteilhafte Ausführungsform vor, dass das Abgabemittel zum Zuführen des endotoxinbindenden Lipopeptids eine Dosiereinrichtung zum Zuführen des endotoxinbindenden Lipopeptids in den extrakorporalen Blutkreislauf an einer dem extrakorporalen Blutkreislauf zugeordneten Lipopeptidzufuhrstelle umfasst. Die Lipopeptidzufuhrstelle ist vorzugsweise stromab des Filters angeordnet. Ist im extrakorporalen Blutkreislauf stromab des Filters zusätzlich ein Dialysator angeordnet, dann ist es günstig, wenn die Lipopeptidzufuhrstelle im extrakorporalen Blutkreislauf stromab des Dialysators angeordnet ist. Bei einer Untervariante der dritten Ausführungsform kann der Filtratkreislauf als offener Filtratkreislauf realisiert sein, der stromab des Filters in den extrakorporalen Blutkreislauf mündet. Bei einer anderen Untervariante der dritten Ausführungsform kann der Filtratkreislauf als ein im Filtratbereich geschlossener Kreislauf realisiert sein, der in den Filter mündet.

Bei dieser Ausführungsform wird das Lipopeptid mittels einer Dosiereinrichtung in den extrakorporalen Blutkreislauf infundiert. Das Lipopeptid liegt vorzugsweise in Form einer Zubereitung zur parenteralen Verabreichung vor, z.B. als Infusionslösung. Die Zubereitung kann optional zumindest einen pharmazeutisch annehmbaren Träger und/oder Hilfsstoff umfassen. Die Zubereitung kann nur eine Art eines endotoxinbindenden Lipopeptids oder eine Mischung von zwei oder mehr Lipopeptiden umfassen, beispielsweise eine Mischung aus Polymyxin B1, B2, B3 und B4. Ein "pharmazeutisch annehmbarer Träger und/oder Hilfsstoff" kann jede Substanz sein, die zur Herstellung von parenteralen Verabreichungsformen wie Injektionen, Infusionslösungen und dergleichen bekannt ist. Für die Erfindung geeignete Formulierungen für Infusionslösungen sind weiter unten in den Beispielen 4 und 5 angeführt.

Vorzugsweise liegt das endotoxinbindende Lipopeptid in Form eines lyophilisierten Pulvers zur Herstellung einer sterilen wässrigen Injektionszubereitung bzw. Infusionszubereitung vor, wobei das Pulver beispielsweise in sterilem Wasser, 5 %iger Dextroselösung, einer Ringerlösung oder einer physiologischen Natriumchloridlösung gelöst werden kann. Polymyxin B kommt vorzugsweise in Form von Polymyxin B-Sulfat zum Einsatz. Das Lipopeptid liegt in der Zubereitung in gelöster Form vorzugsweise in einer Konzentration von 0,04 mg/l bis 13 mg/l, mehr bevorzugt von 0,1 mg/l bis 7 mg/l, am meisten bevorzugt von 0,5 mg/l bis 4 mg/l vor. Vorzugsweise ist die Dosierung so eingestellt, dass die Lipopeptid-Serumkonzentration in einem Bereich von 0,1 $\mu$g/ml bis 0,6 $\mu$g/ml, vorzugsweise 0,1 $\mu$g/ml bis 0,4 $\mu$g/ml, am meisten bevorzugt zwischen 0,1 $\mu$g/ml bis 0,25 $\mu$g/ml liegt, da bei diesen Serumkonzentrationen auch bei schwerwiegenden Krankheitsverläufen wie einer Sepsis, einer schwerer Sepsis oder einem septischen Schock eine effiziente Therapie ohne neuro- und nephrotoxische Nebenwirkungen durchführbar ist.

Die Dosiereinrichtung ist durch dem Fachmann an sich bekannte Infusionsgeräte realisiert, die typischerweise ein Behältnis mit der Infusionslösung (z.B. Infusionsbeutel oder Infusionsflasche), ein Schlauchsystem und ein Pumpmittel zur Dosierung eines gewünschten Volumens pro Zeiteinheit umfasst.

Die Infusionsrate ist von der Serumhalbwertszeit für das Lipopeptid im Patienten abhängig. Beispielsweise liegt die Serumhalbwertszeit für Polymyxin B bei Patienten mit normaler Nierenfunktion typischerweise bei 13 Stunden, jene für Colistin je nach Literaturangabe bei 6 bis 7,4 Stunden. Bei der Behandlung mittels der Perfusionsvorrichtung ist wie weiter unten im Detail beschrieben auch die Clearance des Filters und/oder die Clearance des Abreicherungsmittels für das verabreichte Lipopeptid zu berücksichtigen. Formulierungen für hierfür geeignete Infusionslösungen sowie Dosieranleitungen sind weiter unten in den Beispielen angeführt.

Um die Konzentration des endotoxinbindenden Lipopeptids zu überwachen und die Dosierung der Infusionslösung entsprechend einstellen zu können, ist es von Vorteil, wenn stromab des Filters bzw. des Dialysators und stromauf der Lipopeptidzufuhrstelle ein Messmittel zur Messung der Konzentration des endotoxinbindenden Lipopeptids angeordnet ist. Geeignete Messmittel, z.B. Sensoren, die hierfür zur Anwendung kommen können, sind im Stand der Technik beschrieben, beispielsweise von Jiang *et al.* (Jiang et al. 2004. A synthetic peptide derived from bactericidal/ permability-increasing protein neutralizes endotoxin in vitro and in vivo. International Immunopharmacology 4:527-537). Vorzugsweise wird für die Messung eine geringe Menge Blut aus dem extrakorporalen Blutkreislauf über eine Abzweigleitung zum Messmittel/Sensor geleitet und nach Konzentrationsbestimmung verworfen. Ein Einsetzen des Messmittels/ Sensors direkt in den extrakorporalen Blutkreislauf ist zwar grundsätzlich möglich, wird jedoch weniger bevorzugt, da sich in diesem Fall hohe Ansprüche an die Beschaffenheit des Messmittels/Sensors hinsichtlich Sterilität und Biokompatibilität ergeben. Aus diesen Gründen ist die Variante mit der Abzweigleitung zum Messmittel/Sensor zu bevorzugen.

Der Perfusionsvorrichtung kann ferner ein mittels der Steuerung geregelter Regelkreis zugeordnet sein, wobei die infundierte Menge des Lipopeptids in Abhängigkeit des durch das Messmittel gemessenen Lipopeptid-Istwerts (Lipopeptid-Serumkonzentration) durch Ansteuern der Infusionspumpe in Bezug auf einen vorgebbaren Sollwert oder Sollwertbereich geregelt wird. Der Sollwert bzw. Sollwertbereich der Lipopeptid-Serumkonzentration liegt typischerweise in einem Bereich von 0,01 - 0,8 $\mu$g/ml.

Bei der dritten Ausführungsform ist es ferner von Vorteil, wenn die Steuerung der Perfusionsvorrichtung dazu eingerichtet ist, bei der Dosierung des Lipopeptids in das im extrakorporalen Blutkreislauf geführte Blut die Lipopeptid-Clearance des Körpers, die Lipopeptid-Clearance des Abreicherungsmittels und/oder die Lipopeptid-Clearance des Dialysators zu berücksichtigen. Beispielsweise ist bekannt, dass Träger aus einem Polystyrol-Divinylbenzol-Copolymer neben pathophysiologisch relevanten Komponenten wie Zytokinen auch Lipopeptide wie Polymyxine adsorbieren, so dass die Berücksichtigung der Lipopeptid-Clearance des ersten Trägers (d.h. Träger des Abreicherungsmittels) von Vorteil für die Dosierung des infundierten Lipopeptids ist.

Alternativ zu den oben genannten Ausführungsformen, ist es bei einer vierten Ausführungsforme vorgesehen, dass das Abgabemittel zum Zuführen des endotoxinbindenden Lipopeptids einen im extrakorporalen Blutkreislauf stromab des Filters angeordneten Dialysator umfasst, der dazu eingerichtet ist, dem extrakorporalen Blutkreislauf das endotoxinbindende Lipopeptid mittels einer durch den Dialysator geführten Dialyseflüssigkeit zuzuführen. Bei einer Untervariante kann der Filtratkreislauf als offener Filtratkreislauf realisiert sein, der stromab des Filters in den extrakorporalen Blutkreislauf mündet. Bei einer anderen Untervariante kann der Filtratkreislauf als ein im Filtratbereich geschlossener Kreislauf realisiert sein, der in den Filter mündet. Bei den verwendeten Dialysatoren handelt es sich vorzugsweise um hydrophile Polysulfon-Membranen mit einer Oberfläche von 1,4 - 2,0 m$^2$, die durch Blending mit PVP (Polyvinyl-Pyrrolidon) hergestellt wurden. Beispielsweise werden diese Membranen in Filtern der Firma Fresenius Medical Care verwendet u.a. in den Typen AF 1000 bzw. FX60. Diese Dialysefilter haben einen Siebkoeffizienten für Albumin unter 0,1 %. Denkbar ist auch die Verwendung eines sogenannten High Cutoff Filters, der ebenfalls auf der Nutzung von hydrophilen Polysulfon-Membranen mit einem Siebkoeffizienten von etwa 4% für Albumin beruht. Unter Dialyse-Bedingungen, d.h. es kommt vorrangig eine diffusionsgesteuerte Elimination der zur Entfernung gedachten Substanzen zum Einsatz, beträgt der Albuminverlust unter 5-10 g pro Behandlung. Als Beispiel für ein derartiges Dialysefilter kann der von der Firma Fresenius Medical Care hergestellte EMiC$^2$ - Filter genannt werden Die Flussbedingungen, mit denen derartige Filter im klinischen Einsatz betrieben werden, sind für den Blutfluss 80 - 300 ml/min je nach Einsatzbedingungen entsprechend gewählt: unter den Bedingungen einer so genannten kontinuierlichen veno-venösen Hämodialyse werden Blutflüsse von 60-80 ml/min genutzt, wohingegen bei der Dialysegeräte-gestützten intermittierenden Hämodialyse Blutflüsse von 150 - 300 ml/min in akuten Fällen, also bei Patienten mit einem akuten Nierenversagen, das auch sehr häufig bei der Sepsis auftritt, verwendet werden. Der Dialysatfluss ist bei der intermittierenden Hämodialyse vorzugsweise auf 500 ml/min eingestellt, wohingegen bei der kontinuierlichen veno-venösen Hämodialyse Dialysatflüsse im Verhältnis von 1 : 1 zum Blutfluss die Regel sind. Die Konzentration des Lipopeptids/Polymyxins in der Dialyseflüssigkeit sollte in dem Bereich von 0,2 - 1,0 µg/l liegen, also leicht höher als der angesteuerte Serumkonzentrationswert des zu behandelnden Patienten liegen, da der Siebkoeffizient der genannten Dialysefilter zwischen 0.8 (AF 1000) und 0.9 (EMiC$^2$) liegt, also zwischen 80 und 90%.

Für die Praxis ist es besonders zweckmäßig, wenn der erste bzw. zweite Träger ein neutrales, vorzugsweise synthetisches, Polymer ist. Dadurch kann eine gute Reproduzierbarkeit des Trägermaterials gewährleistet werden. Handelt es sich um ein poröses Polymer, dann kann eine gute Reproduzierbarkeit insbesondere hinsichtlich der Porosität und der Partikelgröße gewährleistet werden. Die Porosität und die Partikelgröße lassen sich darüber hinaus sehr gut variieren. Bei dem Polymer kann es sich sowohl um ein Homopolymer als auch um ein Heteropolymer handeln. Diese Polymere sind auch unter der Bezeichnung "nichtionische macroretikuläre Polymerharze ("nonionic macroreticular polymer resins") bekannt und beispielsweise unter den Handelsbezeichnungen "Amberchrom" und "Amberlite XAD" (Rohm&Haas/Dow Chemical Company) erhältlich.

Für die praktische Durchführung haben sich vernetzte Polystyrol-Polymere und vernetzte Ethylvinylbenzol-Polymere als besonders günstig erwiesen. Bei der extrakorporalen Blutreinigung bestehen hohe Anforderungen an die Sterilität der Vorrichtungsbestandteile, welche mit den Körperflüssigkeiten des Patienten in Kontakt kommen. Vernetzte Polystyrol-Polymere und vernetzte Ethylvinylbenzol-Polymere zeichnen sich durch eine hohe Stabilität gegenüber Hitze und Chemikalien aus und sind in der klinischen Praxis bereits etabliert. Bei einer vorteilhaften Variante ist das vernetzte Polystyrol-Polymer ein Polystyrol-Divinylbenzol-Copolymer. Bei einer weiteren vorteilhaften Variante ist das vernetzte Ethylvinylbenzol-Polymer ein Ethylvinylbenzol-Divinylbenzol-Copolymer.

Es ist natürlich auch möglich, anstelle des bevorzugten Polystyrol-Divinylbenzol-Copolymers bzw. Ethylvinylbenzol-Divinylbenzol-Copolymers andere Neutralharze hoher Hydrophobizität zu verwenden, welche einem einschlägigen Fachmann gut bekannt sind. Repräsentative Beispiele für andere neutrale, hydrophobe Polymere, die für die Erfindung geeignet sind, sind beispielsweise Polymere aus Styrol- und Ethylvinylbenzol-Monomeren, die mit Trivinylcyclohexan, Trivinylbenzol, Divinylnaphthalen, Divinylsulfon, Thrimethylolpropan-Triacrylat, Trimethylpropan-trimethacrylat vernetzt sind oder Harze auf Basis aliphatischer Ester sowie Mischungen davon.

Mit Vorteil ist der erste bzw. der zweite Träger porös und weist eine mittlere Porengröße von 100 nm oder kleiner auf, vorzugsweise liegt sie in einem Bereich von 1 bis 100 nm. Dadurch wird eine besonders große innere Oberfläche für die Abreicherung unerwünschter Bestandteile wie Zytokine bzw. für die adsorptive Beschichtung mit endotoxinbindendem Lipopeptid geschaffen.

Obwohl einem Fachmann auf dem Gebiet wohlbekannt ist, was unter dem Begriff "mittlere Porengröße" zu verstehen ist und wie die Porosität bzw. die mittlere Porengröße gezielt einstellt werden kann, soll dieser Begriff aus Gründen der Klarheit an dieser Stelle dennoch kurz definiert werden. Die mittlere Porengröße bezieht sich auf den mittleren Durchmesser der Poren. Bei einer gaußschen Größenverteilung der Porendurchmesser eines porösen Materials ist der mittlere Porendurchmesser jener, welchem dem Maximum der Verteilungskurve entspricht. Der mittlere Porendurchmesser kann beispielsweise mittels Stickstoffadsorption (wie beschrieben in Weber et al. 2008; Neutral styrene divinylbenzene copolymers for adsorption of toxins in liver failure. Biomacromolecules 9(4):1322-1328)) oder mittels Quecksilberintrusion bestimmt werden. Eingestellt wird die Porengröße eines Polymers durch Variation der Konzentration der beteiligten Monomere bzw. Co-Monomere, des Lösungsmittels oder des Modulators. Je kleiner die Poren des Polymers gewählt sind, umso größer wird die innere Oberfläche des Polymers, die für die Adsorption von Molekülen - in diesem Fall unerwünschte Blutbestandteile wie Zytokine bzw. endotoxinbindende Lipopeptide - zur Verfügung steht. Je größer die Poren, umso besser ist die Zugänglichkeit der Poren für größere Moleküle. Ein Herstellungsverfahren für ein synthetisches, hydrophobes Polymer definierter Porengröße, wie es für die Erfindung zur Anwendung kommen kann, ist in der oben genannten Publikation von Weber et al. beschrieben.

Die Herstellung von solchen Trägern durch Copolymerisation von monovinyl-und polyvinylaromatischen Monomeren, ausgeführt als Suspensionspolymerisation, ist auch aus der US 4 382 124 bekannt. Die polyvinylaromatischen Monomeren dienen dabei als Vernetzer des Polymeren. Als monovinylaromatische Verbindungen werden z.B. Styrol und/oder Ethylstyrol, bevorzugt Ethylstyrol, eingesetzt. Als polyvinylaromatische Verbindung wird bevorzugt Divinylbenzol eingesetzt. Die Porosität wird durch Zugabe von Porogenen zu den Monomeren erhalten, die nach der Polymerisation wieder entfernt werden. Die Porogene können hydrophobe oder hydrophile Eigenschaften haben. Beispiele sind Toluol und Xylol für hydrophobe und C4-C10-Alkohole für hydrophile Porogene. Es können aber auch Gemische dieser beiden Porogen-Klassen eingesetzt werden. Die Porengrößen der polymeren Träger können in weiten Grenzen durch den Vernetzungsgrad des Polymeren, die Art und Menge des Porogens und die Reaktionsbedingungen bei der Polymerisation variiert werden. Dem einschlägigen Fachmann ist bekannt, welche Parameter er zu wählen hat, um einen neutralen, hydrophoben Träger gewünschter Porengröße zu erhalten.

Eine spezielle Ausführung der Herstellung von porösen hydrophoben Trägern besteht darin, dass bevorzugt Styrol-Divinylbenzol-Copolymerisate durch "Hypercrosslinking' nachvernetzt werden. (Davankov et al. J.Polymer Science, 47, 95-101 (1974). Beispiele für solche Träger sind die Hypersol-Macronet Sorbents von der Purolite Company. Auf diese Weise können Träger mit sehr großer innerer Oberfläche hergestellt werden, die praktisch nur Mikroporen <2 nm enthalten.

Träger mit neutraler, hydrophober Oberfläche und variabler mittlerer Porengröße und Partikelgröße können beispielsweise von Rohm&Haas/Dow Chemical Company bezogen werden und sind unter den Handelsbezeichnungen "Amberchrom CG" und "Amberlite XAD" erhältlich. Feinporige Träger mit neutraler, hydrophober Oberfläche und einer mittleren Porengröße von ca. 2,5 nm und kleiner sind von der Firma Purolite erhältlich (z.B. Hypersol-Macronet MN270, Polystyrol-Divinylbenzol-Polymer mit einer mittleren Porengröße von 2,5 nm (25 Å)).

Die Totalporosität eines Trägers mit einer mittleren Porengröße von 1 bis 100 nm beträgt vorzugsweise 0,3 - 0,8 $cm^3$/g Polymer. Die inneren Oberflächen (oder Gesamtoberflächen) liegen bevorzugt in einem Bereich von 100 bis 1500 $m^2$/g.

Mit Vorteil liegt die mittlere Porengröße des ersten bzw. des zweiten Trägers in einem Bereich von 20 nm oder kleiner, vorzugsweise 1 bis 20 nm, oder in einem Bereich von 80 bis 100 nm, da in diesen spezifischen Porengrößenbereichen eine Adsorption von Protein C durch den ersten Träger bzw. den zweiten Träger deutlich geringer ausgeprägt ist als in einem Porengrößenbereich von größer als 20 nm und kleiner als 80 nm. Bei Porengrößen < 20 nm gelangt wenig Protein C in die Poren hinein. Die Protein C-Adsorption durch den Träger steigt bei zunehmender Porengröße an und nimmt bei Porengrößen größer als 80 nm wieder ab. Obwohl die Protein C-Bindung bei größer gewählten Porengrößen (größer als 80 nm) bis auf ein Minimum gesenkt werden kann, ist es für die klinische Anwendung günstig, wenn die mittlere Porengröße nicht größer als 100 nm gewählt ist, da die innere Oberfläche des Trägers ansonsten zu klein werden kann. Protein C, ein Vitamin K-abhängiges Protein im Blutplasma, kann den erfindungsgemäß eingesetzten Filter passieren und kommt folglich mit dem im Filtratkreislauf angeordneten ersten bzw. zweiten Träger in Kontakt. Protein C ist ein wichtiger Regulator des Blutgerinnungsablaufs und hat eine antikoagulatorische Wirkung. Es kann davon ausgegangen werden, dass auch die Adsorption von Protein S deutlich niedriger ist, da Protein S (62 000 Da) ein ähnliches relatives Molekulargewicht wie das Protein C (69 000 Da) aufweist. Dieselben Überlegungen gelten für Gerinnungsfaktoren ähnlichen Molekulargewichts wie z.B. Faktor VII, Faktor IX und Faktor X.

Die Totalporosität eines Trägers mit einer mittleren Porengröße von 20 nm oder kleiner beträgt vorzugsweise 0,4 bis 0,8 $cm^3$/g Polymer. Die inneren Oberflächen (oder Gesamtoberflächen) liegen bevorzugt in einem Bereich von 300 bis 1500 $m^2$/g.

Die Totalporosität eines Trägers mit einer mittleren Porengröße von 80 bis 100 nm beträgt vorzugsweise 0,4 bis 0,8 $cm^3$/g Polymer. Die inneren Oberflächen (oder Gesamtoberflächen) liegen bevorzugt in einem Bereich von 100 bis 500 $m^2$/g.

Bei weiteren Varianten kann der erste bzw. der zweite Träger faserartig oder partikelförmig sein. Vorzugsweise sind der erste bzw. der zweite Träger jedoch partikelförmig. Partikelförmige Träger sind leichter zu handhaben als faserförmige Träger. Zudem lässt sich die Porosität von Partikeln leichter her- und einstellen.

Die mittlere Partikelgröße polymerer Träger kann während der Polymerisation in bekannter Weise, z.B. durch Art und Menge des Suspensionsstabilisators und Geometrie und Drehzahl des Rührers, eingestellt werden. Hydrophobe Träger mit neutraler, hydrophober Oberfläche und variabler mittlerer Porengröße und mittlerer Partikelgröße können beispielsweise von Rohm&Haas/Dow Chemical Company bezogen werden und sind unter den Handelsbezeichnungen "Amberchrom CG" und "Amberlite XAD" erhältlich. Feinporige Träger variabler mittlerer Porengröße und mittlerer Partikelgröße werden beispielsweise von der Firma Purolite hergestellt.

Als Beispiel für einen geeigneten faserartigen Träger sind AC-Fasern der Firma Mast Carbon (UK) zu nennen.

Vorzugsweise weist der erste bzw. der zweite Träger die Form von Mikropartikeln mit einer mittleren Partikelgröße von 300 μm oder kleiner, vorzugsweise 2 bis 300 μm auf. Bei aus diesem Bereich größer gewählten Partikelgrößen ergibt sich durch die kleinere äußere Oberfläche eine bessere Blutverträglichkeit, wohingegen sich kleinere Partikel durch eine höhere dynamische Effektivität auszeichnen.

Bei einer besonders bevorzugten Ausführungsform weist der erste Träger eine mittlere Porengröße von 10 bis 20 nm oder eine mittlere Porengröße von 80 bis 100 nm und eine mittlere Partikelgröße von 75 bis 150 μm auf. Es hat sich herausgestellt, dass sich dieser Partikelgrößenbereich als vorteilhaft hinsichtlich der unerwünschten Adsorption von Protein C auswirkt (siehe unten Beispiel 8). Dadurch können Gerinnungskomplikationen (Venenthrombosen, Lungenembolien), die durch eine unerwünschte Protein C-Adsorption hervorgerufen werden, minimiert werden. Bei einer Partikelgröße von 75 bis 150 μm scheint die äußere Oberfläche des Trägers noch klein genug zu sein, um Protein C, wenn überhaupt, nur in einem unwesentlichen Ausmaß zu binden; allerdings ist sie offenbar immer noch groß genug, um die Diffusionswege für die zu adsorbierenden Substanzen klein zuhalten. Die Partikelgröße beträgt bei dieser bevorzugten Ausführungsform zumindest 75 $\mu$m, da hier noch gerade physiologisch relevante Mengen an Protein C auch nach längerer Inkubation im Blut bzw. Blutplasma verbleiben. Bei einer vorteilhaften Untervariante weist der erste Träger eine mittlere Porengröße von 10 bis 20 nm und eine mittlere Partikelgröße von 75 bis 150 $\mu$m auf. Bei einer mittleren Porengröße von weniger als 10 nm sinkt die Adsorptionseffizienz für die zu entfernenden Entzündungsmediatoren wie Zytokine, insbesondere TNF-$\alpha$, wieder. Bei einer Porengröße von mehr als 20 nm wird die innere Oberfläche kleiner und die Adsorptionskapazität für Zytokine sinkt. Diese Ausführungsform der erfindungsgemäßen Perfusionsvorrichtung samt den nachfolgend beschriebenen vorteilhaften Untervarianten ist daher zur Behandlung von Sepsis, insbesondere septischem Schock, und Sepsis-ähnlichen Zuständen außerordentlich gut geeignet.

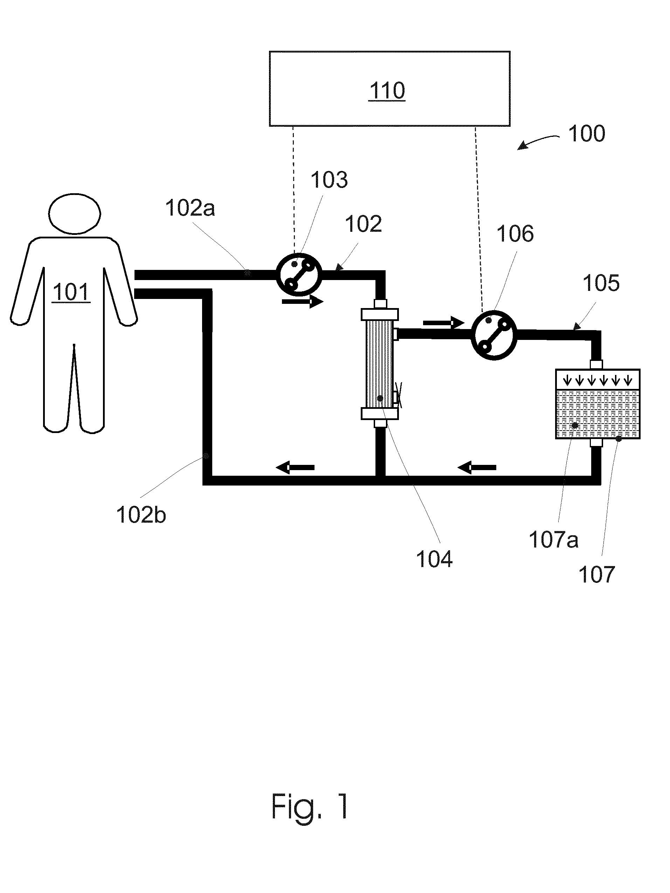

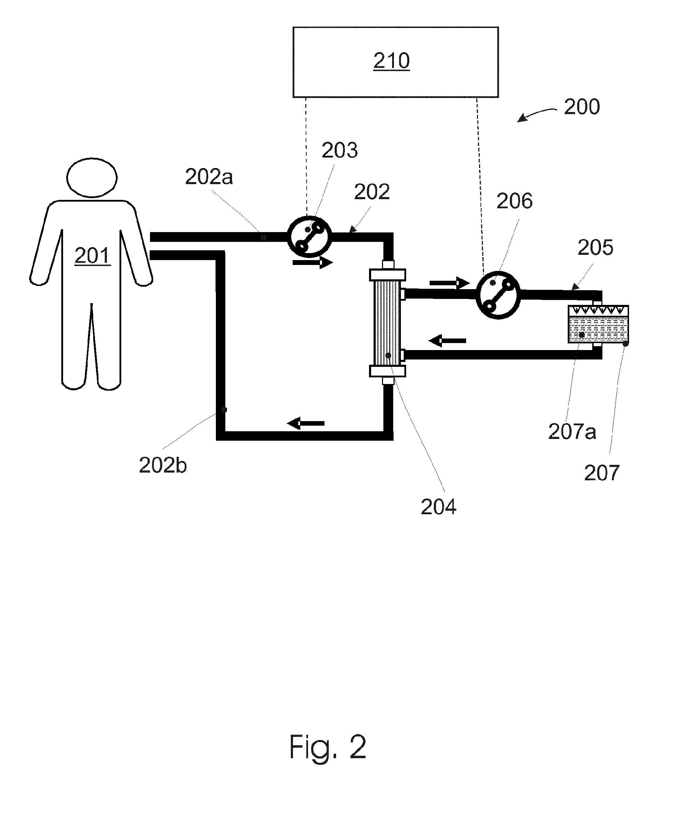

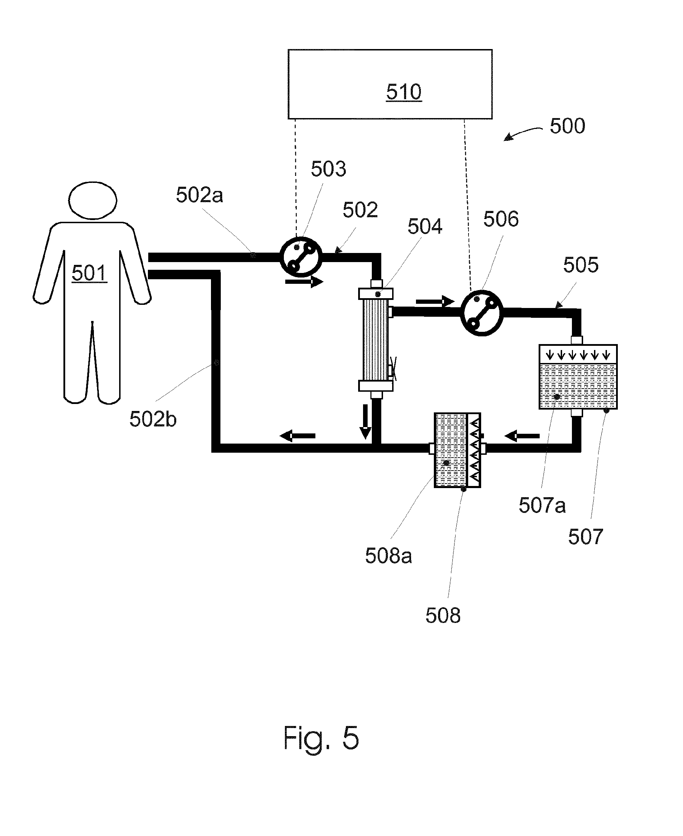

Bei einer besonders vorteilhaften und bevorzugten Untervariante dieser Ausführungsform weist der erste Träger eine mittlere Porengröße von 10 bis 20 nm oder eine mittlere Porengröße von 80 bis 100 nm, vorzugsweise eine mittlere Porengröße von 10 bis 20 nm, und eine mittlere Partikelgröße von 75 bis 150 $\mu$m auf, wobei die Oberfläche des ersten Trägers eine adsorptive Beschichtung aus dem endotoxinbindenden Lipopeptid aufweist, d.h. bei dieser Untervariante fungiert das Abreicherungsmittel wie oben zur ersten Ausführungsform im Detail beschrieben auch als Abgabemittel für das endotoxinbindende Lipopeptid (siehe auch Fig. 1 und 2 weiter unten). Wie bereits erwähnt hat sich in Laborversuchen herausgestellt, dass die adsorptive Beschichtung der Trägeroberfläche mit dem Lipopeptid keinerlei nachteilige Auswirkungen auf die Adsorption der Zytokine hat. Bei Ausführungsformen, bei welchen ein zweiter Träger vorgesehen ist (siehe oben bzw. weiter unten die Ausführungen zu Fig. 5), ist es gemäß einer weiteren Untervariante von Vorteil, wenn der zweite Träger eine Porengröße von 20 nm oder kleiner aufweist.

Da die Protein C-Adsorption mit ansteigender mittlerer Partikelgröße abnimmt, weist der erste Träger bei einer weiteren vorteilhaften Untervariante eine mittlere Partikelgröße von 100 bis 150 $\mu$m auf. Mehr bevorzugt weist der Träger eine mittlere Partikelgröße von 110 bis 130 $\mu$m, idealerweise eine mittlere Partikelgröße von ca. 120 $\mu$m, auf, da bei diesen Partikelgrößen einerseits toxische Substanzen wie Zytokine mit hoher Effizienz und andererseits wichtige Gerinnungsfaktoren wie Protein C kaum mehr adsorbiert werden, auch wenn die Körperflüssigkeit (Blutplasma) mit dem ersten Träger über längere Zeit in Kontakt gebracht wird.

Bei einer Variante der extrakorporalen Perfusionsvorrichtung mündet der Filtratkreislauf an einer Position stromab des Filters in den extrakorporalen Blutkreislauf. Bei dieser Variante ist der Filtratkreislauf offen und das fraktionierte Plasma wird nach Durchlaufen des Filtratkreislaufs stromab des Filters direkt dem extrakorporalen Blutkreislauf zugeführt (siehe schematische Darstellung dieser Variante in den Figuren 1, 3, 5 und 7). Das Grundprinzip dieser Variante kommt derzeit in der Immunadsorption zum Einsatz (beispielsweise Apheresevorrichtungen der Firma AsahiKasei, Japan). Das Abreicherungsmittel bzw. das Abgabemittel (erster bzw. zweiter Träger) ist bei dieser Variante vorzugsweise in einer im Filtratkreislauf angeordneten, vom Blutplasma durchfließbaren Einrichtung, die beispielsweise als Säule oder Kartusche ausgebildet sein kann, angeordnet.

Bei einer anderen Variante der extrakorporalen ist der Filtratkreislauf geschlossen und das fraktionierte Plasma gelangt über die Membran des Filters wieder in das im extrakorporalen Blutkreislauf strömende Blut (siehe schematische Darstellung dieser Variante in Fig. 2, 4 und 6). Das Grundprinzip der zweiten Variante ist durch das Blutreinigungssystem Prometheus® (Fresenius Medical Care GmbH, Deutschland) bekannt [Falkenhagen D, Strobl W, Vogt G, Schrefl A, Linsberger I, Gerner FJ, Schoenhofen M.: Fractionated plasma separation and adsorption system: a novel system for blood purification to remove albumin bound substances. Artif Organs. 1999 Jan;23(1):81-6]. ). Das Abreicherungsmittel bzw. das Abgabemittel (erster bzw. zweiter Träger) kann in einer im Filtratkreislauf angeordneten, vom Blutplasma durchfließbaren Einrichtung, die beispielsweise als Säule oder Kartusche ausgebildet sein kann, angeordnet sein.

Bei einer vorteilhaften Ausführungsform mündet der Filtratkreislauf in den Filter und bildet also einen im Filtratbereich geschlossenen Kreislauf, wobei der erste Träger die Form von Mikropartikeln hat und der Filtratkreislauf eine Suspension dieser Mikropartikel umfasst, wobei die Mikropartikel eine mittlere Partikelgröße von 20 $\mu$m oder kleiner, vorzugsweise eine mittlere Partikelgröße von 8 $\mu$m oder kleiner, idealerweise eine mittlere Partikelgröße von 5 $\mu$m oder kleiner, aufweisen. Diese Ausführungsform ist eine Weiterbildung der oben genannten Ausführungsformen, bei welchen nur der erste Träger (mit oder ohne eine adsorptive Beschichtung mit endotoxinbindendem Lipopeptid) im Filtratkreislauf angeordnet ist. Der mikropartikelförmige erste Träger (mit oder ohne Lipopeptid-Beschichtung) zirkuliert dabei als Suspension im Filtratkreislauf. Durch die sehr klein gewählten Partikelgrößen kann die Gefahr einer Lungenembolie umgangen werden, falls die Mikropartikel beispielsweise durch ein Filterleck in den extrakorporalen Blutkreislauf und weiter in den Körper des Patienten gelangen. Ein extrakorporaler Plasmakreislauf, in welchem eine Suspension von Mikropartikeln enthalten ist, stellt einen zentralen Bestandteil eines Microspheres-based Detoxification Systems (MDS) dar und wurde bereits in der EP 0776223 B und der US 5855782 beschrieben.

Die Erfindung kommt mit Vorteil zur Behandlung einer Infektion mit gram-negativen Bakterien, insbesondere zur Prophylaxe oder zur Behandlung einer systemischen inflammatorischen Reaktion (SIRS), einer Sepsis, einer schweren Sepsis oder eines septischen Schocks zur Anwendung. Repräsentative Beispiele für mittels der erfindungsgemäßen Perfusionsvorrichtung behandelbare Krankheitsbilder sind jene, die infolge einer Infektion mit gram-negativen Bakterien auftreten und in weiterer Folge in SIRS, einer Sepsis, einer schweren Sepsis mit Multiorganversagen oder einem septischen Schock münden können.

Als repräsentative Beispiele für gram-negative Bakterien sind *Escherichia spp, Haemophilus influenzae, Pseudomonas aeruginosa, Pasteurella, Enterobacter spp., Salmonella spp., Shigella spp.* zu nennen. Besonders vorteilhaft ist die Erfindung bei gram-negativen Bakterien, für die ein vermehrtes Auftreten multiresistenter Stämme beobachtet wurde, wobei hier *Pseudomonas aeruginosa* als besonders relevanter Vertreter hervorzuheben ist.

Wie oben bereits angeführt, kommt bzw. kann es beim Einsatz von Antibiotika bei einer Infektion mit gram-negativen Bakterien und durch die durch die Antibiotikagabe induzierte Zelllyse zu einer vermehrten Endotoxinausschüttung kommen. Eine vermehrte Endotoxinausschüttung wurde beispielsweise für Antibiotika beschrieben, die bevorzugt an das PBP-3 ("penicillin-binding protein-3") binden, z.B. die häufig eingesetzten Antibiotika der Gruppe der Cephalosporine wie Ceftazidimin. Die Erfindung kommt daher mit Vorteil als zusätzliche therapeutische oder prophylaktische Maßnahme im Rahmen einer konventionellen Behandlung von bakteriellen Infektionserkrankungen mittels Antibiotika zur Anwendung, um die durch Antibiotikagabe induzierte Endotoxinausschüttung und Induktion von Zytokinen abzufangen.

In einem weiteren Aspekt kommt die Erfindung mit Vorteil zur Prophylaxe oder zur Behandlung einer Entzündungsreaktion infolge eines akuten Leberversagens oder einer akuten Dekompensation bei chronischem Leberversagen, insbesondere einer systemischen inflammatorischen Reaktion (SIRS), einer Sepsis, einer schweren Sepsis mit Multiorganversagen oder eines septischen Schocks zur Anwendung. Beim Patienten mit intakter Leberfunktionen werden die aus dem Darm in die Blutbahn übertretenden Endotoxine vom Retikuloendothelialen System (RES) bzw. den Kupfferschen Sternzellen durch Endozytose eliminiert. Bei Patienten mit chronischem Leberversagen kann es zu einer akuten Dekompensation kommen. In diesem Fall können Endotoxine der normalen Darmflora die Darmbarriere überwinden und daher die Leber ungehindert passieren und zu einer systemischen inflammatorischen Reaktion (SIRS), einer Sepsis, einer schweren Sepsis mit Multiorganversagen oder einem septischen Schock führen.

Die Erfindung betrifft ferner ein Verfahren zur Prophylaxe oder zur Behandlung von Erkrankungen und Zuständen, die durch eine Endotoxinämie verursacht sind, mittels der erfindungsgemäßen extrakorporalen Perfusionsvorrichtung. Die oben angeführten Definitionen und Weiterbildungen sind auf das Verfahren gleichermaßen anzuwenden.

Vorzugsweise erfolgt vor Beginn der Behandlung des Patienten mittels der erfindungsgemäßen Perfusionsvorrichtung eine einmalige Bolusgabe, um rasch eine Lipopeptid-Serumkonzentration von vorzugsweise 0,01 µg/ml bis 0,8 µg/ml aufzubauen und einzustellen. Im Rahmen dieser Offenbarung wird unter dem Begriff "Bolus" somit eine einmalige parenterale Verabreichung des endotoxinbindenden Lipopeptids in Form einer Zubereitung vorzugsweise in Form einer Injektions- oder Infusionszubereitung verstanden. Die Bolusgabe kann mit Vorteil im Zusammenhang mit allen oben genannten Ausführungsformen der erfindungsgemäßen Vorrichtung zur Anwendung kommen. Beispiele für Injektionslösungen für die Bolusgabe sind unten in Beispiel 4 angeführt.

Die Erfindung wird im Folgenden anhand von nicht einschränkenden Beispielen und Zeichnungen näher erläutert. Die Zeichnungen zeigen in:

Fig. 1 eine schematische Darstellung einer Ausführungsform einer erfindungsgemäßen extrakorporalen Perfusionsvorrichtung mit offenem Filtratkreislauf, wobei das im Filtratkreislauf angeordnete Abreicherungsmittel auch gleichzeitig als Abgabemittel für Polymyxin B fungiert,

Fig. 2 eine schematische Darstellung einer weiteren Ausführungsform einer erfindungsgemäßen extrakorporalen Perfusionsvorrichtung mit geschlossenem Filtratkreislauf, wobei das im Filtratkreislauf angeordnete Abreicherungsmittel auch gleichzeitig als Abgabemittel für Polymyxin fungiert,

Fig. 3 eine schematische Darstellung einer weiteren Ausführungsform einer erfindungsgemäßen extrakorporalen Perfusionsvorrichtung mit offenem Filtratkreislauf und einer Dosiereinrichtung für Polymyxin,

Fig. 4 eine schematische Darstellung zwei weiterer Ausführungsformen einer erfindungsgemäßen extrakorporalen Perfusionsvorrichtung mit geschlossenem Filtratkreislauf und einer Dosiereinrichtung für Polymyxin,

Fig. 5 eine schematische Darstellung einer weiteren Ausführungsform einer erfindungsgemäßen extrakorporalen Perfusionsvorrichtung mit offenem Filtratkreislauf, wobei das Abgabemittel für Polymyxin stromab des Abreicherungsmittels im Filtratkreislauf angeordnet ist,

Fig. 6 eine schematische Darstellung einer weiteren Ausführungsform einer erfindungsgemäßen extrakorporalen Perfusionsvorrichtung mit geschlossenem Filtratkreislauf, wobei das Abgabemittel für Polymyxin als Mikropartikelsuspension im Filtratkreislauf vorliegt, und

Fig. 7 eine schematische Darstellung einer weiteren Ausführungsform einer erfindungsgemäßen extrakorporalen Perfusionsvorrichtung mit offenem Filtratkreislauf und einem stromab des Filters im extrakorporalen Blutkreislauf angeordneten Dialysator zur Abgabe von Polymyxin.

Die Fig. 1 zeigt eine schematische Darstellung einer extrakorporalen Perfusionsvorrichtung 100 (extrakorporale Blutreinigungsvorrichtung 100). Die Perfusionsvorrichtung 100 weist einen extrakorporalen Blutkreislauf 102 mit einem arteriellen Zulauf 102a (arterieller Schenkel) von einem Patienten 101 zu einem Filter 104 und einen venösen Ablauf 102b (venöser Schenkel) vom Filter 104 zum Patienten 101 auf. Das Patientenblut wird mittels einer Blutpumpe 103 (Pumprate $Q_{Blut}$ = 60 - 300 ml/min je nach Behandlungsverfahren) im Blutkreislauf 102 geführt. Der Filter 104 hat einen Siebkoeffizienten von 5 % für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von $\geq$ 0,6 und für Fibrinogen $\leq$ 0,1). Durch den Filter 104 wird ein Teil des Blutplasmas (= fraktioniertes Plasma) abfiltriert und einem Filtratkreislauf 105 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 105 ist als offener Kreislauf realisiert, der stromab des Filters 104 in den venösen Schenkel 102b mündet. Das fraktionierte Blutplasma wird mittels einer Filtratpumpe 106 (Pumprate $Q_{frakt.}$ Plasma = 15-20% des $Q_{Blut}$) durch den Filtratkreislauf 105 geführt. Der Perfusionsvorrichtung 100 ist zur automatisierten Steuerung der Vorrichtung 100 ferner eine Steuerung 110 zugeordnet, welche auch mit den Pumpen 103, 106 über Signalverbindungen in Verbindung steht. Die Steuerung 110 ist sinnvollerweise auch zur zentralen Datenerfassung und zur Datenausgabe eingerichtet.

Das durch den Filtratkreislauf 105 geführte fraktionierte Plasma wird durch eine im Filtratkreislauf 105 angeordnete Säule 107 geleitet. Die Säule 107 enthält ein Adsorberbett 107a aus einem Träger mit einer neutralen, hydrophoben Oberfläche, wobei die Trägeroberfläche eine adsorptive Beschichtung aus Lipopeptid-Molekülen, hier Polymyxin, aufweist. In der Fig. 1 ist der Träger ein Polystyrol-Divinylbenzol-Polymer mit einer mittleren Partikelgröße von 120 $\mu$m und einer mittleren Porengröße von 15 bis 20 nm, wobei die Oberfläche des Polymers eine adsorptive Beschichtung mit Polymyxin aufweist (Herstellung eines mit Polymyxin beschichteten Polymers siehe Beispiel 1). Das Adsorberbett 107a fungiert daher einerseits als Abreicherungsmittel für Zytokine wie TNF-$\alpha$, IL-6, IL-10, indem diese am Träger adsorbieren und aus dem Plasma entfernt werden. Andererseits fungiert das Adsorberbett 107a auch als Abgabemittel zur fortlaufenden Abgabe von Polymyxin in das Blutplasma, indem eine sehr geringe Menge an Polymyxin fortlaufend in das im Filtratkreislauf 105 geführte fraktionierte Blutplasma abgegeben wird (Desorption). Von dort gelangt das Polymyxin weiter in den venösen Ablauf 102b des extrakorporalen Blutkreislaufs 102, wo es mit den im Blut vorhandenen Endotoxinen einen Komplex bildet und diese unschädlich macht.

Die Fig. 2 zeigt eine schematische Darstellung einer extrakorporalen Perfusionsvorrichtung 200 (extrakorporale Blutreinigungsvorrichtung 200). Die Perfusionsvorrichtung 200 weist einen extrakorporalen Blutkreislauf 202 mit einem arteriellen Zulauf 202a (arterieller Schenkel) von einem Patienten 201 zu einem Filter 204 und einen venösen Ablauf 202b (venöser Schenkel) vom Filter 204 zum Patienten 201 auf. Das Patientenblut wird mittels einer Blutpumpe 203 (Pumprate $Q_{Blut}$ = 30 - 70 ml/min) im Blutkreislauf 202 geführt. Der Filter 204 hat einen Siebkoeffizienten von 5% für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von $\geq$ 0,6 und für Fibrinogen $\leq$ 0,1). Durch den Plasmafilter 204 wird ein Teil des Blutplasmas (= fraktioniertes Plasma) abfiltriert und einem Filtratkreislauf 205 zugeführt. Ein Filter mit einem Siebkoeffizienten von 5% für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 205 ist als ein im Filtratbereich geschlossener Kreislauf realisiert, wobei das fraktionierte Blutplasma mittels einer Filtratpumpe 206 (Pumprate $Q_{frakt.}$ Plasma = 15 - 25% des $Q_{Blut}$) durch den Filtratkreislauf 205 geführt wird. Der Perfusionsvorrichtung 200 ist zur automatisierten Steuerung der Vorrichtung 200 ferner eine Steuerung 210 zugeordnet, welche auch mit den Pumpen 203, 206 über Signalverbindungen in Verbindung steht. Die Steuerung 210 ist sinnvollerweise auch zur zentralen Datenerfassung und zur Datenausgabe eingerichtet.

Das durch den Filtratkreislauf 205 geführte fraktionierte Plasma wird durch eine im Filtratkreislauf 205 angeordnete Säule 207 geleitet. Die Säule 207 enthält ein Adsorberbett 207a aus einem Träger mit einer neutralen, hydrophoben Oberfläche, wobei die Trägeroberfläche eine adsorptive Beschichtung aus Lipopeptid-Molekülen, hier Polymyxin, aufweist. In der Fig. 2 ist der Träger ein Polystyrol-Divinylbenzol-Polymer mit einer mittleren Partikelgröße von 120 $\mu$m und einer mittleren Porengröße von 15 bis 20 nm, wobei die Oberfläche des Polymers eine adsorptive Beschichtung mit Polymyxin aufweist (Herstellung eines mit Polymyxin beschichteten Polymers siehe Beispiel 1). Das Adsorberbett 207a fungiert daher einerseits als Abreicherungsmittel für Zytokine wie TNF-$\alpha$, IL-6, IL-10, indem diese am Träger adsorbieren und aus dem Plasma entfernt werden. Andererseits fungiert das Adsorberbett 207a auch als Abgabemittel zur fortlaufenden Abgabe von Polymyxin in das Blutplasma, indem eine sehr geringe Menge an Polymyxin fortlaufend in das im Filtratkreislauf 205 geführte fraktionierte Blutplasma abgegeben wird (Desorption). Von dort gelangt das Polymyxin weiter in den extrakorporalen Blutkreislaufs 202. Die Polymyxin-Moleküle bilden anschließend mit den im Blut vorhandenen Endotoxinen einen Komplex.

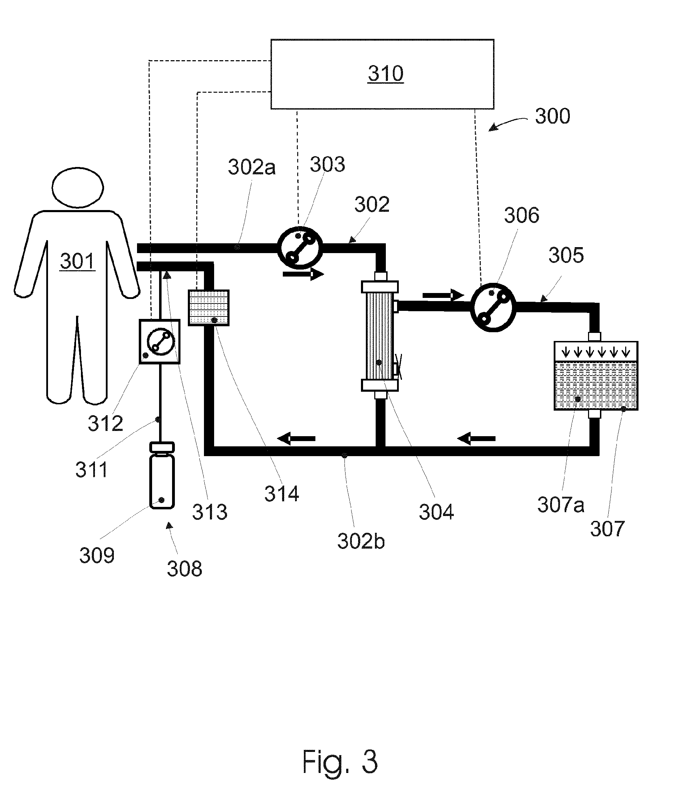

Die Fig. 3 zeigt eine schematische Darstellung einer extrakorporalen Perfusionsvorrichtung 300 (extrakorporale Blutreinigungsvorrichtung 300). Die Perfusionsvorrichtung 300 weist einen extrakorporalen Blutkreislauf 302 mit einem arteriellen Zulauf 302a (arterieller Schenkel) von einem Patienten 301 zu einem Filter 304 und einen venösen Ablauf 302b (venöser Schenkel) vom Filter 304 zum Patienten 301 auf. Das Patientenblut wird mittels einer Blutpumpe 303 (Pumprate $Q_{Blut}$ = 60-300 ml/min) im Blutkreislauf 302 geführt. Der Filter 304 hat einen Siebkoeffizienten von 5 % für Substanzen mit einer molaren Masse von 340 000 g/mol

(340 kDa), hier ein Filter des Typs Albuflow® (Hersteller: Fresenius Medical Care; Material: Polysulfon-Hohlfasern; Siebkoeffizient für Albumin von $\geq 0,6$ und für Fibrinogen $\leq 0,1$). Durch den Filter 304 wird ein Teil des Blutplasmas (= fraktioniertes Plasma) abfiltriert und einem Filtratkreislauf 305 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 305 ist als offener Kreislauf realisiert, der stromab des Filters 304 in den venösen Schenkel 302b mündet. Das fraktionierte Blutplasma wird mittels einer Filtratpumpe 306 (Pumprate $Q_{frakt.}$ Plasma = 15-25% des $Q_{Blut}$) durch den Filtratkreislauf 305 geführt.

Das durch den Filtratkreislauf 305 geführte fraktionierte Plasma wird durch eine im Filtratkreislauf 305 angeordnete Säule 307 geleitet. Die Säule 307 enthält ein Adsorberbett 307a aus einem Träger mit einer neutralen, hydrophoben Oberfläche. In der Fig. 3 ist der Träger ein Polystyrol-Divinylbenzol-Polymer mit einer mittleren Partikelgröße von 120 $\mu$m und einer mittleren Porengröße von 15 bis 20 nm. Das Adsorberbett 307a fungiert als Abreicherungsmittel für Zytokine wie TNF-$\alpha$, IL-6, IL-10, indem diese am Träger adsorbieren und aus dem Plasma entfernt werden.

Zur Abgabe eines endotoxinbindenden Lipopeptids ist der Perfusionsvorrichtung 300 eine an sich bekannte Infusionseinrichtung 308 umfassend einen Infusionsbehälter 309 (z.B. Infusionsflasche oder Infusionsbeutel) mit einer Lipopeptid-Infusionslösung, hier eine Polymyxin-Infusionslösung, einen Infusionsschlauch 311 und eine Infusionspumpe 312 zugeordnet. Geeignete Infusionslösungen sind weiter unten in Beispiel 5 beschrieben. Das Polymyxin wird an einer Lipopeptid-Zufuhrstelle 313 in den venösen Ablauf 302b des extrakorporalen Blutkreislaufs 302 infundiert. Die Polymyxin-Moleküle bilden anschließend mit den im Blut vorhandenen Endotoxinen einen Komplex.

In der Fig. 3 ist ferner eine vorteilhafte Weiterbildung dargestellt, bei welcher stromab des Filters 304 und stromauf der Lipopeptid-Zufuhrstelle 313 ein Polymyxin-Sensor 314 angeordnet ist. Hierfür kann beispielsweise ein Polymyxin-Sensor wie zuvor von Jiang *et al.* (Jiang et al. 2004. A synthetic peptide derived from bactericidal/permability-increasing protein neutralizes endotoxin in vitro and in vivo. International Immunopharmacology 4:527-537) beschrieben eingesetzt werden. Vorzugsweise wird für die Messung eine geringe Menge Blut aus dem extrakorporalen Blutkreislauf 302 über eine Abzweigleitung zum Sensor 314 geleitet und nach Konzentrationsbestimmung des Polymyxins verworfen. Der Perfusionsvorrichtung 300 ist zur automatisierten Steuerung der Vorrichtung 300 ferner eine Steuerung 310 zugeordnet, welche auch mit den Pumpen 303, 306, 311 und gegebenenfalls dem Polymyxin-Sensor 314 über Signalverbindungen in Verbindung steht. Die Steuerung 310 ist sinnvollerweise auch zur zentralen Datenerfassung und zur Datenausgabe eingerichtet. Der Perfusionsvorrichtung 300 kann ferner ein mittels der Steuerung 310 geregelter Regelkreis zugeordnet sein, wobei die infundierte Menge des Polymyxins in Abhängigkeit des durch den Sensor 314 gemessenen Polymyxin-Istwerts (Polymyxin-Serumkonzentration) durch Ansteuern der Infusionspumpe 312 in Bezug auf einen vorgebbaren Sollwert oder Sollwertbereich geregelt wird. Der Sollwert bzw. Sollwertbereich der Polymyxin-Serumkonzentration liegt typischerweise in einem Bereich von 0,01 - 0,8 $\mu$g/ml.

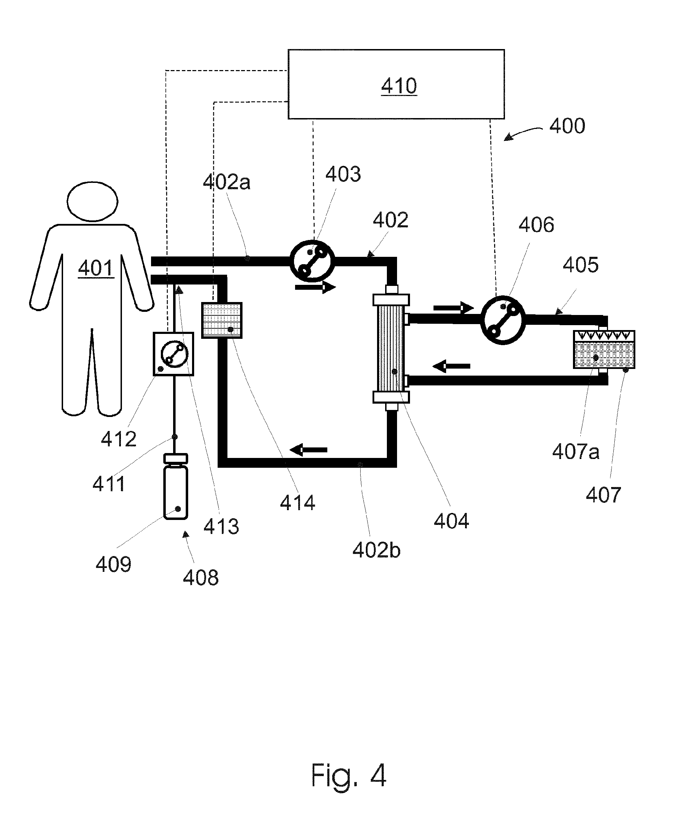

Die Fig. 4 zeigt eine schematische Darstellung einer extrakorporalen Perfusionsvorrichtung 400 (extrakorporale Blutreinigungsvorrichtung 400). Die Perfusionsvorrichtung 400 weist einen extrakorporalen Blutkreislauf 402 mit einem arteriellen Zulauf 402a (arterieller Schenkel) von einem Patienten 401 zu einem Filter 404 und einen venösen Ablauf 402b (venöser Schenkel) vom Filter 404 zum Patienten 401 auf. Das Patientenblut wird mittels einer Blutpumpe 403 (Pumprate $Q_{Blut}$ = 60-300 ml/min) im Blutkreislauf 402 geführt. Der Filter 404 hat einen Siebkoeffizienten von 5 % für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von $\geq 0,6$ und für Fibrinogen $\leq 0,1$). Durch den Filter 404 wird ein Teil des Blutplasmas (= fraktioniertes Plasma) abfiltriert und einem Filtratkreislauf 405 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 405 ist als ein im Filtratbereich geschlossener Kreislauf realisiert, wobei das fraktionierte Blutplasma mittels einer Filtratpumpe 406 (Pumprate $Q_{frakt.\ Plasma}$ = 15-25% des $Q_{Blut}$) durch den Filtratkreislauf 405 geführt wird.

Das durch den Filtratkreislauf 405 geführte fraktionierte Plasma wird durch eine im Filtratkreislauf 405 angeordnete Säule 407 geleitet. Die Säule 407 enthält ein Adsorberbett 407a aus einem Träger mit einer neutralen, hydrophoben Oberfläche. In der Fig. 4 ist der Träger ein Polystyrol-Divinylbenzol-Polymer mit einer mittleren Partikelgröße

von 120 μm und einer mittleren Porengröße von 15 bis 20 nm. Das Adsorberbett 407a fungiert als Abreicherungsmittel für Zytokine wie TNF-α, IL-6, IL-10, indem diese am Träger adsorbieren und aus dem Plasma entfernt werden.

Zur Abgabe eines endotoxinbindenden Lipopeptids ist der Perfusionsvorrichtung 400 eine an sich bekannte Infusionseinrichtung 408 umfassend einen Infusionsbehälter 409 (z.B. Infusionsflasche oder Infusionsbeutel) mit einer Lipopeptid-Infusionslösung, hier eine Polymyxin-Infusionslösung, einen Infusionsschlauch 411 und eine Infusionspumpe 412 zugeordnet. Geeignete Infusionslösungen sind weiter unten in Beispiel 5 beschrieben. Das Polymyxin wird an einer Lipopeptid-Zufuhrstelle 413 in den venösen Ablauf 402b des extrakorporalen Blutkreislaufs 402 infundiert. Die Polymyxin-Moleküle bilden anschließend mit den im Blut vorhandenen Endotoxinen einen Komplex.

In der Fig. 4 ist in Analogie zur Fig. 3 ferner eine vorteilhafte Weiterbildung dargestellt, bei welcher stromab des Filters 404 und stromauf der Lipopeptid-Zufuhrstelle 413 ein Polymyxin-Sensor 414 angeordnet ist. Hierfür kann beispielsweise ein Polymyxin-Sensor wie zuvor von Jiang *et al.* (Jiang et al. 2004. A synthetic peptide derived from bactericidal/permabilityincreasing protein neutralizes endotoxin in vitro and in vivo. International Immunopharmacology 4:527-537) beschrieben eingesetzt werden. Vorzugsweise wird für die Messung eine geringe Menge Blut aus dem extrakorporalen Blutkreislauf 402 über eine Abzweigleitung zum Sensor 414 geleitet und nach Konzentrationsbestimmung des Polymyxins verworfen. Der Perfusionsvorrichtung 400 ist zur automatisierten Steuerung der Vorrichtung 400 ferner eine Steuerung 410 zugeordnet, welche auch mit den Pumpen 403, 406, 411 und gegebenenfalls dem Polymyxin-Sensor 414 über Signalverbindungen in Verbindung steht. Die Steuerung 410 ist sinnvollerweise auch zur zentralen Datenerfassung und zur Datenausgabe eingerichtet. Der Perfusionsvorrichtung 400 kann ferner ein mittels der Steuerung 410 geregelter Regelkreis zugeordnet sein, wobei die infundierte Menge des Polymyxins in Abhängigkeit des durch den Sensor 414 gemessenen Polymyxin-Istwerts (Polymyxin-Serumkonzentration) durch Ansteuern der Infusionspumpe 412 in Bezug auf einen vorgebbaren Sollwert oder Sollwertbereich geregelt wird. Der Sollwert bzw. Sollwertbereich der Polymyxin-Serumkonzentration liegt typischerweise in einem Bereich von 0,01 - 0,8 μg/ml. Die Fig. 5 zeigt eine schematische Darstellung einer extrakorporalen Perfusionsvorrichtung 500 (extrakorporale Blutreinigungsvorrichtung 500). Die Perfusionsvorrichtung 500 weist einen extrakorporalen Blutkreislauf 502 mit einem arteriellen Zulauf 502a (arterieller Schenkel) von einem Patienten 501 zu einem Filter 504 und einen venösen Ablauf 502b (venöser Schenkel) vom Filter 504 zum Patienten 501 auf. Das Patientenblut wird mittels einer Blutpumpe 503 (Pumprate $Q_{Blut}$ = 60-300 ml/min) im Blutkreislauf 502 geführt. Der Filter 504 hat einen Siebkoeffizienten von 5 % für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von ≥ 0,6 und für Fibrinogen ≤ 0,1). Durch den Filter 504 wird ein Teil des Blutplasmas (= fraktioniertes Plasma) abfiltriert und einem Filtratkreislauf 505 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 505 ist als offener Kreislauf realisiert, der stromab des Filters 504 in den venösen Schenkel 502b mündet. Das fraktionierte Blutplasma wird mittels einer Filtratpumpe 506 (Pumprate $Q_{frakt. Plasma}$ = 15-25% des $Q_{Blut}$) durch den Filtratkreislauf 505 geführt. Der Perfusionsvorrichtung 500 ist zur automatisierten Steuerung der Vorrichtung 500 ferner eine Steuerung 510 zugeordnet, welche auch mit den Pumpen 503, 506 über Signalverbindungen in Verbindung steht. Die Steuerung 510 ist sinnvollerweise auch zur zentralen Datenerfassung und zur Datenausgabe eingerichtet. In der Fig. 5 ist der Filtratkreislauf 505 als offener Kreislauf realisiert. Der Filtratkreislauf 505 kann aber auch als ein in sich geschlossener Kreislauf realisiert sein.

Das durch den Filtratkreislauf 505 geführte fraktionierte Plasma wird durch eine im Filtratkreislauf 505 angeordnete Säule 507 geleitet. Die Säule 507 enthält ein Adsorberbett 507a aus einem Träger mit einer neutralen, hydrophoben Oberfläche. In der Fig. 5 ist der Träger ein Polystyrol-Divinylbenzol-Polymer mit einer mittleren Partikelgröße von 120 μm und einer mittleren Porengröße von 15 bis 20 nm. Das Adsorberbett 507a fungiert als Abreicherungsmittel für Zytokine wie TNF-α, IL-6, IL-10, indem diese am Träger adsorbieren und aus dem Plasma entfernt werden.

Zur Abgabe eines Lipopeptids ist im Filtratkreislauf 505 stromab der Säule 507 eine weitere Säule 508 angeordnet. Die Säule 508 enthält ein Trägerbett 508a aus einem Träger mit einer neutralen hydrophoben Oberfläche, wobei die Trägeroberfläche eine adsorptive Beschichtung aus Lipopeptid-Molekülen, hier Polymyxin, aufweist. In der Fig. 5 ist der Träger ein Polystyrol-Divinylbenzol-Polymer mit einer mittleren Partikelgröße von 120 μm und einer mittleren Porengröße von 15 bis 20 nm, wobei die Oberfläche des Polymers eine adsorptive Beschichtung mit Polymyxin aufweist (Herstellung eines mit Polymyxin beschichteten Polymers siehe Beispiel 1). Das Trägerbett 508a fungiert als Abgabemittel zur fortlaufenden Abgabe von Polymyxin in das Blutplasma, indem eine sehr geringe Menge an Polymyxin fortlaufend in das im Filtratkreislauf 505 geführte fraktionierte Blutplasma abgegeben wird (Desorption). Von dort gelangt das Polymyxin weiter in den extrakorporalen Blutkreislaufs 502. Die Polymyxin-Moleküle bilden anschließend mit den im Blut vorhandenen Endotoxinen einen Komplex.

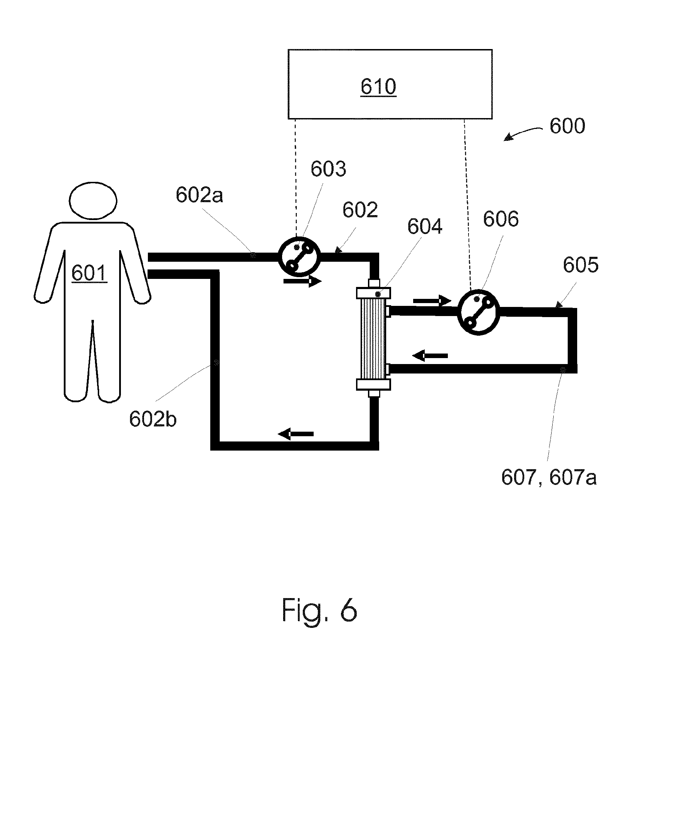

Die Fig. 6 zeigt eine schematische Darstellung einer extrakorporalen Perfusionsvorrichtung 600 (extrakorporale Blutreinigungsvorrichtung 600). Die Perfusionsvorrichtung 600 weist einen extrakorporalen Blutkreislauf 602 mit einem arteriellen Zulauf 602a (arterieller Schenkel) von einem Patienten 601 zu einem Filter 604 und einen venösen Ablauf 602b (venöser Schenkel) vom Filter 604 zum Patienten 601 auf. Das Patientenblut wird mittels einer Blutpumpe 603 (Pumprate $Q_{Blut}$ = 60-300 ml/min) im Blutkreislauf 602 geführt. Der Filter 604 hat einen Siebkoeffizienten von 5 % für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von $\geq$ 0,6 und für Fibrinogen $\leq$ 0,1). Durch den Filter 604 wird ein Teil des Blutplasmas (= fraktioniertes Plasma) abfiltriert und einem Filtratkreislauf 605 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 605 ist als ein im Filtratbereich geschlossener Kreislauf realisiert, wobei das fraktionierte Blutplasma mittels einer Filtratpumpe 606 (Pumprate $Q_{frakt.\ Plasma}$ = 15-25 % des $Q_{Blut}$) durch den Filtratkreislauf 605 geführt wird.

Der Perfusionsvorrichtung 600 ist zur automatisierten Steuerung der Vorrichtung 600 ferner eine Steuerung 610 zugeordnet, welche auch mit den Pumpen 603, 606 über Signalverbindungen in Verbindung steht. Die Steuerung 610 ist sinnvollerweise auch zur zentralen Datenerfassung und zur Datenausgabe eingerichtet.

Der Filtratkreislauf 605 umfasst dabei als Abreicherungs/Abgabemittel 607 eine (nicht im Detail dargestellte) Suspension des Trägers 607a, d.h. das Abreicherungs/Abgabemittel 607 bzw. der Träger 607a ist mikropartikelförmig und liegt als Suspension im fraktionierten Plasma verteilt vor und zirkuliert als Suspension im Filtratkreislauf 605. Der mikropartikelförmige Träger 607a besitzt eine neutrale, hydrophobe Oberfläche, wobei die Trägeroberfläche eine adsorptive Beschichtung aus Lipopeptid-Molekülen, hier Polymyxin, aufweist. In der Fig. 6 ist der Träger 607a ein Polystyrol-Divinylbenzol-Polymer mit einer mittleren Partikelgröße von 5 $\mu$m +/-3-4 $\mu$m und einer mittleren Porengröße von 15 bis 20 nm (Bezugsquelle Polymer: Rohm&Haas), wobei die Oberfläche des Polymers eine adsorptive Beschichtung mit Polymyxin aufweist (Herstellung eines mit Polymyxin beschichteten Polymers siehe Beispiel 1). Der mikropartikelförmige Träger 607a fungiert daher einerseits als Abreicherungsmittel für Zytokine wie TNF-$\alpha$, IL-6, IL-10, indem diese am Träger adsorbieren und aus dem Plasma entfernt werden. Andererseits fungiert der mikropartikelförmige Träger 607a auch als Abgabemittel zur fortlaufenden Abgabe von Polymyxin in das Blutplasma, indem eine sehr geringe Menge an Polymyxin fortlaufend in das im Filtratkreislauf 605 geführte fraktionierte Blutplasma abgegeben wird (Desorption). Von dort gelangt das Polymyxin weiter in den extrakorporalen Blutkreislaufs 602. Die Polymyxin-Moleküle bilden anschließend mit den im Blut vorhandenen Endotoxinen einen Komplex.

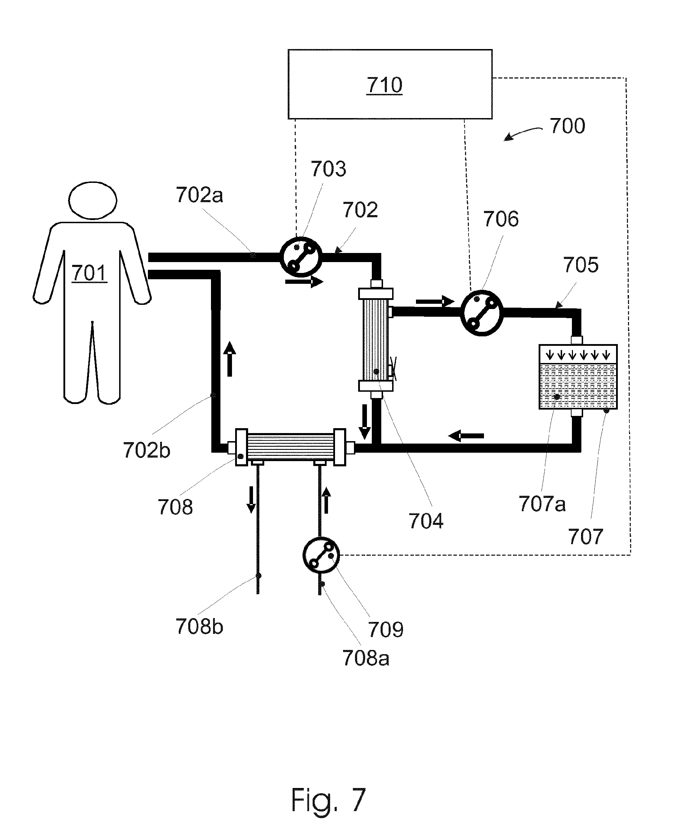

Die Fig. 7 zeigt eine schematische Darstellung einer extrakorporalen Perfusionsvorrichtung 700 (extrakorporale Blutreinigungsvorrichtung 700). Die Perfusionsvorrichtung 700 weist einen extrakorporalen Blutkreislauf 702 mit einem arteriellen Zulauf 702a (arterieller Schenkel) von einem Patienten 701 zu einem Filter 704 und einen venösen Ablauf 702b (venöser Schenkel) vom Filter 704 zum Patienten 701 auf. Das Patientenblut wird mittels einer Blutpumpe 703 (Pumprate $Q_{Blut}$ = 60-300 ml/min, je nach Dialyseverfahren) im Blutkreislauf 702 geführt. Der Filter 704 hat einen Siebkoeffizienten von 5 % für Substanzen mit einer molaren Masse von 340 000 g/mol (340 kDa), hier ein Filter des Typs Albuflow® (Hersteller: Fresenius Medical Care; Material: Polysulfon-Hohlfasern; Siebkoeffizient für Albumin von $\geq$ 0,6 und für Fibrinogen $\leq$ 0,1). Durch den Filter 704 wird ein Teil des Blutplasmas (= fraktioniertes Plasma) abfiltriert und einem Filtratkreislauf 705 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 705 ist als offener Kreislauf realisiert, der stromab des Filters 704 in den venösen Schenkel 702b mündet. Das fraktionierte Blutplasma wird mittels einer Filtratpumpe 706 (Pumprate $Q_{frakt.\ Plasma}$ = 15-25% des $Q_{Blut}$) durch den Filtratkreislauf 705 geführt. In der Fig. 7 ist der Filtratkreislauf 705 als offener Kreislauf realisiert. Der Filtratkreislauf 705 kann aber auch als ein in sich geschlossener Kreislauf realisiert sein.

Das durch den Filtratkreislauf 705 geführte fraktionierte Plasma wird durch eine im Filtratkreislauf 705 angeordnete Säule 707 geleitet. Die Säule 707 enthält ein Adsorberbett 707a aus einem Träger mit einer neutralen, hydrophoben Oberfläche. In der Fig. 7 ist der Träger ein Polystyrol-Divinylbenzol-Polymer mit einer mittleren Partikelgröße von 120 $\mu$m und einer mittleren Porengröße von 15 bis 20 nm. Das Adsorberbett 707a fungiert als Abreicherungsmittel für Zytokine wie TNF-$\alpha$, IL-6, IL-10, indem diese am Träger adsorbieren und aus dem Plasma entfernt werden.

Zur Abgabe eines Lipopeptids ist im venösen Ablauf 702b des extrakorporalen Blutkreislaufs 702 ein Dialysator 708 (Dialysefilter 708) angeordnet. Im Dialysator 708 wird das Blut über eine semipermeable Membran mit der Dialysierlösung in Verbindung gebracht. Die Dialysierlösung wird mittels einer Dialysierlösungspumpe 709 über einen Dialysierlösungszulauf 708a in den Dialysator 708 gepumpt. Nach Durchlauf des Dialysators 708 wird das Dialysat über einen Dialysatablauf 708b abgeleitet und entsorgt. Das Lipopeptid, hier Polymyxin, wird mittels der Dialysierlösung dem Blut zugeführt. Bei der in Fig. 7 dargestellten Ausführungsform fungiert somit der Dialysator 708 als Abgabemittel für das Lipopeptid (Polymyxin) in den extrakorporalen Blutkreislauf 702. Die Polymyxin-Moleküle bilden anschließend mit den im Blut vorhandenen Endotoxinen einen Komplex.

Der Dialysator 708 umfasst vorzugsweise eine hydrophile Polysulfon-Membran mit einer Oberfläche von 1,4 - 2,0 m$^2$, die durch Blending mit PVP (Polyvinyl-Pyrrolidon) hergestellt wurde. Beispielsweise werden diese Membranen in Filtern der Firma Fresenius Medical Care verwendet u.a. in den Typen AF 1000 bzw. FX60. Diese Dialysefilter haben einen Siebkoeffizienten für Albumin unter 0,1 %. Denkbar ist auch die Verwendung eines sogenannten High Cutoff Filters, der ebenfalls auf der Nutzung von hydrophilen Polysulfon-Membranen mit einem Siebkoeffizienten von etwa 4% für Albumin beruht. Unter Dialyse-Bedingungen, d.h. es kommt vorrangig eine diffusionsgesteuerte Elimination der zur Entfernung gedachten Substanzen zum Einsatz, beträgt der Albuminverlust unter 5-10 g pro Behandlung. Als Beispiel für ein derartiges Dialysefilter kann der von der Firma Fresenius Medical Care hergestellte EMiC$^2$ - Filter genannt werden Die Flussbedingungen, mit denen derartige Filter im klinischen Einsatz betrieben werden, sind für einen Blutfluss von 60 - 300 ml/min je nach Einsatzbedingungen entsprechend gewählt: unter den Bedingungen einer so genannten kontinuierlichen veno-venösen Hämodialyse werden Blutflüsse von 60-80 ml/min genutzt, wohingegen bei der Dialysegeräte-gestützten intermittierenden Hämodialyse Blutflüsse von 150 - 300 ml/min in akuten Fällen, also bei Patienten mit einem akuten Nierenversagen, das auch sehr häufig bei der Sepsis auftritt, verwendet werden. Der Dialysatfluss ist bei der intermittierenden Hämodialyse vorzugsweise auf 500 ml/min eingestellt, wohingegen bei der kontinuierlichen veno-venösen Hämodialyse Dialysatflüsse im Verhältnis von 1 : 1 zum Blutfluss die Regel sind. Die Konzentration des Lipopeptids/Polymyxin in der Dialyseflüssigkeit sollte in dem Bereich von 0,2 - 1,0 $\mu$g/l liegen, also leicht höher als der angesteuerte Serumkonzentrationswert des zu behandelnden Patienten liegen, da der Siebkoeffizient der genannten Dialysefilter zwischen 0.8 (AF 1000) und 0.9 (EMiC$^2$) liegt, also zwischen 80 und 90%.

Der Perfusionsvorrichtung 700 ist zur automatisierten Steuerung der Vorrichtung 700 ferner eine Steuerung 710 zugeordnet, welche auch mit den Pumpen 703, 706, 709 über Signalverbindungen in Verbindung steht. Die Steuerung 710 ist sinnvollerweise auch zur zentralen Datenerfassung und zur Datenausgabe eingerichtet.

**1. Beispiel 1: Polymyxin B (PMB)-Desorption in Plasma und fraktioniertem Plasma (Verwendung eines Albuflow-Filters) mit unterschiedlich PMB-beschichtetem Träger (mittlere Partikelgröße: 120 $\mu$m, mittlere Porengröße: 15-20 nm)**

1.1 PMB-Beschichtung

*Träger:* Amberchrom CG161c (Polystyrol-Divinylbenzol-Copolymer, Dow Chemical Company), mittlere Partikelgröße 120 $\mu$m, mittlere Porengröße 15 nm; zugängliche Oberfläche 900 m$^2$/g Polymer (trocken). Das Trockengewicht pro ml feuchten Träger beträgt 18% (w/v).

*Polymyxin B (PMB):* Polymyxin B Sulfat (Sigma Aldrich)

Die PMB-Lösung (10 mg/ml in Aqua Dest) wird bei bei 121 °C, 30 min lang autoklaviert und anschließend wird der Träger wird in 15 ml Greinerröhrchen wie folgt (Tabelle 1.1) mit PMB beschichtet: 3 ml Träger mit 7,5 ml PMB-Lösung

*Tabelle 1.1*

| Beschichtung PMB in mg pro ml Träger | Träger [ml] | PMB-Lösung [ml] | NaCl [ml] |
|---|---|---|---|
| 0 | 3 | 0 | 7,5 |
| 1 | 3 | 0,3 | 7,2 |
| 2,5 | 3 | 0,75 | 6,75 |
| 5 | 3 | 1,5 | 6 |
| 7,5 | 3 | 2,25 | 5,25 |
| 10 | 3 | 3 | 4,5 |

Die Beschichtung wird über Nacht am Rollenmischer bei Raumtemperatur durchgeführt. Danach wird der Träger 2 mal mit 10 ml NaCl-Lösung (steril) gewaschen und eine 50 %ige Suspension hergestellt.

1.2 Batchtest

Freshfrozen Plasma (Citratplasma) wurde mit Hilfe des Albuflow®-Filters (Fresenius Medical Care, Deutschland) fraktioniert und bei -20°C gemeinsam mit dem Vollplasma eingefroren.

A: Vollplasma
B: fraktioniertes Plasma

*Tabelle 1.2: Batchansätze*

|  | 0 mg/ml | 1 mg/ml | 2,5 mg/ml | 5 mg/ml | 7,5 mg/ml | 10 mg/ml |
|---|---|---|---|---|---|---|
| **A** Vollplasma | 0A 1+2 | 1A 1+2 | 2,5A 1+2 | 5A 1+2 | 7,5A 1+2 | 10A 1+2 |
| **B** fraktioniertes Plasma | 0B 1+2 | 1B 1+2 | 2,5B 1+2 | 5B 1+2 | 7,5B 1+2 | 10B 1+2 |

Im Doppelansatz (siehe Tabelle 1.2) werden je 0,5 ml Träger mit je 4,5 ml Plasma = 10 % (v/v) Ansatz bei 37 °C 60 min lang am Envirogenie inkubiert. Danach wird der Träger abzentrifugiert und der Überstand für die PMB-Quantifizierung mittes ELISA (Polymyxin-ELISA von Beijing Kwinbon Biotechnology Co., Ltd., China) verwendet.

### 1.3 Ergebnisse

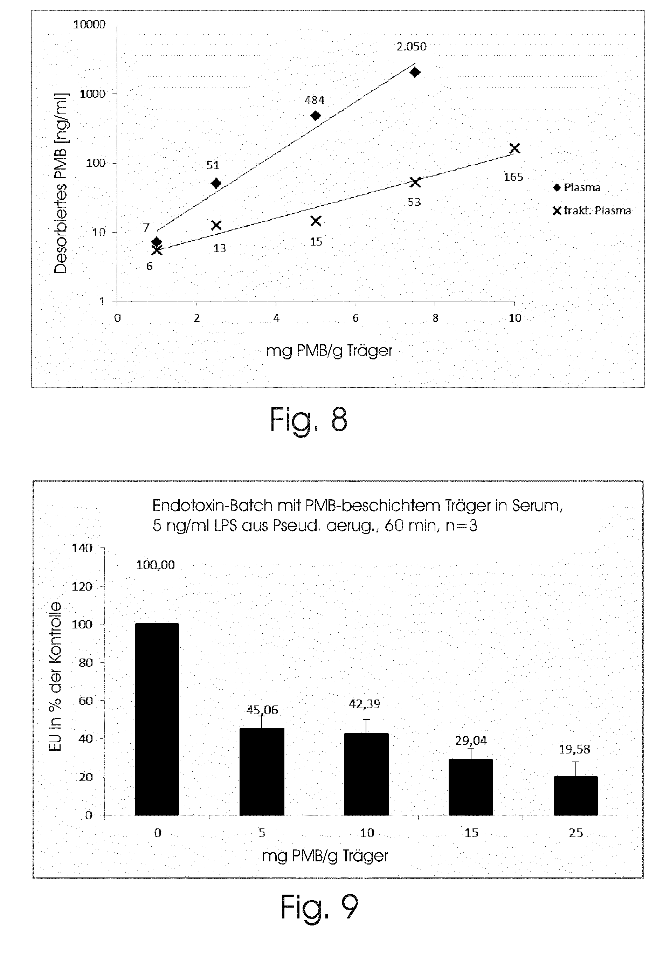

Mit steigender PMB-Konzentration der zur Beschichtung des Trägers eingesetzten PMB-Lösung desorbiert eine höhere Menge an PMB ins Plasma. Das Ergebnis ist in der Fig. 8 (Desorption von PMB in Abhängigkeit der PMB-Beschichtungskonzentration).

Um im fraktioniertem Plasma, durch die Desorption, einen PMB-Plasmaspiegel von ca. 150 ng/ml zu erreichen, muss der in diesem Versuch verwendete Träger mit 10 mg PMB pro ml Adsorber beschichtet werden.

## 2. Beispiel 2: Endotoxin-Batch mit unterschiedlich PMB-beschichtetem Träger in Serum

### 2.1 Versuchsaufbau

Konditionierter Träger (Amberchrom CG161c: Ethylvinylbenzol-Divinylbenzol-Copolymer (Dow Chemical Company), mittlere Partikelgröße 120 $\mu$m, mittlere Porengröße 15 nm) wird mit unterschiedlicher Menge an Polymyxin B (PMB) beschichtet: 0, 5, 10, 15 und 25 mg/ g feuchten Träger. Diese werden im Dreifachansatz in einem Endotoxin-Batchtest auf ihre LPS-Inaktivierung in Serum getestet.

### 2.2 Versuchsdurchführung

*PMB-Beschichtung:*

Es werden Trägerproben mit unterschiedlichen PMB-Konzentrationen (5 mg, 10 mg, 15 mg und 25 mg pro g feuchten Träger) hergestellt (siehe Protokoll oben in Beispiel 1). Die PMB-Lösung (10 mg/ml in Aqua Dest) und der Träger in 50 %iger Suspension werden bei 121 °C, 30 min lang autoklaviert und der Träger wird in 15 ml Greinerröhrchen wie folgt mit PMB beschichtet (Tabelle 2.2):

*Tabelle 2.2:*

| mg PMB/g Adsorber | 50 %igeAdsorbersuspension [ml] | PMB-Lösung [ml] | NaCl [ml] |
|---|---|---|---|
| 0 | 2 | 0 | 3 |
| 5 | 2 | 0,5 | 2,5 |
| 10 | 2 | 1 | 2 |
| 15 | 2 | 1,5 | 1,5 |
| 25 | 2 | 2,5 | 0,5 |

Die Beschichtung wird 4 Stunden lang am Überkopfschüttler (Enviro-Genie, Frequenz: 25:50) bei Raumtemperatur durchgeführt. Danach wird der Träger 2 mal mit 10 ml NaCl-Lösung (steril) gewaschen und eine 50 %ige Suspension hergestellt.

*Herstellung von Serum:*

Den Spender werden 7 Blutröhrchen (Vacuette mit Serum Beads für Gerinnungsaktivierung) ä 8 ml abgenommen. Die mit Blut gefüllten Röhrchen werden 30 min stehengelassen. Das geronnene Blut wird danach zentrifugiert und das Serum gewonnen (in einem sterilen Erlenmeyerkolben gepoolt).

*Endotoxin(LPS)-Lösung:*

LPS: *Pseudomonas aeruginosa,* L-7018 Fa. Sigma Lot: 128K4115, Lagerung -70°C, a 100 μl 10$^{-3}$ g/ml (1mg/ml)

Aus dieser LPS-Stocklösung wird mit steriler NaCl-Lösung eine LPS-Lösung mit einer Konzentration von 10 μg/ml hergestellt. Das LPS wird im Batch mit einer Endkonzentrationen von 5 ng/ml Ansatz eingesetzt. 10 μl LPS-Lösung mit einer Konzentration von 10 μg/ml werden in 20 ml Serum pipettiert. Der Batchansatz wird in 2 ml Blutabnahmeröhrchen im Dreieransatz durchgeführt.

2.3 Ergebnisse:

Die LPS-Inaktivierung von mehr als 50 % konnte schon mit der niedrigsten beschichteten PMB-Konzentration erreicht werden. Das Ergebnis ist in der Fig. 9 dargestellt (EU = Endotoxin Units).

**3. Beispiel 3:** Endotoxin(LPS)-Inaktivierung in Abhängigkeit der Polymyxin-Konzentration anhand von Endotoxinen aus *E. coli* und *Pseudomonas aeruginosa.*

3.1. Ziel

Ziel dieses Versuches ist es, die Polymyxin B (PMB)-konzentrationsabhängige Endotoxininaktivierung im Plasma zu ermitteln (Batchtest I). Weiteres soll untersucht werden inwiefern diese Endotoxininaktivierung eine Hemmung der Cytokinausschüttung zu Folge hat (Batchtest II).

3.2. Blutspende

Einem Spender werden 9 Blutabnahmeröhrchen (zu je 9 ml) mit 5 IU Heparin gespiket abgenommen. Das Plasma wird abzentrifugiert und das Zellpellet am Rollenmischer inkubiert. Das Plasma wird mit Endotoxin (LPS) gespiket und für den Batchtest I verwendet:

3.3. LPS-spike, Polymyxin B-Lösungen und Batchtest I

LPS: *Pseudomonas aeruginosa* (L-7018 Fa. Sigma Lot: 128K4115, -70°C, a 100 μl 10-3 g/ml (1mg/ml))

LPS: *E. coli* (L-4130 Fa. Sigma Lot: 110M4086M, -70 °C, a 100 μl 10-3 g/ml (1mg/ml))

Das LPS wird im Batch mit einer Endkonzentrationen von 0,5 ng/ml eingesetzt. Die Ansätze werden in 3 ml pyrogenfreie Glasvials durchgeführt. Im Batchtest I werden im Zweifach-Ansatz unterschiedliche PMB-Konzentrationen (Fa. Sigma, P-1004) zugesetzt und für 60 min am Überkopf-Schüttler bei 37 °C inkubiert (siehe Tabelle 3.5).

Im Batchtest I werden PMB-Konzentrationen mit 0 (ohne PMB), 10, 100, 250, 500 und 1000 ng/ml eingesetzt. Dafür werden sterile PMB-Lösungen (pyrogenfrei autoklaviert bei 121 °C, 90 min) mit folgenden Konzentrationen hergestellt (Tabelle 3.3):

*Tabelle 3.3:*

|  | PMB [ng/ml] | PMB [ng/ml] im Batch (1:15) |
|---|---|---|
| PMB-Lösung A | 150 | 10 |
| PMB-Lösung B | 1500 | 100 |
| PMB-Lösung C | 3750 | 250 |
| PMB-Lösung D | 7500 | 500 |
| PMB-Lösung E | 15000 | 1000 |
| NaCl-Lösung | 0 | 0 |

3.4. Endotoxin-Analyse

Mit Hilfe eines Limulus Amebocyte Lysate test (LAL) von Charles River werden die Endotoxine in Form von EU/ml gemessen.

3.5. Cytokinbatch (Batchtest II)

Das mit LPS und PMB gespikte Plasma wird nach dem Batchtest I auf das vom Blutspender gewonnene Zellkonzentrat im Verhältnis 1:1 zurückgeführt (siehe Tabelle 3.5). Für den Cytokinbatch wurden die Proben aus Batchtest I mit einer PMB-Konzentration von 0 (ohne PMB), 250, 500 und 1000 ng/ml herangezogen. Als Kontrolle wurde eine Probe ohne LPS und mit 1000 ng/ml PMB mitgeführt. Nach den Inkubationszeiten von 4 h und 12 h bei 37 °C am Rollenmixer (5 Umdrehungen/min) werden Proben gezogen, abzentrifugiert und 50 µl Plasma bei -80 °C für die spätere Cytokin-Quantifizierung eingefroren. Die Versuchsdaten zum Cytokinbatch sind in der Tabelle 3.5 aufgelistet.

*Tabelle 3.5:*

| LPS *Pseudomonas aeruginosa* | | | | | | | | |
|---|---|---|---|---|---|---|---|---|
| PMB [ng/ml] | PMB-Lsg | Plasma +0,5ng/ml LPS | Inkubation | LAL | EU/ml | Cytokin-Batch | Probe 4h | Probe 12 h |
| 0 | 100 µl NaCl | 1400 µl | 60min | #1 | 0,333 | 1500 µl Zellenkonzentrat + 1500 µl LPS-PMB Plasma | 250 µl → 50 µl Plasma -80°C | 250 µl→ 50 µl Plasma -80°C |
| 0 | 100 µl NaC | 1400 µl | 60min | #2 | 0,229 | | | |
| 10 | 100 µl Lsg A | 1400 µl | 60min | #3 | 0,178 | | | |
| 10 | 100 µl Lsg A | 1400 µl | 60min | #4 | 0,167 | | | |
| 100 | 100 µl Lsg B | 1400 µl | 60min | #5 | 0,112 | | | |
| 100 | 100 µl Lsg B | 1400 µl | 60min | #6 | 0,137 | | | |
| 250 | 100 µl Lsg C | 1400 µl | 60min | #7 | 0,108 | 1500 µl Zellenkonzentrat + 1500 ul LPS-PMB Plasma | 250 µl→ 50 µl Plasma -80 °C | 250 µl→ 50 µl Plasma -80°C |
| 250 | 100 µl Lsg C | 1400 µl | 60min | #8 | 0,123 | | | |
| 500 | 100 µl Lsg D | 1400 µl | 60min | #9 | 0,091 | 1500µl Zellenkonzentrat + 1500 µl LPS-PMB Plasma | 250 µl→ 50 µl Plasma -80 °C | 250 µl→ 50 µl Plasma -80°C |
| 500 | 100 µl Lsg D | 1400 µl | 60min | #10 | 0,081 | | | |
| 1000 | 100 µl Lsg E | 1400 µl | 60min | #11 | 0,062 | 1500 µl Zellenkonzentrat + 1500 µl LPS-PMB Plasma | 250 µl→ 50µl Plasma-80°C | 250 µl→ 50 µl Plasma -80°C |
| 1000 | 100 µl Lsg E | 1400 µl | 60min | #12 | 0,061 | | | |
| | | | | | | | | |
| LPS *E. coli* | | | | | | | | |
| PMB [ng/ml] | PMB-Lsg | Plasma+0,5ng/ml LPS | Inkubation | LAL | EU/ml | Cytokin-Batch | Probe 4 h | Probe 12 h |
| 0 | 100µl NaCl | 1400µl | 60min | #13 | 1,8 | 1500 µl Zellenkonzentrat + 1500 µl LPS-PMB Plasma | 250 µl→ 50 µl Plasma -80°C | 250 µl→ 50 µl Plasma -80°C |
| 0 | 100µl NaCl | 1400µl | 60min | #14 | 1,841 | | | |
| 10 | 100 µl Lsg A | 1400 µl | 60min | #15 | 0,77 | | | |

(fortgesetzt)

| LPS *E. coli* | | | | | | | | |
|---|---|---|---|---|---|---|---|---|
| PMB [ng/ml] | PMB-Lsg | Plasma+0,5ng/ml LPS | Inkubation | LAL | EU/ml | Cytokin-Batch | Probe 4 h | Probe 12 h |
| 10 | 100 μl Lsg A | 1400 μl | 60min | #16 | 0,871 | | | |
| 100 | 100 μl Lsg B | 1400 μl | 60min | #17 | 0,379 | | | |
| 100 | 100 μl Lsg B | 1400 μl | 60min | #18 | 0,382 | | | |
| 250 | 100 μl Lsg C | 1400 μl | 60min | #19 | 0,281 | 1500 μl Zellenkonzentrat + 1500 μl LPS-PMB Plasma | 250 μl→ 50 μl Plasma -80°C | 250 μl→ 50 μl Plasma -80°C |
| 250 | 100 μl Lsg C | 1400 μl | 60min | #20 | 0,29 | | | |
| 500 | 100 μl Lsg D | 1400 μl | 60min | #21 | 0,209 | 1500 μl Zellenkonzentrat + 1500 μl LPS-PMB Plasma | 250 μl→ 50 μl Plasma -80 °C | 250 μl→ 50 μl Plasma -80°C |
| 500 | 100 μl Lsg D | 1400 μl | 60min | #22 | 0,209 | | | |
| 1000 | 100 μl Lsg E | 1400 μl | 60min | #23 | 0,154 | 1500 μl Zellenkonzentrat + 1500 LPS-PMB Plasma | 250 μl→ 50 μl Plasma -80°C | 250 μl→ 50 μl Plasma -80 °C |
| 1000 | 100 μl Lsg E | 1400 μl | 60min | #24 | 0,16 | | | |

3.6. Ergebnisse

*Endotoxinbatch (Batchtest I):*

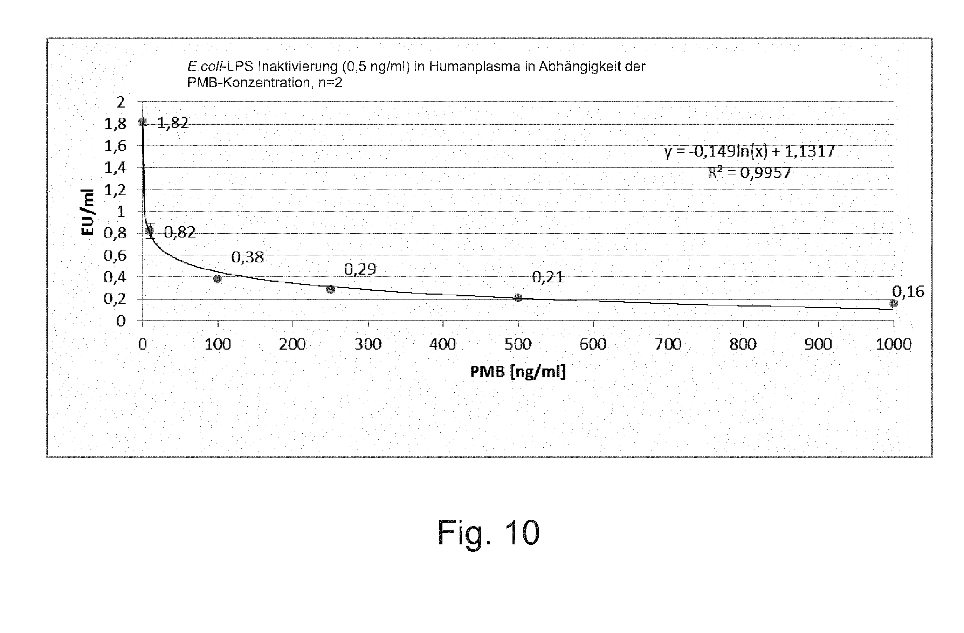

**Fig. 10** zeigt die Hemmung von LPS aus *E. coli* in Plasma (ursprüngliche LPS-Konzentration: 0,5 ng/ml) in Abhängigkeit der PMB-Konzentration (n=2) nach einer Inkubationszeit von 60 min.

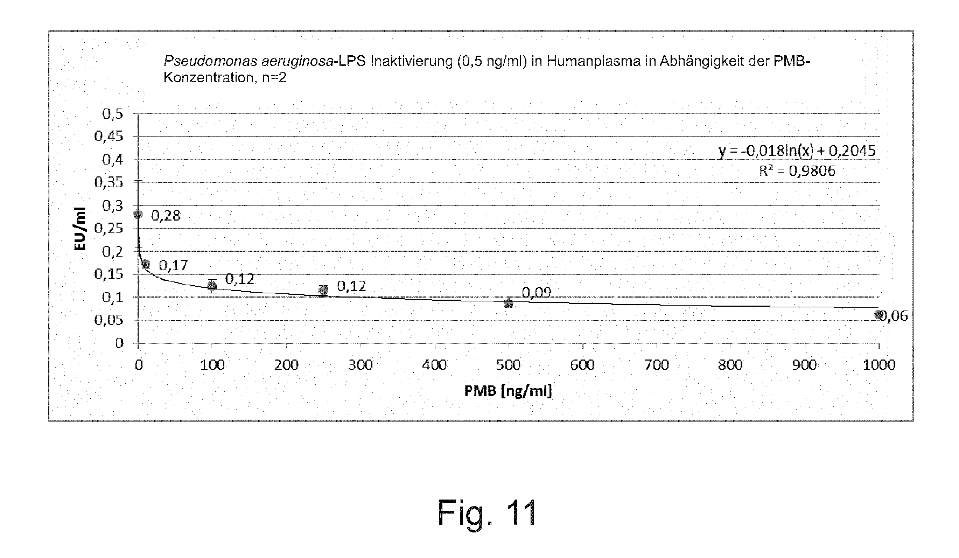

**Fig. 11** zeigt die Hemmung von LPS aus *Pseudomonas aeruginosa* in Plasma (ursprüngliche LPS-Konzentration: 0,5 ng/ml) in Abhängigkeit der PMB-Konzentration (n=2) nach einer Inkubationszeit von 60 min.

Die Ergebnisse zeigen deutlich, dass bereits bei einer sehr geringen PMB-Konzentration im Plasma, d.h. in einem Bereich von 50 bis 300 ng/ml (0,05 bis 0,3 μg/ml) eine starke Inhibierung von LPS aus *E. coli* und *Pseudomonas aeruginosa* stattfindet, wobei bei ansteigender PMB-Konzentration die LPS-Inhibierung nicht mehr signifikant zunimmt. Folglich sind bereits sehr geringe Konzentrationen an PMB ausreichend, um LPS (Endotoxine) in ihrer Aktivität zu hemmen. Bei diesen geringen Konzentrationen sind neuro- bzw. nephrotoxische Nebenwirkungen auszuschließen.

*Cytokinbatch (Batchtest II):*

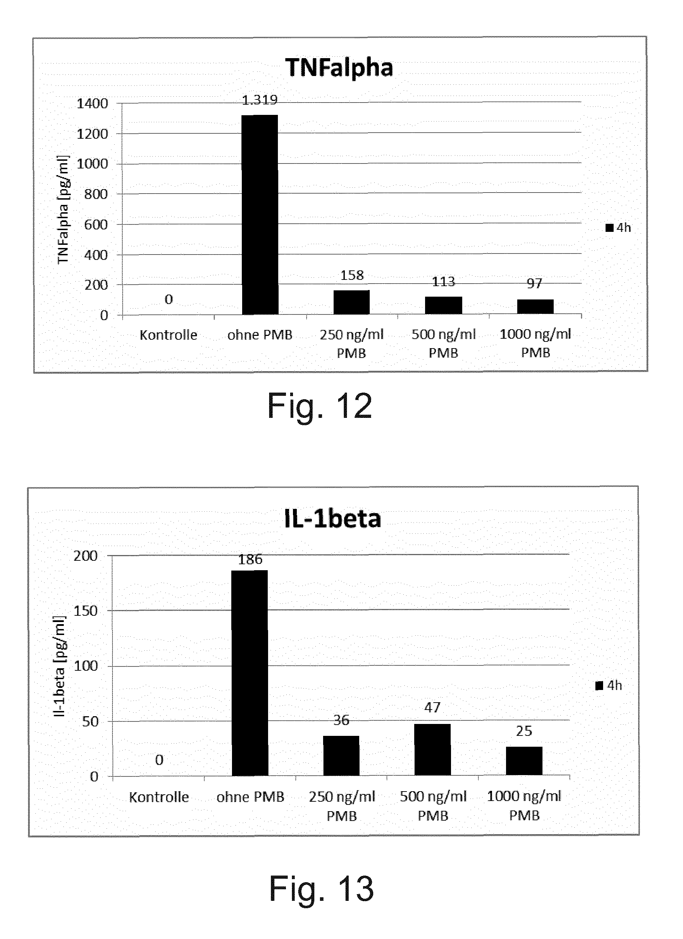

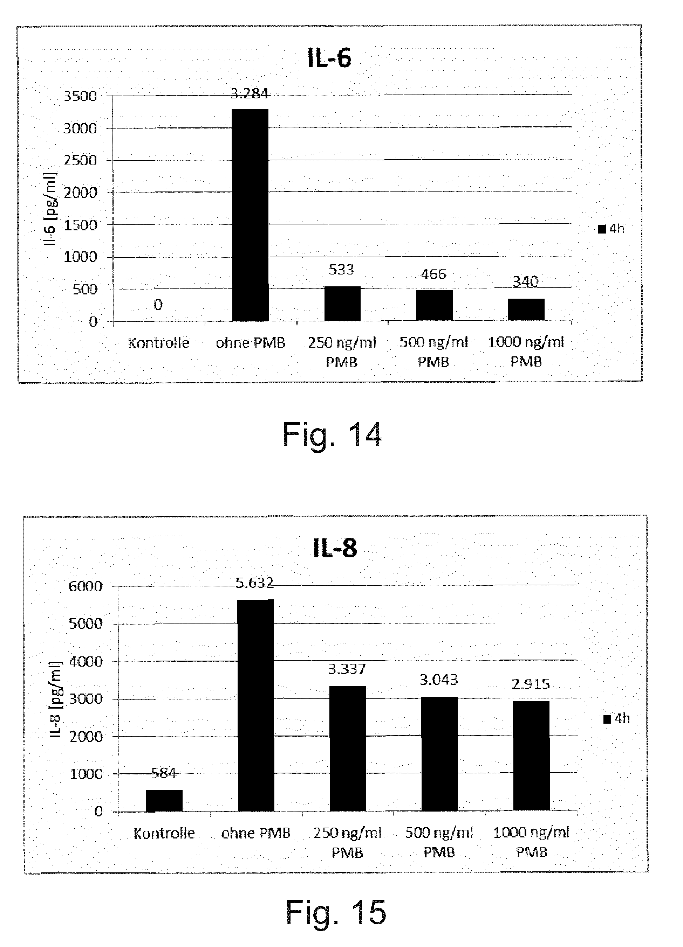

Die Ausschüttung der Cytokine TNF-alpha **(Fig. 12),** IL-1beta **(Fig. 13),** IL-6 **(Fig. 14)** und IL-8 (Fig. 15) durch die Blutzellen in Abhängigkeit der PMB-Konzentration (ohne PMB, 250 ng/ml, 500 ng/ml und 1000 ng/ml; Kontrolle mit 1000 ng/ml ohne LPS) in LPS *(E. coli)*-gespiktem Plasma nach 4 Stunden Inkubation ist in den Figuren 3 bis 6 dargestellt. Die Ergebnisse aus Batchtest II zeigen deutlich, dass bereits bei sehr geringen PMB-Konzentrationen nicht nur eine starke Inhibierung von LPS (siehe Batchtest I), sondern in Folge auch eine starke Inhibierung der Cytokinausschüttung stattfindet. Besonders ausgeprägt zeigt sich dies in der Inhibierung des Schlüsselmediators TNF-alpha **(Fig.12).**

**4. Beispiel 4: Beispiele für Formulierungen für Zubereitungen zur parenteralen Verabreichung von Polymyxin B (PMB) - Injektionslösungen (für Bolusgabe):**

4.1. Bolusgabe für eine PMB-Serumkonzentration von 100 ng/ml Plasma

Annahme: Patient mit 70 kg Körpergewicht und 60 % des Körpergewichts sind Verteilungsvolumen für PMB → 42000 ml Verteilungsvolumen.

Angestrebt ist eine PMB-Serumkonzentration von 100 ng PMB/ml Plasma → es werden insgesamt 4,2 mg PMB benötigt.

Injektionslösung für eine Bolusgabe über einen Zeitraum von 60 min: 4,2 mg PMB in 100 ml physiologischer Kochsalzlösung = fertige Injektionslösung für Bolusgabe über einen Zeitraum von 60 min.

### 4.2. Bolusgabe für eine PMB-Serumkonzentration von 250 ng/ml Plasma

Annahme: Patient mit 70 kg Körpergewicht und 60 % des Körpergewichts sind Verteilungsvolumen für PMB → 42000 ml Verteilungsvolumen.

Angestrebt ist eine PMB-Serumkonzentration von 250 ng PMB/ml Plasma → es werden insgesamt 10,5 mg PMB benötigt.

Injektionslösung für eine Bolusgabe über einen Zeitraum von 120 min: 10,5 mg PMB in 100 ml physiologischer Kochsalzlösung = fertige Injektionslösung für Bolusgabe über einen Zeitraum von 120 min.

Sobald durch die Bolusgabe die gewünschte PMB-Serumkonzentration eingestellt ist, wird diese durch PMB-Freisetzung mittels des, der erfindungsgemäßen Perfusionsvorrichtung zugeordneten, Abgabemittels wie oben beschrieben aufrechterhalten.

### 5. Beispiel 5: Beispiele für Infusionslösungen zur Infusion von Polymyxin B (PMB) in den extrakorporalen Blutkreislauf an einer Lipidzufuhrstelle sowie Dosierungsanleitungen

Annahme: Patient mit 70 kg → Verteilungsvolumen für PMB (60 % d. Körpermasse) 42000 ml Körperflüssigkeit mit 100 ng PMB/ml → 4,2 mg PMB im Verteilungsvolumen (siehe unter 2.1.1.).

Infusionslösung für eine 24 h-Infusion bei einer Serumhalbwertszeit von 6 h: Angenommene Halbwertszeit für PMB in Serum 6 h: 2,1 mg PMB pro 6 h bzw. 8,4 mg PMB/Tag werden abgebaut → 8,4 mg PMB in 1 L physiologischer Kochsalzlösung = Infusionslösung für 24 h-Infusion.

Infusionslösung für eine 24 h-Infusion bei einer Serumhalbwertszeit von 14 h: Halbwertszeit für PMB in Serum 14 Stunden: 4,2 mg PMB/14 h bzw. 7,2 mg PMB/Tag werden abgebaut → 7,2 mg PMB in 1 L physiologischer Kochsalzlösung = Infusionslösung für 24 h- Infusion.

### 6. Beispiel 6: Dosierungsanleitung zur Infusion von Polymyxin B (PMB) in den extrakorporalen Blutkreislauf an einer Lipidzufuhrstelle unter Berücksichtigung der PMB-Gesamtclearance der Perfusionsvorrichtung

Nachstehendes Berechnungsbeispiel berücksichtigt neben der PMB-Patientenclearance auch die Clearance eines im extrakorporalen Blutkreislauf angeordneten Dialysators (Dialysefilters) und die Clearance des Trägers des Abreicherungsmittels. Das Berechnungsbeispiel setzt eine bereits bestehende PMB-Serumkonzentration voraus. Diese erfolgt durch eine Bolusgabe vor Beginn der Behandlung, wobei hierfür die unter Beispiel 4 beschriebenen Injektionslösungen verwendet werden können.

Zur Berechnung der Dosierung von Polymyxin B via Infusion in den extrakorporalen Blutkreislauf an einer Lipidzufuhrstelle werden die PMB-Clearance des Patientenkörpers, des Dialysators und des Abreicherungsmittels berücksichtigt:

- Die PMB-Dialyseclearance (CDial) kann experimentell ermittelt werden und ist vom Plasmafluß sowie vom eingesetzten Dialysefilter-Typ abhängig. Im angeführten Beispiel beträgt diese 60 ml/min.

- Die PMB-Clearance des Abreicherungsmittels (Cads) ist vom eingesetzten Trägermaterial sowie vom Filtratfluss abhängig. Im angeführten Beispiel beträgt diese 45 ml/ min.

- Die PMB-Patientenclearance wurde im angeführten Beispiel aus der Halbwertszeit für PMB von 13,6 ermittelt und beträgt 36 ml/min.

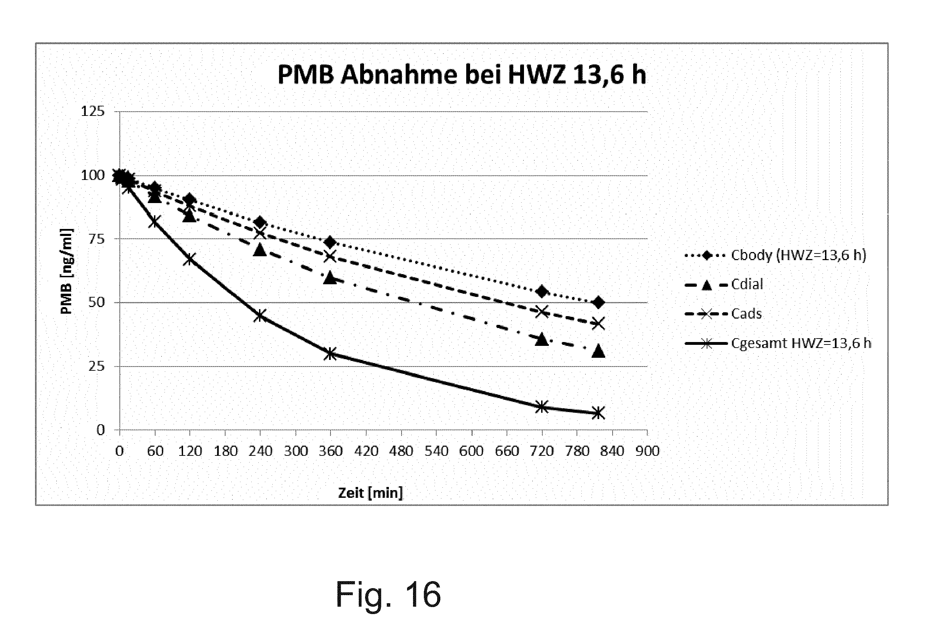

Aus den einzelnen PMB-Clearance-Raten ergibt sich durch Addition die PMB-Gesamtclearance (Cgesamt). Die daraus resultierende Abnahme des PMB ist in **Fig. 16** dargestellt. Der in **Fig. 16** ersichtliche negative Anstieg der PMB-Abnahme (Cgesamt) zu einem bestimmten Zeitpunkt entspricht der notwendigen PMB-Infusion, um die PMB-Serumkonzentration des zugehörigen Zeitpunkts aufrecht zu erhalten.

Für das angeführte Beispiel ergeben sich folgende Infusionsraten:

=> 0,84 mg PMB/h während der Behandlung mit Dialyse und Adsorption

Daraus ergibt sich bei 6-stündiger extrakorporaler Behandlung folgende <u>zu infundierende PMB-Menge:</u>

6 Stunden Behandlung mit Dialyse und Adsorption:     5,1 mg

**7. Beispiel 7: Verbesserte Adsorption von Zytokinen durch die Verwendung eines Albuflow-Filters (Vergleich Plasma und fraktioniertes Plasma)**

<u>7.1 Batchtest Zytokinadsorption</u>

*Versuchsbeschreibung:*

Es sollen Adsorber mit unterschiedlichen Porengrößen (30nm und 15-20 nm) bezüglich Adsorption von TNFa, IL-6 IL-10 in Vollplasma und im fraktioniertem Plasma getestet werden.

*Versuchsaufbau:*

Träger: Polystyrol-Divinylbenzol-Copolymer, CG300c (Träger A), CG161c (Träger B), Dow Chemical Group

Träger A: Partikelgröße: 120 $\mu$m, Porengröße: 30 nm
Träger B: Partikelgröße: 120 $\mu$m, Porengröße: 15-20 nm

Plasma: unfraktioniertes Citratplasma tiefgefroren (Fresh Frozen Plasma, gewonnen mittels Blutzentrifugation)
Fraktioniertes Citratplasma: gewonnen durch den Einsatz des Albuflow-Filters (Fresenius Medical Care, Deutschland).
Zytokinspike (TNF-$\alpha$, IL-6, IL-10) gemäß unten stehender Tabelle 7.1.
Träger A und B werden konditioniert: der Träger wird mit 2,5 fachen Volumen Ethanol absolut gewaschen und der Träger wird abzentrifugiert. Der Überstand wird verworfen und der Träger wird 1 Stunde lang mit 2,5 fachen Volumen Ethanol absolut bei Raumtemperatur inkubiert, dann abzentrifugiert und der Überstand wird abermals verworfen. Die gleiche Prozedur wird anschließend mit doppelt destilliertem Wasser und schlussendlich mit physiologischer Kochsalzlösung durchgeführt. Nach der Konditionierung wird der Träger zusätzlich noch 3x mit 0,9 %iger NaCl-Lösung gewaschen.
Der Batchtest wird im Dreieransatz sowohl in Vollplasma als auch in fraktioniertem Plasma (Vorbehandlung durch den Albuflow-Filter) durchgeführt.
Vor Versuchsbeginn werden die Träger mit ungespiktem Vollplasma beziehungsweise mit fraktioniertem Plasma für 15 min inkubiert, 1x mit NaCl gewaschen und dann für den Batchtest verwendet.
Batchansatz: Je 1 ml Adsorber (feucht) + 9 ml Citratplasma am Rollenmischer bei 37 °C für 60 min.

*Tabelle 7.1: Zytokinspike*

| | Lot Nr | Stock-Konzentration [μg/mL] | Stockverdünnung 1:10 | Stockverd. zu 40 ml Plasma | erwartete Endkonzentration im Plasma [pg/ml] | Gemessene Endkonzentration im Plasma |
|---|---|---|---|---|---|---|
| TNF-α | AA27/1082 | 10 | 10 Stock + 90 NaCl | 8 μL | 500 | 625 |
| IL-6 | OJZ0411121 | 10 | 10 Stock + 90 NaCl | 35 μL | 200 | 355 |
| IL-10 | EYB0211041 | 10 | 10 Stock + 90 NaCl | 80 μL | 300 | 517 |

*Analysen:*

Die Quantifizierung von TNFa, IL-6 und IL-10 erfolgte mittels kommerzieller ELISA-Kits der Firma R&D Systems.

7.2 Ergebnisse

Im fraktionierten Plasma konnte eine verbesserte Zytokinadsorption ermittelt werden.

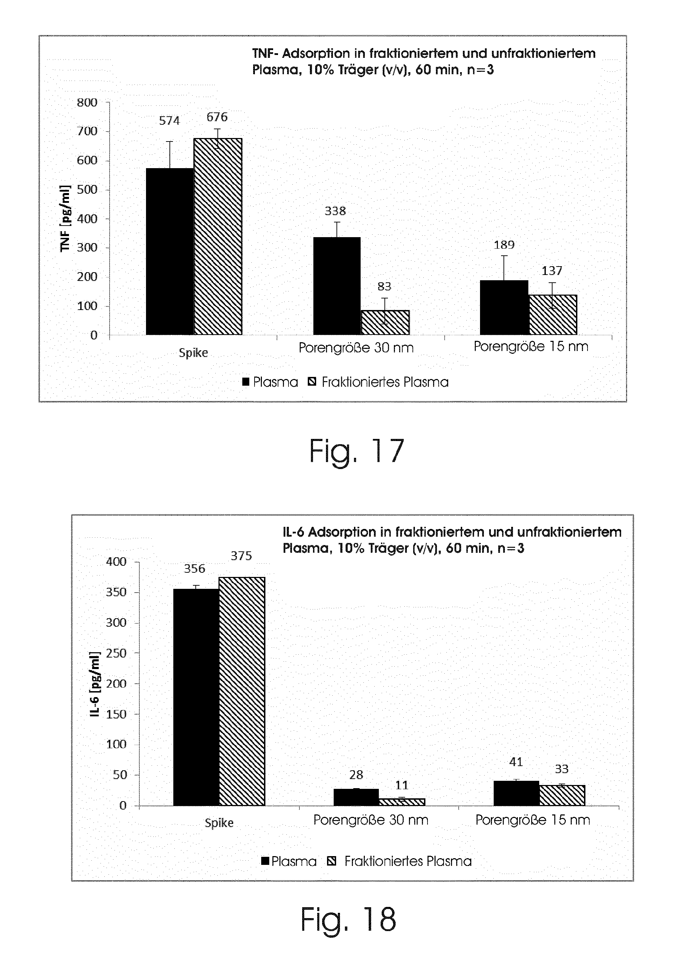

Die Fig. 17-19 zeigen die verbesserte Adsorption der Zytokine TNF-$\alpha$, IL-6 und IL-10 durch Verwendung eines Albuflow-Filter im Vergleich zu einem Plasmafilter.

**8. BEISPIEL 8: Austesten von Ethylvinylbenzol-Divinylbenzol-Copolymeren gleicher Porengröße und verschiedener Partikelgröße hinsichtlich ihrer Adsorptionseigenschaften**

Ethylvinylbenzol-Divinylbenzol-Copolymere (Träger) mit gleichen mittleren Porengrößen (15-20 nm), aber mit unterschiedlichen mittleren Partikelgrößen von 3-5 $\mu$m, 35 $\mu$m, 75 $\mu$m bzw. 120 $\mu$m werden hinsichtlich der Adsorptionseigenschaft für Protein C verglichen.

8.1 Bereitstellen neutraler, hydrophober Polymere

Die in diesem Beispiel verwendeten Ethylvinylbenzol-Divinylbenzol-Copolymere (Amberchrom CG 161, Rohm&Haas/Dow Chemical Company) mit gleicher mittlerer Porengröße und unterschiedlicher mittlerer Partikelgröße sind in der Tabelle 8.1 aufgelistet.

*Tabelle 8.1: Ethylvinylbenzol-Divinylbenzol-Copolymere*

| Bezeichnung des Ethylvinylbenzol-Divinylbenzol-Copolymers | Mittlere Porengröße [nm] | Mittlere Partikelgröße [$\mu$m] |
|---|---|---|
| #2000 | 15-20 | 3-5 |
| #1785 | 15-20 | 35 |
| #1760 | 15-20 | 75 |
| #2004 | 15-20 | 120 |

8.2. Trägervorbereitung und Batchtest

Die in Tabelle 8.1 genannten Träger #2000, #1785, #1760 und und #2004 wurden im Batchtest hinsichtlich ihrer Adsorptionseigenschaften für Protein C untersucht und miteinander verglichen.

Die Träger wurden konditioniert und direkt vor dem Batchtest für 15 min in Plasma inkubiert, abzentrifugiert und anschließend im Batchtest eingesetzt.

Konditionierung von Trägern: Trockene Träger sollten vor der Verwendung konditioniert werden, um eine gute Benetzung mit wässrigen Lösungen bzw. mit Plasma zu ermöglichen. Trockene, hydrophobe Träger werden folgendermaßen vorbehandelt: Die benötigte Menge trockener Träger wird in ein 50 ml Greiner-Röhrchen gegeben und mit dem 5-fachem Volumen unvergälltem Ethanol gewaschen (suspendiert und 5 min bei 4000 rpm zentrifugiert). Der Überstand wird abgehoben und verworfen und mit frischem, unvergälltem Ethanol wieder suspendiert und für 1 h inkubiert (Enviro Genie, Frequenz 25:50). Nach der Inkubation wird die Trägersuspension abzentrifugiert (5 min bei 4000 rpm zentrifugiert) und der Überstand verworfen. Der Träger wird nun mit dem 5-fachem Volumen destilliertem Wasser gewaschen (suspendiert und 5 min bei 4000 rpm zentrifugiert). Der Überstand wird abgehoben und verworfen und mit frischem destilliertem Wasser wieder suspendiert und für 1 h inkubiert (Enviro Genie, Frequenz 25:50). Nach der Inkubation wird die Trägersuspension abzentrifugiert (5 min bei 4000 rpm zentrifugiert) und der Überstand verworfen. Der Träger wird nun mit dem 5-fachem Volumen physiologischer Kochsalzlösung gewaschen (suspendiert und 5 min bei 4000 rpm zentrifugiert). Der Überstand wird abgehoben und verworfen und mit frischer physiologischer Kochsalzlösung wieder suspendiert und für 1 h inkubiert (Enviro Genie, Frequenz 25:50). Nach der Inkubation wird die Trägersuspension abzentrifugiert (5 min bei 4000 rpm zentrifugiert) und der Überstand verworfen. Zuletzt wird mit physiologischer Kochsalzlösung eine 50 %ige Trägersuspension hergestellt und bis zur Verwendung im Kühlschrank aufbewahrt.

Für den Batchansatz (Dreifachansatz, n=3) wurden je 150$\mu$l Träger (feucht) mit 1350 $\mu$l Citratplasma in 15er Greinerröhrchen überschichtet. Die Röhrchen wurden im Enviro-Genie mit 25/50 rpm bei 37°C für 60 min geschüttelt. Als Kontrolle (120 $\mu$l NaCl + 1350 $\mu$l Citratplasma) wurde ein Röhrchen ohne Träger mitgeführt.

Nach 15 min und nach 60 min wurden für die Protein C-Analyse Proben zu je 500 μl gezogen. Protein C wurde am Sysmex (Siemens, CA560) mit den dazugehörigen Reagenzien (Siemens, OUVV17) analysiert.

8.3. Analysen und Ergebnisse

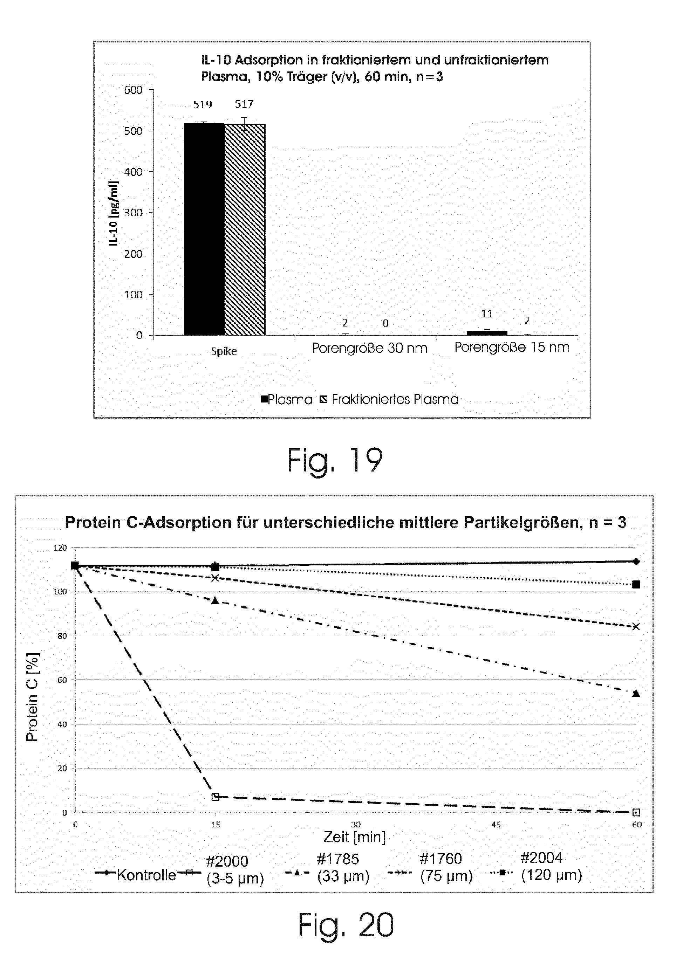

Fig. 20 zeigt die Protein C-Konzentration (Angabe in [%] in Bezug auf die physiologische Protein-C Konzentration in humanem Plasma) im Zeitverlauf für die einzelnen Träger. Anhand der Kurven zeigt sich sehr deutlich die Abhängigkeit der Protein C-Adsorption von der mittleren Partikelgröße. Eine ausgeprägte Protein C-Adsorption wurde bei den Trägern #2000 und #1785 festgestellt. Beim Träger #2000 war Protein C bereits nach 15 Minuten fast gänzlich aus dem Plasma entfernt. Durch die Träger #1760 und #2004 hingegen wurde Protein C in einem wesentlich geringeren Ausmaß aus dem Plasma adsorbiert. Die für den Träger #1760 beobachtete Protein C-Abnahme von - 25% (nach 60 min Inkubation) befindet sich gerade noch in einem Bereich, in welchem physiologisch relevante Mengen an Protein C im Plasma verbleiben. Die geringste Protein C-Adsorption, welche nach 60 min Inkubation lediglich 8% in Bezug auf die Protein C-Ausgangskonzentration betrug, wurde für Träger #2004 festgestellt. Die Protein C-Adsorption (in % bezogen auf die Protein C-Ausgangskonzentration) durch die einzelnen Träger ist in der Tabelle 8.3 aufgelistet.

*Tabelle 8.3:*

| Träger: | Protein-C-Adsorption nach 15 min Inkubation | Protein C-Adsorption nach 60 min Inkubation |
|---------|---------------------------------------------|---------------------------------------------|
| #2000 | 94 % | 99 % |
| #1785 | 14 % | 52% |
| #1760 | 5% | 25 % |
| #2004 | 1% | 8% |

## 9. Beispiel 9: Vergleich der PMB-Desorption in Plasma zwischen Adsorbern mit unterschiedlicher Poren und Partikelgröße und somit unterschiedlich verfügbarer Adsorptionsoberfläche

9.1 Träger

CG161c:

Der Träger (Rohm & Haas/Dow Chemical Company; im Folgenden auch als Adsorber bezeichnet) besteht aus einer porösen Polystyrol-Divinylbenzol Matrix. Die durchschnittliche Porengröße beträgt 15 nm, die durchschnittliche Partikelgröße beträgt 120 μm und die zugängliche Oberfläche beträgt 900 m$^2$/g Adsorber (trocken). Das Trockengewicht pro ml feuchten Adsorber beträgt 18 % (w/v).

HPR10:

Der Träger (Rohm & Haas/Dow Chemical Company; im Folgenden auch als Adsorber bezeichnet) besteht aus einer porösen Polystyrol-Divinylbenzol Matrix. Die durchschnittliche Porengröße beträgt 30-40nm, die durchschnittliche Partikelgröße beträgt 10 μm und die zugängliche Oberfläche beträgt 500 m$^2$/g Adsorber (trocken). Das Trockengewicht pro ml feuchten Träger beträgt 30 % (w/v).

9.2 Beschichtung der Träger mit Polymyxin B (PMB)

Die PMB-Lösung (Sigma Aldrich, 10 mg/ml in Aqua Dest) wird bei 121 °C, 30 min lang autoklaviert und anschließend der jeweilige Träger (CG161c bzw. HPR10) in 15 ml Greinerröhrchen wie folgt mit PMB beschichtet (Tabelle 9.2): 3 ml Träger mit 7,5 ml PMB Lösung

*Tabelle 9.2:*

| Beschichtung PMB in mg pro ml Träger | Träger [ml] | PMB-Lösung [ml] | NaCl [ml] |
|--------------------------------------|-------------|-----------------|-----------|
| 0 | 3 | 0 | 7,5 |
| 2,5 | 3 | 0,75 | 6,75 |
| 5 | 3 | 1,5 | 6 |

(fortgesetzt)

| Beschichtung PMB in mg pro ml Träger | Träger [ml] | PMB-Lösung [ml] | NaCl [ml] |
|---|---|---|---|
| 10 | 3 | 3 | 4,5 |
| 15 | 3 | 4,5 | 3 |
| 20 | 3 | 1,5 | 6 |
| 25 | 3 | 0 | 7,5 |

Die Beschichtung wird über Nacht am Rollenmischer bei Raumtemperatur durchgeführt. Danach wird der Adsorber2 mal mit 10 ml NaCl-Lösung (steril) gewaschen und eine 50 %ige Suspension hergestellt.

9.3 Batchtest

Im Doppelansatz werden je 0,5 ml Trägersuspension mit je 4,5 ml Citratplasma = 10 % (v/v) Ansatz bei 37 °C 60 min lang am Envirogenieinkubiert. Danach wird der Träger abzentrifugiert und der Überstand für die PMB-Quantifizierung mittes ELISA (Polymyxin-ELISA von Beijing Kwinbon Biotechnology Co., Ltd., China) verwendet.

9.4 Ergebnis

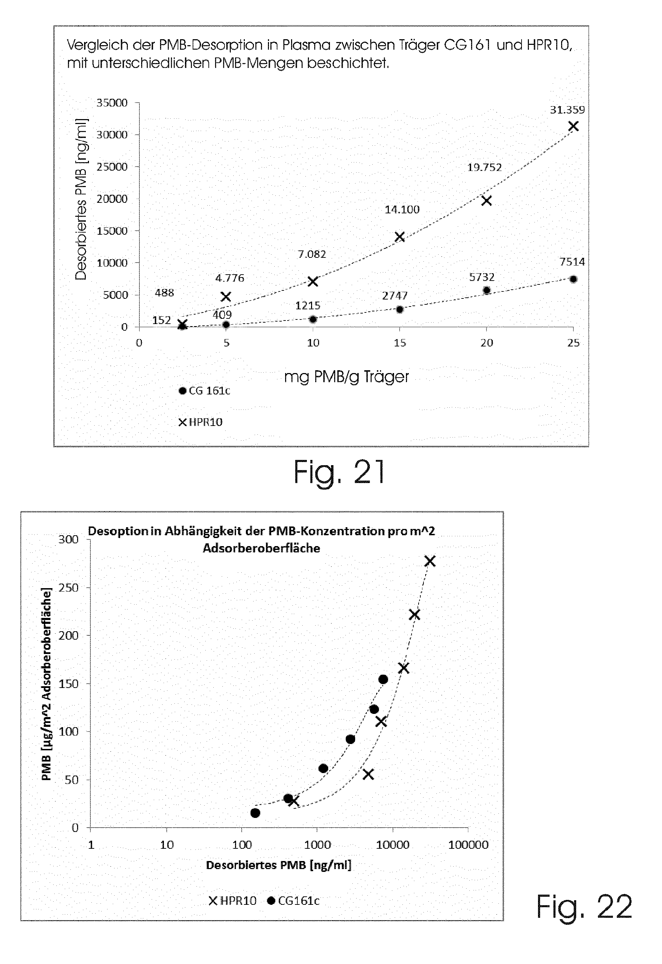

Aus dem Ergebnis (siehe **Fig. 21, Fig. 22, Fig. 23** und **Fig. 24)** ist klar ersichtlich, dass die Desorptionsrate von Polymyxin ins Plasma sehr stark von der verfügbaren Trägeroberfläche abhängig ist. Das heißt die Desorption von Polymyxin ist abhängig von der pro m$^2$ hydrophob gebundenen Polymyxinmenge. Dies geht auch aus den folgenden Berechnungstabellen (Tabelle 9.4.1 und Tabelle 9.4.2) klar hervor.

*Tabelle 9.4.1: Beispiel CG161c*

| mg PMB/ml Adsorber | Desorption PMB [ng/ml] | Oberfläche [m^2/g Adsorber (trocken)] | Trockenanteil % [w/v] | Oberfläche [m^2/ml Adsorber (feucht)] | **PMB [μg/m^2 Adsorberoberfläche]** |
|---|---|---|---|---|---|
| 2,5 | 152 | 900 | 18 | 162 | 15 |
| 5 | 409 | 900 | 18 | 162 | 31 |
| 10 | 1215 | 900 | 18 | 162 | 62 |
| 15 | 2747 | 900 | 18 | 162 | 93 |
| 20 | 5732 | 900 | 18 | 162 | 123 |
| 25 | 7514 | 900 | 18 | 162 | 154 |

*Tabelle 9.4.2: Beispiel HPR10*

| mg PMB/ml Adsorber | Desorption PMB [ng/ml] | Oberfläche [m^2/g Adsorber (trocken)] | Trockenanteil % [w/v] | Oberfläche [m^2/ml Adsorber (feucht)] | **PMB [μg/m^2 Adsorberoberfläche]** |
|---|---|---|---|---|---|
| 2,5 | 488 | 300 | 30 | 90 | 28 |
| 5 | 4776 | 300 | 30 | 90 | 56 |
| 10 | 7082 | 300 | 30 | 90 | 111 |
| 15 | 14100 | 300 | 30 | 90 | 167 |
| 20 | 19752 | 300 | 30 | 90 | 222 |
| 25 | 31359 | 300 | 30 | 90 | 278 |

9.5 Berechnungsbeispiele

Um durch die PMB-Desorption vom Träger (im Folgenden auch als Adsorber bezeichnet) die PMB-Konzentration im Plasma während einer Behandlung genau zu definieren sind in vitro Desorptions-Experimente (wie in Beispiel 9 durchgeführt) für den jeweiligen Adsorber notwendig. Durch die in den Experimenten gewonnenen Daten kann man durch den Beschichtungsgrad des Trägers (Menge an PMB pro g Adsorber) die Desorption ins Plasma und somit die PMB-Konzentration im Plasma sehr genau einstellen (siehe **Fig. 21 bis 24).** Wie in Beispiel 1 gezeigt werden konnte ist die Desorptionsrate in fraktioniertem Plasma niedriger und somit separat zu bestimmen.

Rechenbeispiel 1:

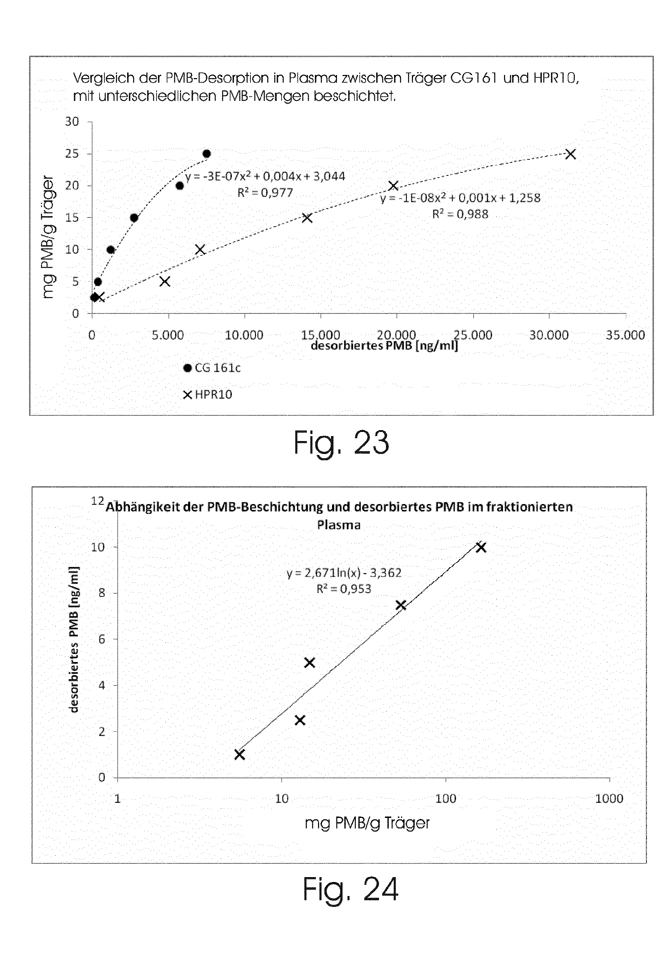

Es soll durch die Verwendung des PMB-beschichteten Adsorber HPR10 im extrakorporalen Blutkreislauf eine PMB-Konzentration in Plasma von 0,8 µg/ml erzielt werden. Durch Vorversuche (siehe **Fig. 23)** wurde experimentell die Funktion bestimmt, welche den Zusammenhang zwischen beschichteter PMB-Menge pro g Adsorber und desorbierter PMB-Menge ins Plasma beschreibt. In diesem Fall lautet diese:

$$PMB \left[ \frac{mg}{g\ Adsorber} \right] = 0{,}00000001x^2 + 0{,}0012x + 1{,}258$$

x = erwünschte PMB-Konzentration in Plasma = 0,8 µg/ml = 800 ng/ml
Wenn man für x = 800 ng/ml einsetzt bekommt man die PMB-Menge die man pro g Träger hydrophob binden muss: 2,224 mg pro g Träger (HPR10)

Rechenbeispiel 2:

Es soll durch die Verwendung des PMB-beschichteten Adsorbers CG161c im extrakorporalen Blutkreislauf eine PMB-Konzentration in Plasma von 0,8 µg/ml erzielt werden. Durch Vorversuche (siehe **Fig. 23)** wurde experimentell die Funktion bestimmt, welche den Zusammenhang zwischen beschichteter PMB-Menge pro g Adsorber und desorbierter PMB-Menge ins Plasma beschreibt. In diesem Fall lautet diese:

$$PMB \left[ \frac{mg}{g\ Adsorber} \right] = 0{,}0000003x^2 + 0{,}0048x + 3{,}0442$$

x = erwünschte PMB-Konzentration in Plasma = 0,8 µg/ml = 800 ng/ml
Wenn man für x = 800 ng/ml einsetzt bekommt man die PMB-Menge die man pro g Adsorber hydrophob binden muss: 7,076 mg pro g Adsorber (CG161c)

Rechenbeispiel 3:

Es soll durch die Verwendung des PMB-beschichteten Adsorbers HPR10 im extrakorporalen Blutkreislauf eine PMB-Konzentration in Plasma von 0,1 µg/ml erzielt werden. Durch
Vorversuche (siehe **Fig. 23)** wurde experimentell die Funktion bestimmt, welche den Zusammenhang zwischen beschichteter PMB-Menge pro g Adsorber und desorbierter PMB-Menge ins Plasma beschreibt. In diesem Fall lautet diese:

$$PMB \left[ \frac{mg}{g\ Adsorber} \right] = 0{,}00000001x^2 + 0{,}0012x + 1{,}258$$

x = erwünschte PMB-Konzentration in Plasma = 0,1 µg/ml = 100 ng/ml
Wenn man für x = 100 ng/ml einsetzt bekommt man die PMB-Menge die man pro g Adsorber hydrophob binden muss: 1,378 mg pro g Adsorber (HPR10)

Rechenbeispiel 4:

Es soll durch die Verwendung des PMB-beschichteten Adsorbers CG161c im extrakorporalen Blutkreislauf eine PMB-Konzentration in Plasma von 0,1 μg/ml erzielt werden. Durch Vorversuche (siehe **Fig. 23)** wurde experimentell die Funktion bestimmt, welche den Zusammenhang zwischen beschichteter PMB-Menge pro g Adsorber und desorbierter PMB-Menge ins Plasma beschreibt. In diesem Fall lautet diese:

$$PMB\left[\frac{mg}{g\ Adsorber}\right] = 0,0000003x^2 + 0,0048x + 3,0442$$

x = erwünschte PMB-Konzentration in Plasma = 0,1 μg/ml = 100 ng/ml
Wenn man für x = 100 ng/ml einsetzt bekommt man die PMB-Menge die man pro g Adsorber hydrophob binden muss: 3,527 mg pro g Adsorber (CG161c)

Rechenbeispiel 5 (fraktioniertes Plasma)

Es soll durch die Verwendung des PMB-beschichteten Adsorbers CG161c im extrakorporalen Blutkreislauf unter der Verwendung des Albuflow-Filters eine PMB-Konzentration in Plasma von 0,15 μg/ml erzielt werden. Durch Vorversuche (siehe **Fig. 24)** wurde experimentell die Funktion bestimmt, welche den Zusammenhang zwischen beschichteter PMB-Menge pro g Adsorber und desorbierter PMB-Menge ins fraktionierte Plasma beschreibt. In diesem Fall lautet diese:

$$PMB\left[\frac{mg}{g\ Adsorber}\right] = 2,6718\ln(x) - 3,3628$$

x = erwünschte PMB-Konzentration in Plasma = 0,15 μg/ml = 150 ng/ml
Wenn man für x = 150 ng/ml einsetzt bekommt man die PMB-Menge die man pro g Adsorber hydrophob binden muss: 10,025 mg pro g Adsorber (CG161c)

Rechenbeispiel 6 (fraktioniertes Plasma):

Es soll durch die Verwendung des PMB-beschichteten Adsorbers CG161c im extrakorporalen Blutkreislauf unter der Verwendung des Albuflow-Filters eine PMB-Konzentration in Plasma von 0,8 μg/ml erzielt werden. Durch Vorversuche (siehe **Fig. 24)** wurde experimentell die Funktion bestimmt, welche den Zusammenhang zwischen beschichteter PMB-Menge pro g Adsorber und desorbierter PMB-Menge ins fraktionierte Plasma beschreibt. In diesem Fall lautet diese:

$$PMB\left[\frac{mg}{g\ Adsorber}\right] = 2,6718\ln(x) - 3,3628$$

x = erwünschte PMB-Konzentration in Plasma = 0,8 μg/ml = 800 μ/ml
Wenn man für x = 800 ng/ml einsetzt bekommt man die PMB-Menge die man pro g Adsorber hydrophob binden muss: 14,497 mg pro g Adsorber (CG161c)

**10. Beispiel 10: Polymyxin B (PMB)-Desorption im Zeitverlauf**

Dieser Versuch sollt zeigen, dass die Gleichgewichtsreaktion (Adsorption und Desorption von Polymyxin (B)) rasch und stabil im Plasma ist.

10.1 Träger

*HPR10:* Der Träger *HPR10* (Rohm & Haas/Dow Chemical Company; im Folgenden auch als Adsorber bezeichnet) besteht aus einer porösen Polystyrol-Divinylbenzol Matrix. Die durchschnittliche Porengröße beträgt 30-40 nm, die durchschnittliche Partikelgröße beträgt 10 $\mu$m und die zugängliche Oberfläche beträgt 500 $m^2$/g Träger (trocken). Das Trockengewicht pro ml feuchten Träger beträgt 30 % (w/v).

## 10.2 Beschichtung des Trägers mit Polymyxin B (PMB)

Die PMB-Lösung (Sigma, 10 mg/ml in Aqua Dest) wird bei 121 °C, 30 min lang autoklaviert und anschließend der Träger (HPR10) in 15 ml Greinerröhrchen wie folgt mit PMB beschichtet (siehe Tabelle 10.2): 3 ml Träger mit 7,5 ml PMB Lösung

*Tabelle 10.2: Der Träger/Adsorber wird mit unterschiedlichen Mengen an PMB beschichtet*

| Beschichtung PMB in mg pro ml Adsorber | Adsorber [ml] | PMB-Lösung [ml] | NaCl [ml] |
|---|---|---|---|
| 5 | 3 | 1,5 | 6 |
| 10 | 3 | 3 | 4,5 |
| 25 | 3 | 0 | 7,5 |

Die Beschichtung wird über Nacht am Rollenmischer bei Raumtemperatur durchgeführt. Danach wird der beschichtete Träger 2 mal mit 10 ml NaCl-Lösung (steril) gewaschen und eine 50 %ige Suspension hergestellt. Als Kontrolle wurde ein Röhrchen ohne Adsorber mitgeführt.

## 10.3 Batchtest

Das Plasma mit 5 IU Heparin wird mit 5 ng/ml LPS(L-7018 Pseud. aerug. Fa. Sigma Lot: 128K4115) gespikt. Es wird im Dreifachansatz 1 % PMB-beschichteter Träger mit dem LPS gespiktem Plasma (30 $\mu$l Träger + 2970 $\mu$l LPS gespiktes Plasma) bei 37 °C am Überkopfschüttler inkubiert und in zeitlichen Abständen (5, 15 und 60 min) Proben für die LAL-Analytik gezogen.

## 10.4 Analytik

Die Analytik erfolgte mit einem LAL-Test.
*Verwendete Materialien für Batchtest und LAL-Tests:*

|  |  | *Lot:* |
|---|---|---|
| Mikrotiterplatten MT 1007 | Fa. Charles River | 1721599k.A. |
| Lal-Test Tubes T 200 | Ch. River Endosafe | 53351 Dk.A. |
| Combitips plus 5ml Biopur | Eppendorf | X131667I |
| Pipettentips | Eppendorf | V125542M |
| Pipettentips | Eppendorf | W130324Q |
| NaCl 0,9% | Mayerhofer | 8G5523 2011-07 |
| Mikrozentrifugentubes | Greiner | 05200108 |
| Charles RiverEndosafe | Endochrome | Kit.Lot: A2112EK1 |

## 10.5 Ergebnis

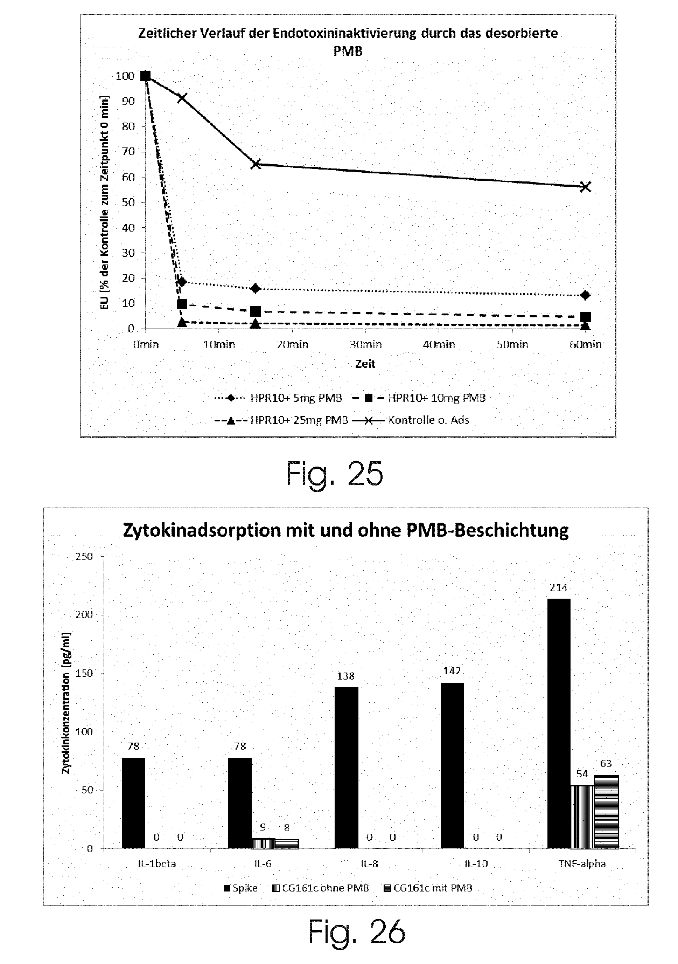

Die Gleichgewichtskonzentration vom desorbierten PMB im Plasma wird sehr schnell erzielt. Die LPS-Inaktivierung nach 5 Minuten ist beinahe gleich wie nach 60 Minuten langer Inkubation (siehe **Fig. 25).**

## 11. BEISPIEL 11: Einfluss der Beschichtung eines mit Polymyxin B(PMB)-beschichteten Trägers auf die Zytokinadsorption

### 11.1 Versuchsbeschreibung

Es soll in einem 10% (v/v) Batchtest getestet werden, wie gut der PMB beschichtete Adsorber CG161c im Vergleich zum unbeschichtetenAdsorber CG161c für die Adsorption von Zytokinen geeignet ist. 5 ng/ml Endotoxin (LPS) aus *Pseudomonas aeruginosa* werden ebenfalls zugesetzt.

11.2 Versuchsaufbau

*Träger:* Amberchrom CG161(mittlere Partikelgröße 120μm, mittlere Porengröße 15nm)

*Beschichtung mit PMB:*

Die PMB-Lösung (Sigma Aldrich, 10 mg/ml in Aqua Dest) und der Träger in 50 %iger Suspension werden in 15 ml Greinerröhrchen wie folgt mit PMB beschichtet (Tabelle 11.2.1):

*Tabelle 11.2.1:*

|  | 50 %igeAdsorbersuspension [ml] | PMB-Lösung [ml] | NaCl [ml] |
|---|---|---|---|
| 3xAnsatz | 2 | 1 | 2 |

Die Beschichtung wird über Nacht am Enviro-Genie (25:50) bei Raumtemperatur durchgeführt. Danach wird der Träger 2 mal mit 10 ml NaCl-Lösung (steril) gewaschen und eine 50 %ige Suspension hergestellt.

*Batchansatz:*

3fach Ansatz: je 1 ml Adsorber (feucht) + 9 ml Spike
15er Greinerröhrchen werden im Enviro-Genie 60 min mit 25/50 rpm bei 37°C geschüttelt.

*Zytokine:*

Die Stocklösung wird 1:10 in Plasma (freshfrozenplasma, Plasmaspendezentrum Retz) verdünnt (1:10; 5 μL Stock + 45 μL Plasma). Die Endkonzentration des Plasmaspikes (100mL) für die verwendeten Zytokine ist in der untenstehenden Tabelle 11.2.2 dargestellt:

*Tabelle 11.2.2:*

|  | Lot Nr | Stock-Konzentration [μg/mL] | Stockverdünnung 1: 10 | Stockverd. zu Plasma [100 mL] | Endkonzentration im Plasma [pg/mL] |
|---|---|---|---|---|---|
| TNF-α |  | 10 | 5 μL + 45 μl | 15 μL | 500 |
| IL-1β |  | 5 | 5 μL + 45 μl | 65 μL | 250 |
| IL-6 |  | 10 | 5 μL + 45 μl | 35 μL | 200 |
| IL-8 |  | 10 | 5 μL + 45 μl | 35 μL | 200 |
| IL-10 |  | 10 | 5 μL + 45 μl | 40 μL | 300 |

*Endotoxine (LPS):*

*Pseudomonas aeruginosa:* L-7018 Fa. Sigma Lot: 128K4115, -70°C, a 100 μl 10-3 g/ml (1mg/ml).
LPS wird im Batch mit einer Endkonzentrationen von 5 ng/ml Ansatz eingesetzt →50 μl $10^{-5}$er Lösung in 100 ml Plasma (Tabellen 11.2.3 und 11.2.4)

*Tabelle 11.2.3:*

| $10^{-4}$ | $10^{-5}$ |  |
|---|---|---|
| 0,9 | 0,900 | NaCl |
| 0,1 | 0,100 | LPS |
| 100 μg/ml | 10 μg/ml | LPS-Konzentration |

*Tabelle 11.2.4:*

|  | 0 min | 60 min |
|---|---|---|
| Spike ohne Adsorber | 1 | 2 |
| CG161c- 1 ohne PMB |  | 3 |
| CG161c - 2 ohne PMB |  | 4 |
| CG161c - 3 ohne PMB |  | 5 |
| CG161c - 4 mit PMB |  | 6 |
| CG161c - 5 mit PMB |  | 7 |
| CG161c - 6 mit PMB |  | 8 |
| = 8 Proben, d.h. 100 ml Citratplasmaspiken | | |

11.3 Analyse:

Die Zytokinanalyse erfolgt mit Hilfe eines Luminex-Gerätes (Antikörper basierend) der Firma Biorad.

11.4 Ergebnisse:

Die Ergebnisse sind in der **Fig. 26** dargestellt, aus welcher deutlich hervorgeht, dass eine adsorptive Beschichtung der Trägeroberfläche mit Polymyxin B keinerlei Auswirkungen auf die Adsorption der Zytokine hat.

**Patentansprüche**

1. Extrakorporale Perfusionsvorrichtung (100, 200, 300, 400, 500, 600, 700) umfassend einen extrakorporalen Blutkreislauf (102, 202, 302, 402, 502, 602, 702) zum Führen von Blut, einen Filtratkreislauf (105, 205, 305, 405, 505, 605, 705) zum Führen von Blutplasma und eine Steuerung (110, 210, 310,410, 510, 610, 710),
wobei der Filtratkreislauf (105, 205,...705) mit dem extrakorporalen Blutkreislauf (102, 202,...702) mittels eines Filters (104, 204, 304, 404, 504, 604, 704) in Verbindung steht, wobei der Filter (104, 204...704) einen Siebkoeffizienten von 5% für Substanzen mit einer molaren Masse von 340 000 g/mol (relative Molekülmasse von 340 kDa) aufweist, und
wobei im Filtratkreislauf (105, 205,...705) ein Abreicherungsmittel (107, 207, 307, 407, 507, 607, 707) umfassend einen ersten Träger (107a, 207a, 307a, 407a, 507a, 607a, 707a) mit einer neutralen, hydrophoben Oberfläche angeordnet ist,
**dadurch gekennzeichnet, dass**
die Perfusionsvorrichtung ein Abgabemittel (107, 207, 308, 408, 508, 607, 708) zum Zuführen eines endotoxinbindenden Lipopeptids in den extrakorporalen Blutkreislauf (102, 202,...702) umfasst, wobei das endotoxinbindende Lipopeptid aus der Gruppe bestehend aus Polymyxinen, Polymyxin-Derivaten, deren Prodrugs und einer Kombination davon ausgewählt ist.

2. Extrakorporale Perfusionsvorrichtung nach Anspruch 1, **dadurch gekennzeichnet, dass** das endotoxinbindende Lipopeptid ein Polymyxin ausgewählt aus der Gruppe bestehend aus Polymyxin B, Colistin und deren Prodrugs ist.

3. Extrakorporale Perfusionsvorrichtung (100, 200, 600) nach Anspruch 1 oder 2, **dadurch gekennzeichnet, dass** das Abreicherungsmittel (107, 207, 607) das Abgabemittel (107, 207, 607) zum Zuführen des endotoxinbindenden Lipopeptids umfasst, wobei die Oberfläche des ersten Trägers (107a, 207a, 607a) eine adsorptive Beschichtung aus dem endotoxinbindenden Lipopeptid aufweist.

4. Extrakorporale Perfusionsvorrichtung (500) nach Anspruch 1 oder 2, **dadurch gekennzeichnet, dass** das Abgabemittel (508) zum Zuführen des endotoxinbindenden Lipopeptids im Filtratkreislauf (505) stromab des Abreicherungsmittels (507) angeordnet ist, wobei das Abgabemittel (508) einen zweiten Träger (508a) mit einer neutralen, hydrophoben Oberfläche umfasst, wobei die Oberfläche des zweiten Trägers (508a) eine adsorptive Beschichtung aus dem endotoxinbindenden Lipopeptid aufweist.

5. Extrakorporale Perfusionsvorrichtung (100, 200, 500, 600) nach Anspruch 3 oder 4, **dadurch gekennzeichnet, dass** das an der Oberfläche des ersten bzw. zweiten Trägers (107a, 207a, 508a, 607a) adsorbierte endotoxinbindende Lipopeptid in einer Menge vorliegt, die bei Zufuhr des Lipopeptids eine Lipopeptid-Serumkonzentration von 0,01 μg/ml bis 0,8 μg/ml ergibt.

6. Extrakorporale Perfusionsvorrichtung (100, 200, 500, 600) nach Anspruch 3 oder 4, **dadurch gekennzeichnet, dass** der erste bzw. der zweite Träger (107a, 207a, 508a, 607a) eine Gesamtoberfläche von 100 bis 1500 m$^2$/g aufweist, wobei 50 bis 2000 mg an endotoxinbindendem Lipopeptid in Bezug auf die Trägergesamtoberfläche adsorptiv an der Oberfläche des ersten bzw. zweiten Trägers (107a, 207a, 508a, 607a) gebunden sind.

7. Extrakorporale Perfusionsvorrichtung (300, 400) nach Anspruch 1 oder 2, **dadurch gekennzeichnet, dass** das Abgabemittel (308, 408) zum Zuführen des endotoxinbindenden Lipopeptids eine Dosiereinrichtung (308, 408) zum Zuführen des endotoxinbindenden Lipopeptids in den extrakorporalen Blutkreislauf (302, 402) an einer dem extrakorporalen Blutkreislauf (302, 402) zugeordneten Lipopeptidzufuhrstelle (313, 413) umfasst.

8. Extrakorporale Perfusionsvorrichtung (300, 400) nach Anspruch 7, **dadurch gekennzeichnet, dass** die Lipopeptidzufuhrstelle (313, 413) im extrakorporalen Blutkreislauf (302, 402) stromab des Filters (304, 404) angeordnet ist.

9. Extrakorporale Perfusionsvorrichtung nach Anspruch 8, **dadurch gekennzeichnet, dass** im extrakorporalen Blutkreislauf stromab des Filters ein Dialysator angeordnet ist, wobei die Lipopeptidzufuhrstelle im extrakorporalen Blutkreislauf stromab des Dialysators angeordnet ist.

10. Extrakorporale Perfusionsvorrichtung (300, 400) nach Anspruch 8 oder 9, **dadurch gekennzeichnet, dass** stromab des Filters (304, 404) bzw. des Dialysators und stromauf der Lipopeptidzufuhrstelle (313, 413) ein Messmittel (314, 414) zur Messung der Konzentration des endotoxinbindenen Lipopeptids angeordnet ist.

11. Extrakorporale Perfusionsvorrichtung (300, 400) nach einem der Ansprüche 7 bis 10, **dadurch gekennzeichnet, dass** die Steuerung (310, 410) der Perfusionsvorrichtung dazu eingerichtet ist, bei der Dosierung des Lipopeptids in das im extrakorporalen Blutkreislauf (302, 402) geführte Blut die Lipopeptid-Clearance des Körpers, die Lipopeptid-Clearance des Abreicherungsmittels (307, 407) und/oder die Lipopeptid-Clearance des Dialysators zu berücksichtigen.

12. Extrakorporale Perfusionsvorrichtung (700) nach Anspruch 1 oder 2, **dadurch gekennzeichnet, dass** das Abgabemittel zum Zuführen des endotoxinbindenden Lipopeptids einen im extrakorporalen Blutkreislauf (702) stromab des Filters (704) angeordneten Dialysator (708) umfasst, der dazu eingerichtet ist, dem extrakorporalen Blutkreislauf (702) das endotoxinbindende Lipopeptid mittels einer durch den Dialysator (708) geführten Dialyseflüssigkeit zuzuführen.

13. Extrakorporale Perfusionsvorrichtung nach einem der Ansprüche 1 bis 12, **dadurch gekennzeichnet, dass** der erste bzw. der zweite Träger (107a, 207a, 307a, 407a, 507a, 508a, 607a, 707a) aus einem neutralen, vorzugsweise synthetischen Polymer gebildet sind.

14. Extrakorporale Perfusionsvorrichtung nach Anspruch 13, **dadurch gekennzeichnet, dass** das Polymer ausgewählt ist aus einem vernetzten Polystyrol-Polymer oder einem vernetzten Ethyldivinylbenzol-Polymer, vorzugsweise einem Polystyrol-Divinylbenzol-Copolymer oder einem Ethylvinylbenzol-Divinylbenzol-Copolymer.

15. Extrakorporale Perfusionsvorrichtung nach einem der Ansprüche 1 bis 14, **dadurch gekennzeichnet, dass** der erste bzw. der zweite Träger (107a, 207a, 307a, 407a, 507a, 508a, 607a, 707a) porös ist und eine mittlere Porengröße von 100 nm oder kleiner aufweist.

16. Extrakorporale Perfusionsvorrichtung nach Anspruch 15, **dadurch gekennzeichnet, dass** der erste bzw. der zweite Träger (107a, 207a, 307a, 407a, 507a, 508a, 607a, 707a) eine mittlere Porengröße von 20 nm oder kleiner oder eine mittlere Porengröße von 80 bis 100 nm aufweist.

17. Extrakorporale Perfusionsvorrichtung nach einem der Ansprüche 1 bis 16, **dadurch gekennzeichnet, dass** der erste bzw. der zweite Träger (107a, 207a, 307a, 407a, 507a, 508a, 607a, 707a) faserartig oder partikelförmig ist.

18. Extrakorporale Perfusionsvorrichtung nach Anspruch 17, **dadurch gekennzeichnet, dass** der erste bzw. der zweite Träger (107a, 207a, 307a, 407a, 507a, 508a, 607a, 707a) die Form von Mikropartikeln mit einer mittleren Partikelgröße von 300 µm oder kleiner hat.

19. Extrakorporale Perfusionsvorrichtung nach Anspruch 18, **dadurch gekennzeichnet, dass** der erste Träger (107a, 207a, 307a, 407a, 507a, 607a, 707a) eine mittlere Porengröße von 10 bis 20 nm oder 80 bis 100 nm und eine mittlere Partikelgröße von 75 bis 150 µm, vorzugsweise eine mittlere Partikelgröße von 110 bis 130 µm, idealerweise eine mittlere Partikelgröße von 120 µm aufweist.

20. Extrakorporale Perfusionsvorrichtung (600) nach einem der Ansprüche 3, 7 oder 12, **dadurch gekennzeichnet, dass** der Filtratkreislauf (605) in den Filter (604) mündet und dass der erste Träger (607a) die Form von Mikropartikeln hat und der Filtratkreislauf (605) eine Suspension dieser Mikropartikel umfasst, wobei die Mikropartikel eine mittlere Partikelgröße von 20 µm oder kleiner, vorzugsweise eine mittlere Partikelgröße von 8 µm oder kleiner, idealerweise eine mittlere Partikelgröße von 5 µm oder kleiner, aufweisen.

Fig. 1

Fig. 2

Fig. 3

Fig. 4

Fig. 5

Fig. 6

Fig. 7

Fig. 8

Fig. 9

E.coli-LPS Inaktivierung (0,5 ng/ml) in Humanplasma in Abhängigkeit der PMB-Konzentration, n=2

$y = -0,149\ln(x) + 1,1317$
$R^2 = 0,9957$

Fig. 10

Fig. 11

Fig. 12

Fig. 13

EP 2 679 301 A1

Fig. 14

Fig. 15

Fig. 16

Fig. 17

Fig. 18

Fig. 19

Fig. 20

Fig. 21

Fig. 22

Vergleich der PMB-Desorption in Plasma zwischen Träger CG161 und HPR10, mit unterschiedlichen PMB-Mengen beschichtet.

$y = -3E\text{-}07x^2 + 0,004x + 3,044$
$R^2 = 0,977$

$y = -1E\text{-}08x^2 + 0,001x + 1,258$
$R^2 = 0,988$

desorbiertes PMB [ng/ml]

mg PMB/g Träger

● CG 161c
✕ HPR10

## Fig. 23

Abhängikeit der PMB-Beschichtung und desorbiertes PMB im fraktionierten Plasma

$y = 2,671\ln(x) - 3,362$
$R^2 = 0,953$

desorbiertes PMB [ng/ml]

mg PMB/g Träger

## Fig. 24

**Fig. 25**

**Fig. 26**

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DIETER FALKENHAGEN ET AL: "Fluidized Bed Adsorbent Systems for Extracorporeal Liver Support", THERAPEUTIC APHERESIS AND DIALYSIS, Bd. 10, Nr. 2, April 2006 (2006-04), Seiten 154-159, XP055044253, ISSN: 1744-9979, DOI: 10.1111/j.1744-9987.2006.00357.x | 1,2, 13-18 | INV. B01J20/26 A61M1/36 B01J20/28 B01J20/32 A61K31/745 |
| Y | * Zusammenfassung * * Seite 155, rechte Spalte; Abbildung 2 * * Seite 157, rechte Spalte - Seite 158, rechte Spalte; Abbildung 4 * ----- | 3-6,20 | |
| Y,D | WO 2010/083545 A2 (ZENTRUM FUER BIOMEDIZINISCHE T [AT]; FALKENHAGEN DIETER [AT]; WEBER VI) 29. Juli 2010 (2010-07-29) | 3-6,20 | |
| A | * Seite 1, Absatz 2 * * Seite 7, Absatz 4 - Seite 9, Absatz 5; Ansprüche 1,3,5-8,11-16 * ----- | 7-12,19 | |

**RECHERCHIERTE SACHGEBIETE (IPC)**

B01J
A61M
A61K

Der vorliegende Recherchenbericht wurde für alle Patentansprüche erstellt

| Recherchenort | Abschlußdatum der Recherche | Prüfer |
|---|---|---|
| Den Haag | 11. April 2013 | Klemps, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 15 7246

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-04-2013

| Im Recherchenbericht angeführtes Patentdokument | Datum der Veröffentlichung | Mitglied(er) der Patentfamilie | Datum der Veröffentlichung |
|---|---|---|---|
| WO 2010083545 A2 | 29-07-2010 | AT 507846 A1<br>CN 102361689 A<br>EP 2389242 A2<br>JP 2012515577 A<br>US 2012152847 A1<br>WO 2010083545 A2 | 15-08-2010<br>22-02-2012<br>30-11-2011<br>12-07-2012<br>21-06-2012<br>29-07-2010 |

Für nähere Einzelheiten zu diesem Anhang : siehe Amtsblatt des Europäischen Patentamts, Nr.12/82

EPO FORM P0461

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20010070424 A1
- WO 2011123767 A1
- WO 2003090924 A
- DE 19515554 A1
- WO 2005082504 A2
- EP 0787500 B1
- EP 0958839 B1
- EP 1944046 B1
- DE 19913707 A1
- DE 102004029573 A1
- DE 102005046258 A1
- EP 0110409 A
- EP 0129786 A2
- WO 2010083545 A
- WO 2011160149 A
- WO 2007142611 A1
- US 5510242 A
- WO 2011133287 A1
- US 4382124 A
- EP 0776223 B
- US 5855782 A

**In der Beschreibung aufgeführte Nicht-Patentliteratur**

- **TETTA et al.** *Nephrol Dial Transplant,* 1998, vol. 13, 1458-1464
- **CANTALUPPI et al.** *Critical Care,* 2010, vol. 14, R4
- **TONY VELKOV et al.** *Journal of Medicinal Chemistry,* 2010, vol. 53 (5), 1898-1916
- **FALAGAS ; KASIAKOU.** *Critical Care,* 2006, vol. 10, R27
- Polymyxin B for Injection 500 000 Units. Hersteller: Bedford Laboratories
- **GARIDEL ; BRANDENBURG.** *Anti-Infective Agents in Medicinal Chemistry,* 2009, vol. 8, 367-385
- **WEBER V. ; LOTH F. ; LINSBERGER I. ; FALKEN-HAGEN D.** *Int. J. Artif. Organs,* vol. 25 (7), 679
- **CRUZ DN et al.** Effektiveness of polymyxin B-immobilized fiber column in sepsis: a systematic review. *Crit. Care,* 2007, vol. 11 (3), 137
- **BLAIS ; YAMAZAKI.** Use of polymyxin-coated polyester cloth in the enzyme immunoassay of Salmonella lipopolysaccharide antigens. *International journal of Food Microbiology,* 1990, vol. 11, 195-204
- **FALKENHAGEN et al.** Fluidized Bed Adsorbent System for Extracorporeal Liver Support. *Therapeutic Apheresis and Dialysis,* 2006, vol. 10 (2), 154-159
- **VELKOV et al.** *Journal of Medicinal Chemistry,* 2010, vol. 53 (5), 1898-1916
- **JIANG et al.** A synthetic peptide derived from bactericidal/permability-increasing protein neutralizes endotoxin in vitro and in vivo. *International Immunopharmacology,* 2004, vol. 4, 527-537
- **WEBER et al.** Neutral styrene divinylbenzene copolymers for adsorption of toxins in liver failure. *Biomacromolecules,* 2008, vol. 9 (4), 1322-1328
- **DAVANKOV et al.** *J.Polymer Science,* 1974, vol. 47, 95-101
- **FALKENHAGEN D ; STROBL W ; VOGT G ; SCHREFL A ; LINSBERGER I ; GERNER FJ ; SCHOENHOFEN M.** Fractionated plasma separation and adsorption system: a novel system for blood purification to remove albumin bound substances. *Artif Organs,* Januar 1999, vol. 23 (1), 81-6
- **JIANG et al.** A synthetic peptide derived from bactericidal/permabilityincreasing protein neutralizes endotoxin in vitro and in vivo. *International Immunopharmacology,* 2004, vol. 4, 527-537